(19) Europäisches Patentamt
European Patent Office
Office européen des brevets

(11) Veröffentlichungsnummer: **0 331 083**
**A2**

(12) **EUROPÄISCHE PATENTANMELDUNG**

(21) Anmeldenummer: 89103410.0

(22) Anmeldetag: 27.02.89

(51) Int. Cl.⁴: **C12N 15/00 , A01H 1/00 , A01H 5/10**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4323

(30) Priorität: 02.03.88 CH 783/88

(43) Veröffentlichungstag der Anmeldung:
06.09.89 Patentblatt 89/36

(84) Benannte Vertragsstaaten:
AT BE CH DE ES FR GB GR IT LI LU NL SE

(71) Anmelder: SCHWEIZERISCHE EIDGENOSSENSCHAFT EIDGENÖSSISCHE TECHNISCHE HOCHSCHULE (ETH)
ETH-Zentrum Rämistrasse 101
CH-8092 Zürich(CH)

(72) Erfinder: Potrykus, Ingo, Prof. Dr.
Im Stigler 54
CH-4312 Magden(CH)
Erfinder: Neuhaus, Gunther, Dr.
Birkenstrasse 3
CH-8708 Männedorf(CH)
Erfinder: Datta, Swapan K., Dr.
Gotthelfstrasse 37
CH-8003 Zürich(CH)
Erfinder: Spangenberg, German, Dr.
Badenerstrasse 255
CH-8003 Zürich(CH)

(74) Vertreter: Zumstein, Fritz, Dr. et al
Zumstein & Klingseisen Patentanwälte
Bräuhausstrasse 4
D-8000 München 2(DE)

(54) **Verfahren zur Herstellung transgener Pflanzen.**

(57) Gegenstand der vorliegenden Erfindung ist ein neuartiges, reproduzierbares Verfahren zur Einschleusung genetischen Materials in pflanzliche Proembryonen bzw. Embryonen unter Verwendung der Mikroinjektionstechnologie.
Weiterhin betrifft die vorliegende Erfindung die Verwendung des erfindungsgemässen Verfahrens zur Herstellung transgener Pflanzen sowie die mit Hilfe dieses Verfahrens erhältlichen transgenen Pflanzen selbst und deren Nachkommen.

EP 0 331 083 A2

Abbildung 2

## Verfahren zur Herstellung transgener Pflanzen

Gegenstand der vorliegenden Erfindung ist ein neuartiges, reproduzierbares Verfahren zur Einschleusung genetischen Materials in zygotische Embryonen von Pflanzen unter Verwendung der Mikroinjektionstechnologie, welches sich durch seine universelle Anwendbarkeit auszeichnet.

Weiterhin betrifft die vorliegende Erfindung die Verwendung des erfindungsgemässen Verfahrens zur Herstellung transgener Pflanzen sowie die mit Hilfe dieses Verfahrens erhältlichen transgenen Pflanzen selbst und deren Nachkommen.

Für die genetische Manipulation des pflanzlichen Erbgutes mit Hilfe der rekombinanten DNA Technologie stehen mittlerweile eine Vielzahl von Verfahren und Techniken zur Verfügung, die in vielen Laboratorien bereits routinemässig angewendet werden.

Zu den am besten untersuchten und am häufigsten verwendeten Verfahren zählt zweifellos das Agrobacterium-Transformationssystem.

Agrobacterium-Zellen besitzen auf ihrem Ti-Plasmid ein grosses DNA-Fragment, die sogenannte T-DNA-Region, das bei der natürlichen Transformation pflanzlicher Zellen in das Pflanzengenom integriert wird.

Dieses natürliche Gen-Transfer-System kann nach Ausführung verschiedener Modifikationen als Gen-Vektor-System für die gezielte Transformation von Pflanzen verwendet werden [Chilton, MD, 1983].

Das Agrobacterium-Transformationssystem hat aber den entscheidenden Nachteil, dass der Wirtsbereich der Agrobakterien auf bestimmte dikotyle Pflanzen sowie auf einige wenige Vertreter der Monokotyledonen (Hernal steens et al, 1984; Hookas-Van-Slagtoen et al., 1984), die aber aus agrarökonomischer Sicht unbedeutend sind, beschränkt ist. Das bedeutet, dass die wichtigsten Kulturpflanzen für einen effektiven Gentransfer nicht zugängig sind.

Darüberhinaus handelt es sich bei den verwendeten Agrobakterien um Pathogene, die bei ihren Wirtspflanzen charakteristische Krankheitssymptome in Form von krebsartigen Gewebewucherungen auslösen und daher nur unter Wahrung strenger Sicherheitsauflagen im Labor gehandhabt werden dürfen.

Alternative Transformationssysteme, die entwickelt wurden, um diese Nachteile des Agrobacterium Transformationssystems auszugleichen und die auf eine Einschleusung exogener DNA in pflanzliche Protoplasten abzielen, wie der direkte Gentransfer Vektor-freier DNA in Protoplasten (Paszkowski et al., 1984, Potrykus et al., 1986) sowie die Mikroinjektion Vektor-freier DNA in Protoplasten (Spangenberg et al., 1986; Steinbiss and Stabel, 1983; Morikawa and Yamada, 1985) oder Zellen (Nomura and Komamine, 1986), oder Kallus (Melnikow P.V. et al; GB 2,200,367) müssen insofern als problematisch angesehen werden, als die Regeneration ganzer Pflanzen aus pflanzlichen Protoplasten einer Vielzahl von Pflanzenspezies, insbesondere aus der Gruppe der Gramineen, immer noch ein-Problem darstellt.

Untersuchungen von Yamada et al., 1986, und Toriyma et al., 1986, u.a. zur Regeneration von Reis-Protoplasten sowie von Harris et al., 1988 (Weizen) und Rhodes et al. (Mais) haben hier erste Fortschritte erkennen lassen.

Ein weiterer Nachteil dieser alternativen Transformations-Systeme betrifft die nach wie vor relativ geringen Transformationsraten, die zur Zeit maximal bei Werten zwischen 1 % und 5 % liegen.

Diese niedrigen Transformationsraten machen es weiterhin notwendig, die einzuschleusende DNA mit Markern (z.B. Antibiotika-Resistenzgene) zu versehen, die eine rasche Selektionierung der Transformanten aus der grossen Zahl nicht-transformierter Zellen ermöglichen.

Dies bedeutet aber, dass zur Zeit kein wirklich befriedigendes Transformations-Verfahren zur Verfügung steht, das eine auch unter kommerziellen Gesichtspunkten effiziente und kostengünstige Produktion transgener Pflanzen mit neuen und nützlichen Eigenschaften erlaubt, insbesondere was die Pflanzen aus der Gruppe der Monocotyledoneae angeht.

Es muss daher als eine vordringliche Aufgabe angesehen werden, Verfahren zu entwickeln, die eine schnelle, effiziente und reproduzierbare Transformation aller Pflanzen, unabhängig von ihrer taxonomischen Stellung und den damit verbundenen Besonderheiten, erlauben und somit eine auch unter kommerziellen Gesichtspunkten effektive und wirtschaftliche Produktion transgener Pflanzen gewährleisten.

In besonderem Masse trifft dies auf Pflanzen aus der Gruppe der Monocotyledoneae zu, insbesondere auf solche aus der Familie Gramineae, in der die aus agrarökonomischer Sicht bedeutendsten Kulturpflanzen wie Weizen, Gerste, Roggen, Hafer, Mais, Reis, Hirse u.a.m. enthalten sind und die daher von ganz besonderem wirtschaftlichem Interesse sind, insbesondere da für die Herstellung transgener monokotyler Pflanzen bisher noch keine befriedigenden Verfahren zur Verfügung stehen.

Erste Ansätze in dieser Richtung liefern zwei erst in jüngster Zeit entwickelte Transformations-Verfahren. Dabei handelt es sich zum einen um die Injektion exogener DNA in die jungen Blütenstände von Roggenpflanzen (de la Pena et al., 1987), zum anderen um eine Agrobacterium-vermittelte Virusinfektion von Mais-Pflanzen mit "Maize Streak Virus" (Grimsley et al., 1987). Doch auch diese neu entwickelten Verfahren sind mit Nachteilen verbunden; so hat sich beispielsweise das zuerst genannte Verfahren als nicht reproduzierbar erwiesen.

Im Rahmen der vorliegenden Erfindung ist es jetzt überraschenderweise gelungen, ein in hohem Masse effizientes Transformationssystem für Pflanzen, unabhängig von deren taxonomischer Zugehörigkeit und den damit verbundenen Besonderheiten, zu entwickeln, das frei ist von den zuvor genannten Beschränkungen der bisher bekannten Verfahren und das sich somit durch seine universelle Anwendbarkeit auszeichnet.

Im einzelnen handelt es sich dabei um ein Verfahren zur Einschleusung von genetischem Material in Pflanzen, das sich dadurch kennzeichnet, dass man eine DNA-haltige Lösung in junge zygotische Embryonen von Pflanzen, die ein hohes Regenerierungspotential in Form einer natürlichen Embryogenese besitzen, mikroinjiziert, die mikroinjizierten Embryonen in geeigneten, die Embryogenese fördernden Kulturmedien mikrokultiviert und diese anschliessend zu ganzen, vollständigen und lebensfähigen Pflanzen regeneriert.

Ein weiterer Gegenstand der vorliegenden Erfindung betrifft die Herstellung transgener ganzer Pflanzen, die unter Verwendung des erfindungsgemässen Verfahrens mit einem rekombinanten DNA Molekül transformiert worden sind, welches in der transformierten Pflanze zu neuen und wünschenswerten Eigenschaften führt sowie die auf diese Weise erzeugten transgenen Pflanzen selbst und deren Nachkommen.

Die vorliegende Erfindung betrifft weiterhin Pflanzen, welche aus transformierten primären oder sekundären Embryonen, die besagtes rekombinantes DNA Molekül enthalten, regeneriert werden sowie deren Samen, darüberhinaus die Nachkommen von Pflanzen, die aus besagtem transgenen Pflanzenmaterial regeneriert worden sind sowie Mutanten und Varianten davon.

Unter den Nachkommen transgener Pflanzen sind im Rahmen dieser Erfindung sowohl auf sexuellem als auch auf asexuellem Wege erzeugte Abkömmlinge der transformierten Mutterpflanze zu verstehen.

Die vorliegende Erfindung umfasst weiterhin hybride genetische Konstruktionen, die eine oder mehrere Gene oder DNA-Sequenzen enthalten, die in der transformierten Pflanze zu neuen und wünschenswerten Eigenschaften führen, weiterhin Klonierungsvehikel und Wirtsorganismen.

Ein weiterer Gegenstand der vorliegenden Erfindung betrifft die Verwendung des erfindungsgemässen Transformations-Verfahrens zur Herstellung transgener Pflanzen mit neuen und wünschenswerten Eigenschaften.

In der folgendenn Beschreibung werden eine Reihe von Ausdrücken verwendet, die in der rekombinanten DNA Technologie, sowie in der Pflanzengenetik gebräuchlich sind. Um ein klares und einheitliches Verständnis der Beschreibung und der Ansprüche sowie des Umfangs, der den besagten Ausdrücken zukommen soll, zu gewährleisten, werden die folgenden Definitionen aufgestellt.

Embryo: Frühes Entwicklungsstadium einer Pflanze bestehend aus Spross- und Wurzelvegetationspunkt und Keimblättern.

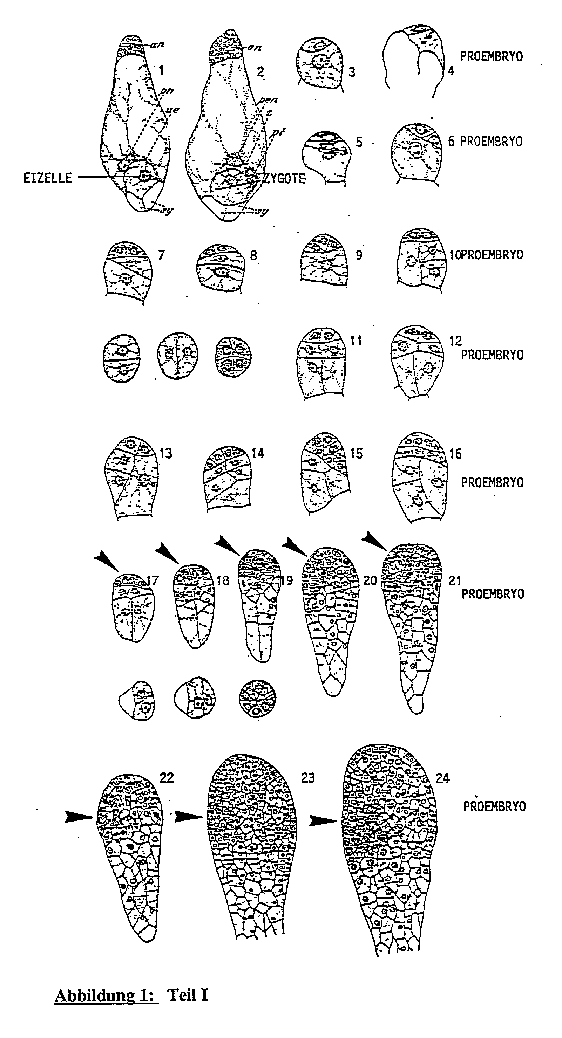

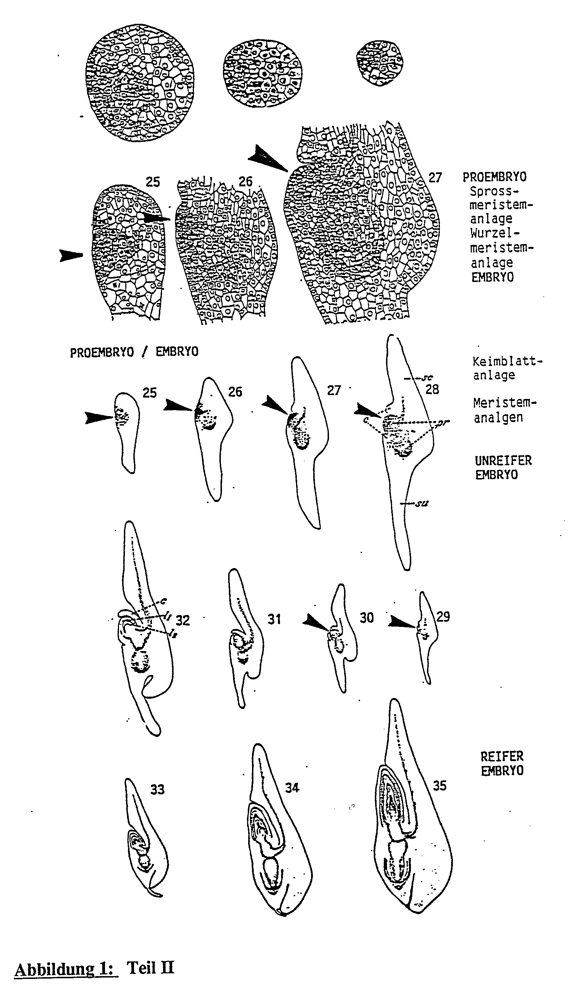

Proembryo: 2 bis vielzelliges Stadium der Embryonalentwicklung, vor der Ausbildung von Spross- und Wurzelvegetationsmeristem sowie Keimblättern (siehe Abb. 1).

Embryogenese: Entwicklung eines Embryos, ausgehend von einer Zelle, die die Potenz zur Embryobildung hat.

Zygotische Embryonen: Embryonen, die aus einer befruchteten Eizelle gebildet werden.

Somatische Embryonen: Embryonen, die aus somatischen Zellen mit embryogener Potenz gebildet werden.

Chimäre: Zelle, Gruppe von Zellen, Gewebe oder Pflanze, zusammengesetzt aus Zellen mit unterschiedlicher genetischer Information.

Pflanzliches Material: In Kultur oder als solches lebensfähige pflanzliche Teile wie Protoplasten, Zellen, Kallus, Gewebe, Embryonen, Pflanzenorgane, Knospen, Samen, u.a. sowie ganze Pflanzen.

Pflanzenzelle: Strukturelle und physiologische Einheit der Pflanze, bestehend aus einem Protoplasten und einer Zellwand.

Protoplast: Aus Pflanzenzellen oder -geweben isolierte, zellwandlose "nackte" Pflanzenzelle mit der Potenz zu einem Zellklon oder einer ganzen Pflanze zu regenerieren.

Zellklon: Aus einer Zelle durch fortlaufende Mitosen entstandene Population von genetisch gleichen Zellen.

Pflanzengewebe: Gruppe von Pflanzenzellen, die in Form einer strukturellen und funktionellen Einheit organisiert sind.

Pflanzenorgan: Strukturelle und funktionelle Einheit aus mehreren Geweben, wie beispielsweise Wurzel, Stamm, Blatt oder Embryo.

Transgene Chimäre: Pflanzen oder Gewebe, welche nicht in allen Zellen sondern nur in Gewebesektoren eine Fremd-DNA stabil ins Genom integriert enthalten.

3

Transgene Pflanze: Pflanze, welche in allen Zellen eine Fremd-DNA stabil ins Genom integriert enthält.

Heterologe(s) Gen(s) oder DNA: Eine DNA Sequenz, die ein spezifisches Produkt oder Produkte kodiert oder eine biologische Funktion erfüllt und die von einer anderen Spezies stammt als derjenigen, in welche das besagte Gen eingeschleust wird; besagte DNA Sequenz wird auch als Fremdgen oder Fremd-DNA bezeichnet.

Homologe(s) Gen(e) oder DNA: Eine DNA Sequenz, die ein spezifisches Produkt oder Produkte kodiert oder eine biologische Funktion erfüllt und die aus der gleichen Spezies stammt, in welche das besagte Gen eingeschleust wird.

Synthetische(s) Gen(e) oder DNA: Eine DNA-Sequenz, die ein spezifisches Produkt oder Produkte kodiert oder eine biologische Funktion erfüllt und die auf synthetischem Wege hergestellt sind.

Pflanzen-Promotor: Eine Kontrollsequenz der DNA Expression, die die Transkription jeder beliebigen homologen oder heterologen DNA Gensequenz in einer Pflanze gewährleistet, sofern besagte Gensequenz in operabler Weise mit einem solchen Promotor verknüpft ist.

Terminations-Sequenz: DNA-Sequenz am Ende einer Transkriptions-Einheit, die das Ende des Transkriptionsvorganges signalisiert.

Ueberproduzierender Pflanzen-Promotor (OPP): Pflanzen-Promotor, der in der Lage ist, in einer transgenen Pflanzenzelle die Expression einer jeden in operabler Weise verknüpften funktionalen Gensequenz(en) in einem Ausmass (gemessen in Form der RNA oder der Polypeptidmenge) zu bewirken, das deutlich höher liegt, als dies natürlicherweise in Wirtszellen, die nicht mit besagtem OPP transformiert sind, beobachtet wird

$3'/5'$ nicht-translatierte Region: Stromabwärts/stromaufwärts der kodierenden Region gelegene DNA-Abschnitte, die zwar in mRNA transkribiert, nicht aber in ein Polypeptid übersetzt werden. Diese Region enthält regulatorische Sequenzen, wie z.B. die Ribosomenbindungsstelle ($5'$) oder das Polyadenylierungssignal ($3'$).

DNA-Expressions-Vektor: Klonierungs-Vehikel, wie z.B. ein Plasmid oder ein Bakteriophage, das alle Signal-Sequenzen enthält, die für die Expression einer inserierten DNA in einer geeigneten Wirtszelle notwendig sind.

DNA-Transfer-Vektor: Transfer-Vehikel, wie z.B. ein Ti-Plasmid oder ein Virus, das die Einschleusung von genetischem Material in eine geeignete Wirtszelle ermöglicht.

Abbildung 1 (nach Randolph et al., 1936) zeigt die verschiedenen Phasen der Entwicklung eines Getreideembryos am Beispiel von Mais (Zea mays). Die Entwicklung der anderen Getreide wie Weizen (Triticum aestivum), Reis (Oryza sativa), Gerste (Hordeum vulgare) und anderer Grammineen verläuft in analoger Weise:

Die Befruchtung der Eizelle im Embryosack (1) führt zur Bildung der Zygote (2). Diese entwickelt sich über fortlaufende Mitosen zu den verschiedenen Phasen des Proembryos (3-25). Der Uebergang vom Proembryo zum Embryo (25-26) ist charakterisiert durch die Bildung von Sprossmeristem- und Wurzelmeristemanlagen und Scutellum (modifiziertes Keimblatt).

Die Abbildungen sind nicht im gleichen Massstab gezeichnet; der Proembryo (17) hat eine Länge von ca. 0,05 mm, der Proembryo (25) von ca. 1 mm, und der Embryo (35) ca. 8-10 mm.

Die Pfeile geben an, wo sich die für die Mikroinjektion der DNA-haltigen Lösung bevorzugten Bereiche befinden.

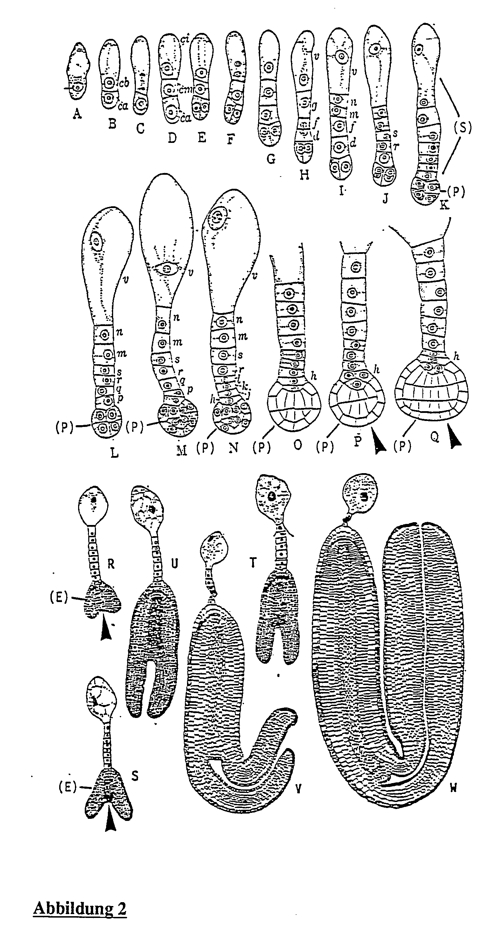

Abbildung 2 (nach Randolph et al., 1936) zeigt die verschiedenen Phasen der Entwicklung eines Dikotylenembryos am Beispiel des Hirtentäschel (Capsella bursa pastoris). Die Grundzüge der Entwicklung sind bei verschiedenen Arten gleich; Details können sehr unterschiedlich sein.

Die Befruchtung der Eizelle im Embryosack führt zur Bildung einer Zygote (A). Diese entwickelt sich durch fortlaufende Mitosen in Proembryo (P) und Suspensor (S). Der Uebergang vom Proembryo (globulär) zum Embryo (E) liegt zwischen den Phasen Q und R und beginnt mit der Bildung der Anlagen der beiden Keimblätter.

Die Pfeile geben die für die Mikroinjektion der DNA-haltigen Lösung bevorzugten Bereiche an.

Das erfindungsgemässe Transformations-Verfahren zur Einschleusung genetischen Materials in Pflanzen basiert auf der Mikroinjektion einer DNA-haltigen Lösung in junge zygotische Embryonen von Pflanzen, welche naturgemäss ein hohes Regenerierungspotential in Form der natürlichen Embryogenese aufweisen.

Bevorzugt im Rahmen dieser Erfindung ist die Mikroinjektion einer DNA-haltigen Lösung in zygotische Proembryonen bzw. Embryonen monokotyler Pflanzen, insbesondere in zygotische Embryonen von Getreiden und anderen Gräsern, für die bisher kein Transformationssystem zu Verfügung steht.

Bis zum Zeitpunkt der vorliegenden Erfindung war es nicht möglich, sehr junge zygotische Embryonen, die sich in einem noch sehr frühen Entwicklungsstadium befinden und Zellzahlen zwischen 4 und $\leq 1000$

Zellen aufweisen, in der Pflanze aufzufinden, zu isolieren und in Form einer in vitro Kultur zu kultivieren.

Man ging vielmehr bisher davon aus, dass solche sehr jungen zygotischen Embryonen (Proembryonen) in Kultur nicht wachstums- und vermehrungsfähig seien. Die bisher vorliegenden Berichte zur Isolierung und Kultivierung zygotischer Embryonen sind beschränkt auf Embryonen, die eine Minimalgrösse von einigen tausend Zellen aufweisen und damit für eine eventuelle Mikroinjektion aufgrund der viel zu hohen Zellzahl weniger geeignet erscheinen.

Im Rahmen der vorliegenden Erfindung ist es jetzt überraschenderweise erstmals gelungen, durch Anwendung spezifischer Verfahrensmassnahmen zygotische Embryonen bereits auf einem frühen Proembryonalstadium (vgl. Abb. 1 und 2) aus den sie umgebenden pflanzlichen Strukturen zu isolieren und in vitro zu kultivieren.

Die Mikroinjektion der DNA-haltigen Lösung in die aus befruchteten Eizellen abgeleiteten Proembryonen und Embryonen wird erfindungsgemäss mit Hilfe einer Mikroinjektionsapparatur durchgeführt, die mit manuell oder motorisch steuerbaren Mikromanipulatoren ausgestattet ist.

Der spezifische Aufbau sowie die Ausstattung besagter Mikroinjektionseinrichtungen sind in der Literatur vielfach beschrieben. Mikroinjektionsapparaturen werden mittlerweile auch von verschiedenen Firmen angeboten und können von diesen als Komplettausrüstung direkt bezogen werden (z.B. ZEISS; Narashige Nikon Instr.; Leitz etc.).

Die auf diese Weise durch Mikroinjektion transformierten Proembryonen und Embryonen können dann nach dem erfindungsgemässen Verfahren durch Mikrokultur zur direkten Embryogenese angeregt und anschliessend sehr einfach zu ganzen Pflanzen regeneriert werden.

Um dieses Ziel zu erreichen, werden die mikroinjizierten Proembryonen und Embryonen zunächst einzeln oder in kleinen Gruppen vorzugsweise in Vertiefungen handelsüblicher Mikrokultivierungsplatten in geeignete Kulturmedien eingebracht und bis zur Ausbildung der embryospezifischen Strukturen mikrokultiviert.

Selbstverständlich können im Rahmen dieser Erfindung auch andere, für die Mikrokultivierung zygotischer Pro-/Embryonen geeignete Kultivierungsverfahren Anwendung finden.

Im Anschluss an die Differenzierung zu Embryonen werden diese schliesslich in ein Vitamin- und Hormon-freies oder Hormon-armes Medium überführt, in welchem die weitere Differenzierung zu reifen Embryonen und schliesslich deren Keimung zum Pflänzchen erfolgt.

Die so erhaltenen Pflänzchen können dann in Erde übertragen und auf die gleiche Weise wie normale Sämlinge weiterkultiviert werden.

Ein wesentlicher Vorteil des erfindungsgemässen Transformations-Verfahrens gegenüber den bisher bekannten Verfahren basiert auf den ausserordentlich hohen Transformationsraten, die sich mit diesem Verfahren erreichen lassen und die mit Werten zwischen 20 % und 60 % um eine Zehnerpotenz höher liegen als die bisher in der Literatur beschriebenen maximal erreichbaren Transformationsraten.

Darüberhinaus können durch die direkte Regeneration von Pflanzen aus Embryonen via einer direkten Embryogenese die negativen Effekte der 'somaklonalen Variation', die bei anderen alternativen Verfahren in der Regel auftreten, insbesondere bei solchen, deren Regenerationsschritt über eine Kallusphase verläuft, gänzlich oder aber zumindest teilweise vermieden werden. Dies bedeutet aber, dass die genetisch veränderte Pflanze - mit Ausnahme des eingeführten Gens - mit der Ausgangspflanze identisch ist, was für die Belange der Pflanzenzüchtung von ausschlaggebender Bedeutung ist.

Ein weiterer entscheidender Vorteil des erfindungsgemässen Verfahrens ist dessen universelle Anwendbarkeit, unabhängig von der taxonomischen Zugehörigkeit der betreffenden Zielpflanze und den damit verbundenen Besonderheiten und verfahrenstechnischen Schwierigkeiten, wie sie bei Anwendung der bisher zur Verfügung stehenden Transformationsverfahren auftreten, z.B. in Form einer Beschränkung auf bestimmte Dikotyledonen bei Verwendung des Agrobacterium-Transformationssystems oder aber einer bisher noch ungenügenden Regenerierbarkeit monokotyler Pflanzen aus Protoplasten bei Anwendung der direkten Gentransfer-Verfahren.

Diese Universalität des erfindungsgemässen Verfahrens beruht darauf, dass man genetisches Material mit Hilfe einer physikalischen Methode (Mikroinjektion) in eine von jeder Pflanze erhältliche Struktur (junge sexuelle Embryonen), unabhängig von deren taxonomischer Zugehörigkeit, überträgt, welche die Ausbildung normaler, fertiler, ganzer Pflanzen im Rahmen der sich anschliessenden Regeneration garantiert.

Neben den bereits erwähnten Vorteilen des erfindungsgemässen Transformationsverfahrens gegenüber den bisher bekannten Verfahen sind als weitere Verfahrensvorteile zu nennen:

a) eine stark verkürzte Regenerationszeit, da die Regeneration über eine direkte Embryogenese erfolgt und die sonst übliche Kallus-Phase sowie die morphogenetische Induktionsphase der in vitro Kulturen entfällt

b) sofern die mikroinjizierten Strukturen einer sekundären Embryogenese oder einer sekundären Adventivsprossbildung zugänglich sind, ist es möglich, eine in vitro Segregation der erhaltenen chimären Transformanten sowie

c) eine in vitro Vermehrung der erhaltenen Transformanten vorzunehmen.

d) bis zu 100%ige Ueberlebensrate der transformierten, mikroinjizierten Strukturen aufgrund einer schonenden Injektionstechnik.

Damit ist es im Rahmen der vorliegenden Erfindung erstmals möglich, unabhängig von den zuvor genannten Beschränkungen mit hoher Effizienz transgene Pflanzen, insbesondere auch solche aus der Gruppe der Monokotyledoneae, mit neuen und wünschenswerten Eigenschaften zu erzeugen, indem man eine DNA-haltige Lösung in zygotische pflanzliche Proembryonen bzw. Embryonen mit hohem Regenerierungspotential mikroinjiziert, die mikroinjizierten zygotischen Proembryonen bzw. Embryonen in geeigneten, die Ausbildung embryoider Strukturen fördernden Kulturmedien mikrokultiviert und aus den so erhaltenen, in der Regel chimären Regeneraten gegebenenfalls nicht-chimäre transgene Pflanzen herstellt.

Im einzelnen umfasst das erfindungsgemässe Verfahren die folgenden spezifischen Verfahrensschritte:

a) Isolierung einzelner, für die Mikroinjektion geeigneter mehr- bis vielzelliger zygotischer Pro-/Embryonen aus den Samenanlagen von Donor-Pflanzen;

b) Selektion der isolierten zygotischen Pro-/Embryonen für die sich anschliessende Mikroinjektion und Einbringen der selektionierten Pro-/Embryonen in ein geeignetes Kulturmedium;

c) Mikroinjektion einer DNA-haltigen Lösung, enthaltend eine oder mehrere DNA-Fragmente in die unter a) isolierten zygotischen Pro-/Embryonen;

$d_1$) Mikrokultivierung der mikroinjizierten zygotischen Pro-/Embryonen in geeigneten, eine direkte Embryogenese fördernden Kulturmedien;

$d_2$) Alternativ: Kultivierung der injizierten Pro-/Embryonen unter Bedingungen, die die Entwicklung einer morphogenen Zellkultur oder die Bildung sekundärer Embryonen ermöglichen; und

f) Regeneration besagter transformierter zygotischer Pro-/Embryonen zu vollständigen in der Regel chimären Pflanzen.

g) Herstellung nicht-chimärer transgener Pflanzen mit neuen und wünschenswerten Eigenschaften.

Donorpflanzen, die als Ausgangsmaterial für die Isolierung zygotischer Proembryonen und Embryonen in Frage kommen, können sowohl unter Freilandbedingungen als auch im Gewächshaus herangezogen werden. Bei der Verwendung von Pflanzen, die zur Induktion der Blütenbildung einer Vernalisation bedürfen, wie z.B. Weizen, Gerste, Hafer, Roggen, Mais etc. als Donorpflanzen, ist darauf zu achten, dass diese Sorten zweckmässigerweise einer Kälteperiode ausgesetzt werden, die zwischen 1 bis 5 Wochen betragen kann, um einen optimalen Blütenansatz und in der Folge eine optimale Ausbildung der Samenanlagen zu gewährleisten. Der Zeitpunkt der Ernte der Donorpflanzen hängt von der verwendeten Pflanzenart sowie von den klimatischen Bedingungen ab, unter denen die Anzucht der Pflanzen erfolgt. In der Regel werden die Donor-Pflanzen 2 bis 20 Tage nach der erfolgten Bestäubung geerntet, insbesondere aber dann, wenn die zygotischen Embryonen ein geeignetes Entwicklungsstadium erreicht haben, das sich zwischen der frühen Proembryonalphase und der Embryonenphase, vorzugsweise zwischen dem 2-Zellstadium und dem ca. 5000-Zellstadium, erstreckt (vgl. Abb. 1 und 2).

Die Isolierung der zygotischen Proembryonen und Embryonen wird vorzugsweise mit Hilfe feiner Skalpelle sowie feiner Nadeln oder fein ausgezogener Pinzetten unter mikroskopischer Kontrolle durchgeführt.

Die Isolierung kann beispielsweise durch Herausschneiden des den Embryosack enthaltenen Nucellusgewebes und anschliessende Präparation des Embryosackes erfolgen, wobei der Pro-/Embryo aus dem isolierten Embryosack herauspräpariert wird. Darüberhinaus kann selbstverständlich auch jedes andere brauchbare Isolierungsverfahren eingesetzt werden.

Die auf diese Weise gewonnenen zygotischen Proembryonen und Embryonen werden anschliessend in einen Tropfen eines flüssigen Kulturmediums überführt. Die sich anschliessende Selektionierung von für eine Mikroinjektion geeigneten Embryonalstadien erfolgt vorzugsweise ebenfalls unter optischer Kontrolle, wobei in der Regel ein Stereomikroskop oder ein Inversionsmikroskop sowie für eine Mikroselektion zellulärer Strukturen geeignete, dem Fachmann bekannte Selektionssysteme verwendet werden, wie sie beispielsweise bei Koop und Schweiger (1985), Spangenberg (1986) oder bei Schweiger et al (1987) beschrieben sind.

Bevorzugt für die sich anschliessende Mikroinjektion sind Embryonalstadien, die von frühen globulären Proembryonalstadien bis hin zu frühen Embryonenstadien reichen. Insebesondere sind an dieser Stelle Embryonalstadien zu nennen, die 2 bis ca. 5000 Zellen umfassen.

Da eine gute Mikroinjizierbarkeit aufgrund nur geringer Zellzahlen in frühen Proembryonalstadien häufig mit einer geringen Regenerationshäufigkeit der mikorinjizierten Strukturen einhergeht, sollten bei der Auswahl der optimalen Stadien möglichst weitere Kriterien mitberücksichtigt werden, wie z.B. eine gute Isolierbarkeit der Proembryonal- bzw. Embryonalstadien sowie eine gute Regenerationsfähigkeit.

Im Falle der Gramineen sind daher neben frühen golbulären Proembryonalstadien mit Scutellum (vgl. Abb. 1, Phase 17 bis 25), insbesondere frühe Embryonalstadien (vgl. Abb. 1, Phase 25 bis 30) als besonders geeignet für die Verwendung in dem erfindungsgemässen Verfahren anzusehen, da sich letztere neben einer guten Isolierbarkeit auch durch eine hohe Regeneratiosfähigkeit auszeichnen. Darüberhinaus ist auch eine gute Mikroinjizierbarkeit gewährleistet, da der für die Mikroinjektion vorgesehene und bevorzugte Bereich (Sprossmeristemanlage) aus nur relativ wenigen Zellen besteht. Ganz besonders bevorzugt im Rahmen dieser Erfindung sind daher frühe Embryonalstadien, die ausser dem Scutellum bereits eine gut erkennbare Spross- und Wurzelmeristemanlage aufweisen (vgl. Abb. 1, Phase 26-27).

Auch für die Einschleusung genetischen Materials in Pflanzen aus der Gruppe der Dicotyledoneae via Mikroinjektion sind insbesondere frühe globuläre Proembryonalstadien (vgl. Abb. 2, Phase O, P, Q) sowie frühe Embryonalstadien (früh- bis spät-Kotelydonar-Stadien) (vgl. Abb. 2, Phase R, S, T) besonders geeignet.

Das Auffinden bzw. Auswählen des für die jeweilige Zielpflanze optimalen Entwicklungsstadiums als Ausgangsmaterial für das erfindungsgemässe Verfahren ist für den Fachmann auf diesem Gebiet aufgrund der vorliegenden, detaillierten Beschreibung ohne unzumutbaren experimentellen Aufwand möglich.

Die Mikroinjektion der DNA-haltigen Lösung in zygotische Proembryonen bzw. Embryonen kann mit Hilfe an sich bekannter Verfahren, die u.a. bei Steinbiss and Stabel, 1983; Neuhaus et al., 1984, 1986; Lawrence and Davies, 1985; sowie bei Morikawa and Jamada, 1985 beschrieben sind, durchgeführt werden.

Im einzelnen kann die Mikroinjektion der pflanzlichen Pro-/Embryonen dabei unter Verwendung einer spezifischen Mikroinjektionsapparatur durchgeführt, deren Kernstück durch Mikromanipulatoren steuerbare Mikropipetten darstellt.

Dabei handelt es sich zum einen um die eigentliche Injektionspipette, deren äusserer Durchmesser zwischen 1,00 mm und 2,00 mm beträgt. Bevorzugt ist ein Durchmesser von 1,2 mm bis 1,5 mm. Der Spitzendurchmesser der Injektionskapillare liegt dagegen bei Werten zwischen $\leq$ 1 $\mu$m und 10 $\mu$m. Ganz besonders geeignet für die Anwendung in dem erfindungsgemässen Verfahren sind Injektionskapillaren mit Spitzendurchmessern zwischen 0,5 $\mu$m und 1 $\mu$m. Man verwendet im Rahmen der vorliegenden Erfindung als Mikroinjektionskapillare vorzugsweise Mikroelektroden kapillaren mit eingeschmolzenen Glasfilamenten aus Borosilikatglas. Selbstverständlich können auch alle anderen, für die Durchführung der Mikroinjektion geeigneten Glasarten oder sonstigen Materialien verwendet werden. Diese Mikroinjektionspipette ist in der Regel über eine Druckregulationseinheit, den sog. Mikroinjektor, mit einer Gasquelle, insbesondere einer Stickstoffquelle oder einer Druckluftquelle verbunden, welche einen sehr fein dosierten Uebertritt der Injektionslösung aus der Mikropipette in das pflanzliche Gewebe gewährleistet. Eine zweite Pipette, die sogenannte Halte-Pipette, dient der Fixierung der zygotischen Embryonen während des Mikroinjektionsvorganges.

Diese Halte-Pipetten haben in der Regel eine schüsselförmige Spitze mit einem Durchmesser zwischen 0,02 mm und 1,00 mm, vorzugsweise zwischen 0,1 mm und 0,5 mm. Durch Anlegen eines schwachen negativen oder positiven Druckes kann man die pflanzlichen Pro-/Embryonen ansaugen und somit fixieren bzw. nach der erfolgten Mikroinjektion der DNA-haltigen Lösung wieder freisetzen. Eine dritte Mikropipette, die sogenannte Sammelpipette, dient dem Einsammeln der mikroinjizierten Embryonen und deren Ueberführung auf geeignete Mikrokulturplatten.

Geeignete Sammelpipetten weisen in der Regel einen inneren Durchmesser zwischen 0,1 mm und 1,0 mm auf, sodass ein störungsfreies Aufsaugen der mikroinjizierten Embryonen gewährleistet ist.

Der eigentliche Mikroinjektionsvorgang beginnt üblicherweise mit dem Einbringen der zuvor isolierten und ausgewählten Proembryonen oder Embryonen in 10 $\mu$l bis 200 $\mu$l eines geeigneten Kulturmediums auf speziell vorbereitete Deckgläser und dem Ansaugen eines geeigneten Pro-/Embryos mit Hilfe der Halte-Pipette, wobei dieser solange durch Freisetzen und anschliessendes Wiederansaugen in seiner Orientierung in bezug auf die Injektionspipette variiert werden kann, bis eine für die Mikroinjektion optimale Ausrichtung des Pro-/Embryos erreicht ist.

Nach dem Eindringen der Mikropipettenspitze in das Embryonalgewebe der Proembryonen oder in das Gewebe der Embryonen erfolgt die Freisetzung der DNA-haltigen Injektionslösung.

Bei der Verwendung von Embryonen ist eine Injektion der DNA-haltigen Lösung in das Gewebe der Sprossmeristemanlage bevorzugt.

Das Injektionsvolumen schwankt in Abhängigkeit von der Zellgrösse. Es liegt üblicherweise zwischen 1 pl und 100 pl pro injizierter Zelle.

7

Bevorzugt im Rahmen dieser Erfindung ist ein Injektionsvolumen von 20 pl bis 40 pl pro injizierter Zelle.

Um eine möglichst gleichmässige Transformation aller Zellen zu gewährleisten, die am Aufbau der zu injizierenden multizellulären Struktur beteiligt sind, werden diese in Abhängigkeit von der vorhandenen Zellzahl zwischen 1 bis 1000 mal, vorzugsweise aber zwischen 4 bis 60 mal injiziert.

Dies kann erfindungsgemäss dadurch erreicht werden, dass die zu injizierenden zygotischen Proembryonen und Embryonen zwischen den verschiedenen Injektionsvorgängen gedreht werden, sodass eine möglichst grosse Anzahl von verschiedenen Zellen getroffen wird.

Als DNA-Lösungen, die für eine Mikroinjektion in pflanzliche Zellen geeignet sind, kommen beispielsweise Pufferlösungen in Frage, welche die transformierende DNA in einer geeigneten Konzentration enthalten.

Die im Rahmen dieser Erfindung bevorzugte DNA-Konzentration liegt bei 0,1 $\mu g/\mu l$ bis 10 $\mu g/\mu l$, insbesondere aber bei 0,5 $\mu g/\mu l$ bis 5 $\mu g/\mu l$. Ganz besonders bevorzugt ist eine DNA-Konzentration von 0,5 $\mu g/\mu l$ bis 1,5 $\mu g/\mu l$

Der pH der Pufferlösung kann zwischen Werten von pH 7 bis pH 8 schwanken; erfindungsgemäss bevorzugt ist ein pH-Bereich zwischen pH 7,5 und pH 7,8.

Für die Integration des Fremdgens in die genomische DNA der Pflanzenzelle ist es vorteilhaft, wenn die transformierende DNA von neutralen DNA-Sequenzen (Träger-DNA) flankiert wird. Unter neutralen DNA-Sequenzen sollen im Rahmen dieser Erfindung insbesondere solche Sequenzen verstanden werden, die mit der Funktion der zu übertragenden, transformierenden DNA nicht in Zusammenhang stehen sondern lediglich für die Integration der eingeschleusten DNA ins pflanzliche Genom von Bedeutung sind. Die die transformierende DNA flankierenden neutralen DNA-Sequenzen können sowohl synthetischen Ursprungs sein als auch durch Behandlung mit geeigneten Restriktionsenzymen aus natürlich vorkommenden DNA-Sequenzen gewonnen werden. So sind beispielsweise als Träger-DNA natürlich vorkommende Plasmide geeignet, die mit einem selektiven Restriktionsenzym geöffnet worden sind. Die zur Genübertragung hergestellte DNA-Sequenz kann aber auch eine ringförmige Struktur (Plasmidstruktur) haben. Solche Plasmide bestehen aus einem DNA-Strang, in den das Fremdgen mit den Expressionssignalen integriert ist.

Ein Beispiel für ein derartiges Plasmid ist das frei erhältliche Plasmid pUC8 (beschrieben in Messing, J. und J. Vieira, Gene 19, 269-276, 1982). Weiter sind als Träger-DNA auch Bruchstücke natürlich vorkommender Plasmide verwendbar. Beispielsweise ist als Träger DNA die Deletionsmutante für Gen VI des Cauliflower-Mosaik-Virus einsetzbar.

Die transformierende DNA kann erfindungsgemäss sowohl in Form offener oder geschlossener Ringe (Plasmid-Struktur) vorliegen, sie kann eine überspialisierte oder aber bevorzugterweise eine linearisierte Form aufweisen.

Bevorzugt im Rahmen dieser Erfindung ist die Verwendung eines Gemisches aus linearisierter und überspiralisierter Form.

Die zygotischen Proembryonen und Embryonen können dann nach erfolgter Mikroinjektion mit Hilfe der Sammel-Pipette aufgesaugt und in ein geeignetes Kulturmedium überführt werden.

Die optische Kontrolle des gesamten Mikroinjektionsvorganges erfolgt üblicherweise unter mikroskopischer Beobachtung. Bevorzugt sind inverse Mikroskope oder Stereomikroskope mit einer Kombination von Auf- und Durchlicht.

Die gewählte Vergrösserung hängt dabei von der Grösse des zu injizierenden Materials ab und schwankt zwischen 10 bis 800 facher Vergrösserung.

Die sich an die Mikroinjektion anschliessende in vitro Kultivierung der transformierten Proembryonen und Embryonen wird vorzugsweise in Kultivierungsmedien durchgeführt, die für die weitere Entwicklung der Embryonen bis hin zur vollständig ausgebildeten Pflanze geeignet sind.

Die Zusammensetzung dieser Medien zeichnet sich in erster Linie dadurch aus, dass sie vor allem die für das Wachstum und die Entwicklung der pflanzlichen Zelle essentiellen mineralischen Stoffe bzw. Elemente in ihren Verbindungen (Makroelemente) enthalten, wie z.B. Stickstoff, Phosphor, Schwefel, Kalium, Calcium, Magnesium und Eisen, neben den sogenannten Spurenelementen (Mikroelemente) wie z.B. Natrium, Zink, Kupfer, Mangan, Bor, Vanadium, Selen und Molybdän. Während die Makroelemente in der Regel in Mengen zwischen 10 mg/l und einigen Hundert mg/l vorliegen, beträgt die Konzentration der Mikroelemente im allgemeinen maximal einige mg/l.

Weitere Bestandteile besagter Medien umfassen eine Reihe von essentiellen Vitaminen wie Nikotinsäure, Pyridoxin, Thiamin, Inosit, Biotin, Liponsäure, Riboflavin, Ca-Pantothenat u.a.m., eine geeignete leicht assimilierbare Kohlenstoffquelle, wie beispielsweise Saccharose oder Glucose, sowie verschiedene Wachstumsregulatoren in Form natürlicher oder synthetischer Phytohormone aus der Klasse der Auxine und Cytokinine im Konzentrationsbereich von 0,01 mg/l bis 20 mg/l.

Dabei ist erfindungsgemäss besonders darauf zu achten, dass sich die Zusammensetzung der Medien sowie die Konzentrationsverhältnisse ihrer einzelnen Komponenten in Abhängigkeit vom Entwicklungsstand der Embryonen ändern kann. Dies trifft in erster Linie auf die verwendeten Wachstumsregulatoren zu, die in einem ausgewogenen Verhältnis zueinander eingesetzt werden müssen, sodass, falls dies gewünscht wird, ein kontrollierter Ablauf der direkten Embryogenese gewährleistet ist.

Bevorzugt im Rahmen dieser Erfindung ist ein Konzentrationsbereich der Phytohormone aus der Klasse der Auxine und der Cytokinine zwischen 0,01 mg/l und 20 mg/l, insbesondere aber zwischen 0,8 mg/l und 1,2 mg/l.

Die Kulturmedien sind darüberhinaus osmotisch stabilisiert durch Zusatz von Zuckeralkoholen (beispielsweise Mannit), Zucker (beispielsweise Glucose) oder Salzionen und sind auf pH-Werte von 4,5 bis 7,5, vorzugsweise von 5,2 bis 6,5, eingestellt.

Neben der Verwendung synthetischer Medien mit einer genau definierten Zusammensetzung aus verschiedenen Einzelkomponenten bekannter Konzentration können erfindungsgemäss auch komplexe Nährmedien verwendet werden, die ganz oder aber zumindest teilweise aus natürlichen Komponenten zusammengesetzt sind.

In diesem Zusammenhang sei beispielhaft auf Kartoffelextrakt, Kokosnussmilch sowie auf flüssiges Endosperm verwiesen, das durch Absaugen aus den Samenanlagen von Pflanzen gewonnen werden kann.

Während die ersten Kultivierungsschritte, die sich unmittelbar an die Mikroinjektion anschliessen, vorzugsweise in flüssigen Kulturmedien, die gegebenenfalls ein verfestigtes Kulturmedium überlagern können, durchgeführt werden, kann im weiteren Verlauf der Embryonalentwicklung wahlweise sowohl auf flüssige als auch auf feste Kulturmedien, die durch Zusatz eines der üblicherweise in der mikrobiologischen Technik verwendeten Verfestigungsmittel hergestellt werden können, oder aber auf eine Kombination aus festen und flüssigen Medien zurückgegriffen werden.

Geeignete Verfestigungsmittel umfassen beispielsweise Agar, Agarose, Alginat, Pektinat, Gelrite®, Gelatine u.a. Bevorzugt sind Agar und Agarose, insbesondere Agar und Agarose vom "Low Melting Type" (LMT).

Bei der Verwendung flüssiger Medien kann es gegebenenfalls zweckmässig sein, dem Medium ein osmotisch neutrales Verdickungsmedium zuzusetzen, sodass die Dichte des Mediums zunimmt, ohne dadurch den osmotischen Wert zu erhöhen.

Dies führt dazu, dass die Embryonen auf dem Medium aufschwimmen und somit ein optimaler Gasaustausch mit der umgebenden Atmosphäre gewährleistet ist.

Bevorzugt im Rahmen dieser Erfindung sind synthetische Polymere, so z.B. Co-Polymere aus Saccharose und Epichlorhydrin mit einem Molekulargewicht zwischen 70 000 und 400 000, ohne jedoch darauf beschränkt zu sein.

Die auf die zuvor beschriebene Weise mikroinjizierten Proembryonen bzw. Embryonen werden unmittelbar nach erfolgter Mikroinjektion vorzugsweise auf handelsübliche Mikrokultivierungsplatten transferiert und zwar in Medien, die eine für die in vitro Kultivierung zygotischer Proembryonen und Embryonen geeignete Zusammensetzung aufweisen.

Die Mikrokultivierung der mikroinjizierten zygotischen Proembryonen bzw. Embryonen wird vorzugsweise in einem flüssigen Kulturmedium durchgeführt, das in Abhängigkeit von den spezifischen Erfordernissen des verwendeten Pflanzenmaterials mit einem osmotisch neutralen Verdickungsmittel versetzt werden kann und das gegebenenfalls einem Festmedium überlagert sein kann.

Die Kultivierungsdichte liegt vorzugsweise zwischen 1 Proembryo bzw. Embryo/$\mu$l und 50 Proembryonen bzw. Embryonen/$\mu$l Kulturmedium, ganz besonders bevorzugt aber zwischen 1 Proembryo-bzw. Embryo/$\mu$l und 5 Proembryonen bzw. Embryonen/$\mu$l Kulturmedium.

Die gewählte Kultivierungsdichte ist abhängig von der Grösse und dem Entwicklungsstand der verwendeten Pro-/Embryonen.

Es erweist sich im Rahmen dieser Erfindung als vorteilhaft, wenn die Kultivierung der mikroinjizierten Proembryonen bzw. Embryonen zumindest zeitweise in einem 2 Kompartimentensystem durchgeführt wird, das als feuchte Kammer fungiert, wobei das äussere Kompartiment beispielsweise von Wasser, von Kulturmedium oder von einer wässrigen Mannit-Lösung gebildet wird, während sich die Embryonen im innern Kompartiment befinden.

Es ist weiterhin vorteihaft, die mikroinjizierten Proembryonen bzw. Embryonen alle 1 bis ca. 12 Tage, vorzugsweise aber alle 2-3 Tage in frisches Kulturmedium zu transferieren bzw. mit frischem Kulturmedium zu überschichten, wobei das Medienvolumen von der Grösse der Embryonen abhängt und in der Regel zwischen 0,5 $\mu$l und 10 $\mu$l beträgt und dabei ein Verhältnis Embryo:Medium von 1:0,2 $\mu$l bis 1:10 $\mu$l vorliegt.

Wenn die auf die zuvor beschriebene Weise mikrokultivierten Embryonen ein bestimmtes Entwicklungsstadium erreicht haben, in der Regel nach 10 bis 30 Tagen, werden die Embryonen zur Differenzierung der

9

Spross- und Wurzelscheitel auf feste Medien transferiert, die gegebenenfalls von einem Flüssigmedium überlagert sein können. In der Regel handelt es sich dabei um die üblicherweise für die Regeneration von Pflanzen verwendeten Kulturmedien, die aufgrund ihres ausgewogenen Gehaltes an Phytohormonen, insbesondere aus der Klasse der Auxine und Cytokinine, eine Differenzierung des Spross- und/oder Wurzelwachstums hervorrufen.

Nach einer Kultivierungsdauer von ca. 4 bis 8 Wochen, je nach verwendetem Pflanzenmaterial, spätestens aber nach dem Auskeimen der Embryonen, können diese auf Festmedien übertragen werden, die keine oder aber nur eine geringe Hormonkonzentration aufweisen und hier bis zur Ausbildung von kleinen Pflänzchen (Keimlinge) weiterkultiviert werden.

Je nach verwendetem Pflanzenmaterial kann es vorteilhaft sein, eine Induktion der Wurzelbildung auszulösen, z.B. durch Uebertragung der Embryonen auf an sich bekannte Wurzel-induzierende Medien [vgl. z.B. Medium S4 (Tabelle 9); H4 (Tabelle 10)].

Die auf diese Weise gebildeten transgenen, in der Regel chimären Pflänzchen können dann in Erde gepflanzt und in gleicher Weise wie normale Sämlinge weiterbehandelt werden.

Aus den auf die zuvor beschriebene Weise erhaltenen transgenen Chimären können dann unter Anwendung an sich bekannter Verfahrensmassnahmen sehr einfach nicht-chimäre transgene Pflanzen hergestellt werden.

Für die Herstellung nicht-chimärer transgener Pflanzen stehen grundsätzlich zwei alternative Wege zur Verfügung.

a) Bei Pflanzen, die einer sekundären Embryogenese oder einer sekundären Adventivspross-Bildung zugänglich sind, ist es möglich, in vitro die sekundären Strukturen , die sich aus Einzelzellen ableiten, von den primär entstandenen, in der Regel chimären Regeneranten zu trennen und zu vereinzeln und somit reine transgene Pflanzen zu erhalten. Dies ist besonders einfach, wenn das oder eines der übertragenen Fremdgene die Anwendung selektiver Kultiverungsbedingungen gestattet, sodass nur die transgenen Zellen überleben.

Die transgenen Regenerate können dann auf die zuvor beschriebene Weise kultiviert und zu ganzen Pflanzen regeneriert werden.

b) Homogen transformierte (nicht-chimäre) Pflanzen können andererseits aber auch durch gegebenenfalls wiederholte Selbstbestäubung von primären transgenen Chimären gewonnen werden. Da die sexuelle Nachkommenschaft aus einer Einzelzelle, nämlich der befruchteten Eizelle hervorgeht, wird ein Teil dieser Nachkommen aus vollständigen,transgenen Pflanzen bestehen und zwar dann, wenn der transgene Bereich der Chimäre an der Bildung der Pollen oder Eizellen beteiligt ist.

Das zuvor beschriebene Verfahren zur Herstellung transgener Pflanzen mit Hilfe der Mikroinjektionstechnologie ist mit allen seinen Teilschritten Bestandteil der vorliegenden Erfindung.

Das erfindungsgemässe Verfahren ist praktisch universell anwendbar, da praktisch alle Pflanzen zygotische Embryonen bilden. Es umfasst sowohl die Mikroinjektion von natürlichen DNA-Sequenzen als auch von artifiziell mit Hilfe der rekombinanten DNA-Technologie erzeugten hybriden Genkonstruktionen.

In erster Linie umfasst vom breiten Umfang der vorliegenden Erfindung sind rekombinante DNA-Moleküle, die DNA-Sequenzen enthalten, welche bei den Transformanten zu nützlichen und wünschenswerten Eigenschaften führen.

Dabei handelt es sich vorzugsweise um rekombinante DNA-Moleküle, die eine oder mehrere Gensequenzen beinhalten, die eine nützliche und wünschenswerte Eigenschaft kodieren und die unter der regulatorischen Kontrolle von in Pflanzen aktiven Expressionssignalen stehen und mit diesen in operabler Weise verknüpft sind, sodass eine Expression besagter Gensequenzen in der transformierten pflanzlichen Zelle gewährleistet ist.

Für die Verwendung in dem erfindungsgemässen Verfahren sind somit in erster Linie alle diejenigen Gene geeignet, die in der pflanzlichen Zelle exprimiert werden und die der Pflanze eine nützliche und/oder gewünschte Eigenschaft verleihen, wie z.B. eine erhöhte Resistenz gegenüber Pathogenen (beispielsweise gegenüber phytophathogenen Pilzen, Bakterien, Viren etc.), eine Resistenz gegenüber Chemikalien [beispielsweise gegenüber Herbiziden (wie z.B. Triazinen, Sulfonylharnstoffen, Imidazolinonen, Triazolpyrimidinen, Bialaphos, Glyphosate, etc.), Insektiziden oder anderen Bioziden]; Resistenz gegenüber nachteiligen (endaphischen oder atmosphärischen) Umwelteinflüssen (beispielsweise gegenüber Hitze, Kälte, Wind, besondere, extreme Bodenbeschaffenheit, Feuchtigkeit, Trockenheit, osmotischer Stress etc.); oder aber zu einer erhöhten oder aber qualitativ besseren Bildung von Reserve- oder Speicherstoffen in Blättern, Samen, Knollen, Wurzel, Stengeln, etc. führen. Zu den wünschenswerten Substanzen, die von transgenen Pflanzen produziert werden können, zählen beispielsweise Proteine, Stärken, Zucker, Aminosäuren, Alkaloide, Riechstoffe, Farben, Fette, etc.

Ebenso werden von der vorliegenden Erfindung Gene umfasst, die pharmazeutisch akzeptable Wirksubstanzen, wie z.B. Alkaloide, Steroide, Hormone, Immunmodulatoren, und andere physiologisch aktive Substanzen kodieren.

Eine Resistenz gegenüber Cytotoxinen kann beispielsweise durch Übertragung eines Gens erreicht werden, das in der Lage ist, bei der Expression in der pflanzlichen Zelle, ein Enzym zur Verfügung zu stellen, welches das Cytotoxin detoxifiziert, wie z.B. die Neomycinphosphotransferase Typ II oder die Aminoglycosidphosphotransferase Typ IV, die zu einer Detoxifikation von Kanamycin, Hygromycin und anderen Aminoglykosidantibiotika beitragen oder eine Glutathion-S-Transferase, Cytochrom P-450 oder andere katabolisch wirksame Enzyme, von denen bekannt ist, dass sie Triazine, Sulfonylharnstoffe oder andere Herbizide detoxifizieren. Eine Resistenz gegenüber Cytotoxinen kann auch durch ein Gen vermittelt werden, das in einer Pflanze eine bestimmte Form eines 'Zielenzyms' (Angriffspunkt der Cytotoxinwirkung) exprimiert, die resistent ist gegen die Aktivität des Cytotoxins, wie z.B. eine Variante der Acetohydroxysäuresynthase, die gegenüber der inhibitorischen Wirkung von Sulfonylharnstoffen, Imidazolinonen oder anderen Herbiziden, die mit diesem spezifischen Stoffwechselschritt interagieren, insensitiv ist; oder eine Variante der EPSP Synthase, die sich gegenüber der Hemmwirkung von Glyphosate als insensitiv erweist. Es kann sich als vorteilhaft erweisen, diese veränderten Zielenzyme in einer Form zu exprimieren, die ihren Transport in das korrekte zelluläre Kompartiment, wie z.B. im obigen Fall in die Chloroplasten, erlaubt.

In bestimmten Fällen kann es von Vorteil sein, die Genprodukte in die Mitochondrien, die Vakuolen, das endoplasmatische Retikulum oder in andere Zellregionen, möglicherweise sogar in die interzellulären Räume (Apoplasten) zu dirigieren.

Eine Resistenz gegenüber bestimmten Pilzklassen kann beispielsweise durch die Einschleusung eines Gens erreicht werden, das Chitinase in den pflanzlichen Geweben exprimiert. Zahlreiche pflanzenpathogene Pilze enthalten Chitin als integralen Bestandteil ihre Hyphen- und Sporenstrukturen, so z.B. die Basidiomyceten (Brand- und Rostpilze), Asomyceten und *Fungi Imperfecti* (einschliesslich *Alternaria* und *Bipolaris*, *Exerophilum turcicum*, *Colletotricum*, *Gleocercospora* und *Cercospora*). Chitinase ist in der Lage das Mycelwachstum bestimmter Pathogene in-vitro zu hemmen. Ein pflanzliches Blatt oder eine Wurzel, die Chitinase konstruktiv oder aber als Antwort auf das Eindringen eines Pathogens exprimiert, ist gegen den Angriff einer grossen Zahl verschiedener Pilze geschützt. Je nach Situation kann eine konstitutive Expression vorteilhaft sein im Vergleich zu einer induzierbaren Expression, die bei zahlreichen Pflanzen als normale Reaktion auf einen Pathogenbefall auftritt, da die Chitinase unmittelbar in hoher Konzentration vorliegt, ohne dass eine lag-Phase für die Neusynthese abgewartet werden muss.

Eine Resistenz gegenüber Insekten kann beispielweise durch ein Gen übertragen werden, das ein Polypeptid kodiert, welches toxisch ist für Insekten und/oder deren Larven, wie z.B. das kristalline Protein von *Bacillus thuringiensis* [Barton et al, 1987; Vaeck *et al*, 1987]. Eine zweite Klasse von Proteinen, die eine Insektenresistenz vermitteln, sind die Proteaseinhibitoren. Proteaseinhibitoren bilden einen gewöhnlichen Bestandteil von pflanzlichen Speicherstrukturen (Ryan, 1973). Es konnte nachgewiesen werden, dass ein aus Sojabohnen isolierter und gereinigter Bowman-Birk Proteaseinhibitor die Darmprotease von Tenebrio-Larven hemmt (Birk *et al*, 1963). Das Gen, welches den Trypsininhibitor aus der Kuherbse kodiert, ist bei Hilder *et al*, (1987) beschrieben.

Ein Gen, das einen Proteaseinhibitor kodiert, kann in einem geeigneten Vektor unter die Kontrolle eines Pflanzenpromotors, insbesondere eines konstruktiven Promotors, wie z.B. den CaMV 35S Promotor [der bei Odell *et al*, (1985) beschrieben ist], gebracht werden. Das Gen, beispielsweise die kodierende Sequenz des Bowman-Birk Proteaseinhibitors aus der Sojabohne, kann mit Hilfe der bei Hammond *et al*, (1984) beschriebenen cDNA Klonierungsmethode erhalten werden. Eine weitere Möglichkeit zur Herstellung eines Proteaseinhibitors besteht in dessen synthetischer Herstellung, sofern dieser weniger als 100 Aminosäuren aufweist, wie z.B. der Trypsininhibitor der Limabohne. Die kodierende Sequenz lässt sich durch Zurückübersetzen der Aminosäuresequenz voraussagen. Zusätzlich werden an beiden Enden Restriktionsschnittstellen eingebaut, die für den jeweils gewünschten Vektor geeignet sind. Das synthetische Gen wird durch Synthese überlappender Oligonukleotidfragmente von 30 bis 60 Basenpaaren hergestellt, indem diese zunächst einer Kinasereaktion unterworfen, dann miteinander verknüpft (Maniatis *et al*, 1982) und schliesslich in einem passenden Vektor kloniert werden. Mit Hilfe der DNA Sequenzierung kann dann ein Klon, der das Insert in einer korrekten Orientierung aufweist, identifiziert werden. Für die Enschleusung in die Embryonen wird isolierte Plasmid-DNA verwendet.

Ebenfalls umfasst von der vorliegenden Erfindung sind Gene, die pharmazeutisch aktive Bestandteile kodieren, z.B. Alkaloide, Steroide, Hormone und andere physiologisch aktive Substanzen sowie Flavine, Vitamine und Farbstoffe. Zu den Genen, die im Rahmen dieser Erfindung in Betracht gezogen werden, gehören daher, ohne jedoch darauf beschränkt zu sein, pflanzenspezifische Gene wie z.B. das Zeingen (Wienand *et al*, 1981), Säuger-spezifische Gene wie das Insulingen, das Somatostatingen, die Interleucingene, das t-PA Gen (Pennica *et al*, 1983) etc., oder aber Gene mikrobiellen Ursprungs wie das NPT II Gen sowie synthetische Gene wie das Insulingen (Itakura *et al*, 1975).

Neben natürlicherweise vorkommenden Genen, die eine nützliche und wünschenswerte Eigenschaft kodieren, können im Rahmen dieser Erfindung auch Gene verwendet werden, die zuvor mit Hilfe chemischer oder gentechnologischer Methoden gezielt modifiziert wurden.

Weiterhin sind von dem breiten Konzept der vorliegenden Erfindung auch solche Gene umfasst, die vollständig durch chemische Synthese hergestellt werden.

Weiterhin geeignet für die Verwendung in dem erfindungsgemässen Verfahren sind sonstige nützliche DNA-Sequenzen, insbesondere nicht-kodierende DNA-Sequenzen mit regulatorischen Funktionen. Diese nicht-kodierenden DNA-Sequenzen sind beispielsweise in der Lage die Transkription einer assoziierten DNA-Sequenz in pflanzlichen Geweben zu regulieren.

In diesem Zusammenhang ist z.B. der Promotor des hps70 Gens zu nennen, der durch einen Hitzeschock induziert werden kann (Spena et al, 1985); die 5′-flankierende Sequenz der nukleär kodierten Sequenz der kleinen Untereinheit des Ribulose-1,5-biphosphatcarboxylase (RUBISCO) Gens [Morelli et al, 1985] oder des Chlorophyll a/b Bindungsproteins, die durch Licht induzierbar sind; oder die 5′-flankierende Region des Alkoholdehydrogenase (adh1-s)Gens aus Mais, die unter anaeroben Bedingungen die Transkription eines assoziierten Reportergens [z.B. Chloramphenicolacetyltransferase-Gen (CAT)] auslöst (Howard et al, 1977).

Eine weitere Gruppe regulierbarer DNA-Sequenzen betrifft chemisch regulierbare Sequenzen, die z.B. in den PR-("pathogenesis related proteine")Proteingenen des Tabaks vorliegen und mit Hilfe chemischer Regulatoren induzierbar sind.

Die zuvor beispielhaft genannten regulierbaren DNA-Sequenzen können sowohl natürlichen als auch synthetischen Ursprungs sein oder aber aus einem Gemisch von natürlichen und synthetischen DNA-Sequenzen bestehen.

Als Gene oder DNA-Sequenzen, die im Rahmen der vorliegenden Erfindung Verwendung finden können, kommen sowohl homologe als auch heterologe Gen(e) oder DNA sowie synthetische Gen(e) oder DNA gemäss der im Rahmen der vorliegenden Erfindung gemachten Definition in Betracht.

Die DNA-Sequenz kann ausschliesslich aus genomischer, aus cDNA bzw. synthetischer DNA konstruiert sein. Eine andere Möglichkeit besteht in der Konstruktion einer hybriden DNA-Sequenz bestehend sowohl aus cDNA als auch aus genomischer DNA und/oder synthetischer DNA.

In diesem Fall kann die cDNA aus demselben Gen stammen wie die genomische DNA oder aber, sowohl die cDNA als auch die genomische DNA können aus verschiedenen Genen stammen. In jedem Fall können aber sowohl die genomische DNA und/oder die cDNA, jede für sich, aus dem gleichen oder aus verschiedenen Genen hergestellt sein.

Wenn die DNA-Sequenz Anteile von mehr als einem Gen beinhaltet, können diese Gene entweder von ein und demselben Organismus, von mehreren Organismen, die verschiedenen Stämmen oder Varietäten derselben Art oder verschiedenen Spezies derselben Gattung angehören, oder aber von Organismen, die mehr als einer Gattung derselben oder einer anderen taxonomischen Einheit (Reich) angehören, abstammen.

Um die Expression besagter Strukturgene in der pflanzlichen Zelle zu gewährleisten, ist es vorteilhaft, wenn die kodierenden Gensequenzen zunächst in operabler Weise mit in pflanzlichen Zellen funktionsfähigen Expressions-Sequenzen verknüpft werden.

Die hybriden Genkonstruktionen im Rahmen der vorliegenden Erfindung enthalten somit neben dem (den) Strukturgen(en) Expressions-Signale, die sowohl Promotor- und Terminator-Sequenzen als auch weitere regulatorische Sequenzen der 3′ und 5′ nicht-translatierten Regionen miteinschliessen.

Jeder Promotor und jeder Terminator, der in der Lage ist, eine Induktion der Expression einer kodierenden DNA-Sequenz (Strukturgen) zu bewirken, kann als Bestandteil der hybriden Gensequenz verwendet werden. Besonders geeignet sind Expressionssignale, die aus Genen von Pflanzen oder Pflanzenviren stammen. Beispiele geeigneter Promotoren und Terminatoren sind z.B. solche der Nopalin-Synthase-Gene (nos), der Octopin-Synthase-Gene (ocs) sowie der Cauliflower Mosaik Virus Gene (CaMV) oder aber homologe DNA-Sequenzen, die noch die charakteristischen Eigenschaften der genannten Expressionssignale aufweisen.

Bevorzugt im Rahmen dieser Erfindung sind die 35S und 19S Expressions-Signale des CaMV Genoms oder deren Homologe, die mit Hilfe molekularbiologischer Methoden, wie sie z.B. bei Maniatis et al., 1982 beschrieben sind, aus besagtem Genom isoliert und mit der kodierenden DNA-Sequenz verknüpft werden können.

Unter Homologen der 35S und 19S Expressionssignale sind im Rahmen dieser Erfindung Sequenzen zu verstehen, die trotz geringer Sequenzunterschiede den Ausgangssequenzen im wesentlichen homolog sind und die nach wie vor die gleiche Funktion wie diese erfüllen.

Als Ausgangsmaterial für die 35S-Transkriptionskontroll-Sequenzen kann erfindungsgemäss z B. das Scal-Fragment des CaMV Stammes "S", das die Nukleotide 6808-7632 der Genkarte umfasst (Frank G et al., 1980), verwendet werden.

Die 19S Promotor- und 5' nicht-translatierte Region befindet sich auf einem Genomfragment zwischen der PStI Stelle (Position 5386) und der HindIII-Stelle (Position 5850) der CaMV Genkarte (Hohn et al., 1982). Die entsprechende Terminator- und 3' nichttranslatierte Region liegt auf einem EcoRV/BgIII-Fragment zwischen Position 7342 und 7643 des CaMV Genoms.

Weiterhin bevorzugt im Rahmen dieser Erfindung sind die Expressionssignale des CaMV Stammes CM 1841, dessen komplette Nukleotidsequenz bei Gardner RC et al, 1981 beschrieben ist.

Ein weiterer wirksamer Vertreter eines Pflanzenpromotors, der Verwendung finden kann, ist ein überproduzierender Pflanzenpromotor. Diese Art von Promotoren sollte, sofern sie in operabler Weise mit der Gensequenz, welche ein gewünschtes Genprodukt kodiert, verknüpft ist, in der Lage sein, die Expression besagter Gensequenz zu vermitteln.

Ueberproduzierende Pflanzenpromotoren, die im Rahmen der vorliegenden Erfindung zur Anwendung kommen können, schliessen den Promotor der kleinen Untereinheit (small subunit; ss) der Ribulose-1,5-bisphosphat-Carboxylase aus Sojabohnen [Berry-Lowe et al., J. Molecular and App. Gen., 1: 483-498 (1982)] sowie den Promotor des Chlorophyll-a/b-Bindungsproteins ein. Diese beiden Promotoren sind dafür bekannt, dass sie in eukaryontischen Pflanzenzellen durch Licht induziert werden [siehe z.B. Genetic Engineering of Plants, an Agricultural Perspective, A. Cashmore, Plenum, New York 1983, Seite 29-38; Coruzzi G. et al., The Journal of Biological Chemistry, 258: 1399 (1983) und Dunsmuir, P. et al., Journal of Molecular and Applied Genetics, 2: 285 (1983)].

Die verschiedenen DNA-Sequenzabschnitte können mit Hilfe an sich bekannter Methoden miteinander zu einer vollständigen kodierenden DNA-Sequenz verknüpft werden. Geeignete Methoden schliessen beispielsweise die in vivo Rekombination von DNA-Sequenzen, die homologe Abschnitte aufweisen sowie die in vitro Verknüpfung von Restriktionsfragmenten mit ein.

Als Klonierungsvektoren verwendet man im allgemeinen Plasmid- oder Virus-(Bakteriophage)-Vektoren mit Replikations- und Kontrollsequenzen, die von Spezies stammen, die mit der Wirtszelle kompatibel sind.

Der Klonierungsvektor trägt in der Regel einen Replikationsursprung, ausserdem spezifische Gene, die zu phaenotypischen Selektionsmerkmalen in der transformierten Wirtszelle führen, insbesondere zu Resistenzen gegenüber Antibiotika oder gegenüber bestimmten Herbiziden. Die transformierten Vektoren können anhand dieser phaenotypischen Marker nach einer Transformation in einer Wirtszelle selektiert werden.

Selektierbare phaenotypische Marker, die im Rahmen dieser Erfindung Anwendung finden können, umfassen beispielsweise, ohne das dies eine Limitierung des Erfindungsgegenstandes darstellt, Resistenzen gegen Ampicillin, Tetracyclin, Hygromycin, Kanamycin, Metotrexat, G418 und Neomycin.

Als Wirtszellen kommen im Rahmen dieser Erfindung Prokaryonten in Frage, einschliesslich bakterieller Wirte, wie z.B. A.tumefaciens, E.coli, S.typhimurium und Serratia marcescens, sowie Cyanobakterien. Ferner können auch eukaryontische Wirte wie Hefen, mycelbildende Pilze und Pflanzenzellen im Rahmen dieser Erfindung verwendet werden.

Das Einspleissen der erfindungsgemässen hybriden Genkonstruktion in einen geeigneten Klonierungsvektor erfolgt mit Hilfe von Standardmethoden wie sie z.B. bei Maniatis et al., 1982 beschrieben sind.

Dabei wird in der Regel der Vektor und die einzuspleissende DNA Sequenz zunächst mit geeigneten Restriktions-Enzymen geschnitten. Geeignete Restriktionsenzyme sind z.B. solche, die Fragmente mit glatten Enden liefern, wie z.B. Smal, Hpal und EcoRV, oder aber Enzyme, die kohäsive Enden bilden, wie z.B. EcoRI, Sacl und BamHI.

Sowohl Fragmente mit glatten Enden wie auch solche mit kohäsiven Enden, die einander komplementär sind, können mit Hilfe geeigneter DNA-Ligasen wieder zu einem einheitlichen durchgehenden DNA-Molekül verknüpft werden.

Glatte Enden können auch durch Behandlung von DNA-Fragmenten, die überhängende kohäsive Enden aufweisen, mit dem Klenow-Fragment der E.coli DNA-Polymerase durch Auffüllen der Lücken mit den entsprechenden komplementären Nukleotiden hergestellt werden.

Anderseits lassen sich auch kohäsive Enden künstlich herstellen, z.B. durch Anfügen komplementärer homopolymerer Schwänze an die Enden einer gewünschten DNA-Sequenz sowie des geschnittenen Vektormoleküls unter Verwendung einer terminalen Desoxynukleotidyl-Transferase oder aber durch Anfügen synthetischer Oligonukleotid-Sequenzen (Linker), die eine Restriktionsschnittstelle tragen und anschliessendes Schneiden mit dem entsprechenden Enzym.

Der Klonierungsvektor und die mit diesem Vektor transformierte Wirtszelle werden erfindungsgemäss dazu verwendet, die Kopienzahl des Vektors zu erhöhen. Mit einer erhöhten Kopienzahl ist es möglich, den Vektor, der die erfindungsgemässe hybride Genkonstruktion trägt, zu isolieren und z.B. für die Einschleusung der chimären Gensequenz in die pflanzliche Zelle zu verwenden.

In einem weiteren Verfahrensschritt werden diese Plasmide dazu verwendet, die ein gewünschtes Genprodukt kodierenden Strukturgene oder nichtkodierende DNA-Sequenzen mit regulatorischer Funktion in die pflanzliche Zelle einzuschleusen und in das Pflanzengenom zu integrieren.

Ein weiterer Gegenstand der vorliegenden Erfindung betrifft daher die Herstellung von pflanzlichen Rezipienten-Zellen, welche besagtes Strukturgen oder sonstige natürliche DNA-Sequenzen in ihr Genom eingebaut enthalten.

Ebenfalls umfasst vom breiten Konzept dieser Erfindung sind transgene Pflanzen, die mit Hilfe des zuvor beschriebenen erfindungsgemässen Verfahrens transformiert worden sind sowie deren asexuelle und/oder sexuelle Nachkommenschaft, welche noch die durch die Transformation der Mutterpflanze bedingte(n) neue(n) und wünschenswerte(n) Eigenschaft(en) aufweisen, sowie allgemein pflanzliches Vermehrungsgut.

Unter den Begriff der asexuellen und/oder sexuellen Nachkommenschaft transgener Pflanzen fallen definitionsgemäss im Rahmen dieser Erfindung somit auch alle Mutanten und Varianten, welche noch die charakteristischen Eigenschaften der transformierten Ausgangspflanze aufweisen, sowie sämtliche Kreuzungs- und Fusionsprodukte mit dem transformierten Pflanzenmaterial.

Unter dem Begriff des pflanzlichen Vermehrungsgutes sollen im Rahmen dieser Erfindung definitionsgemäss z.B. pflanzliche Protoplasten, Zellen, Zellklone, Zellagglomerate, Kallus- Gewebe- und/oder Organkulturen sowie Samen, Pollen, Eizellen, Zygoten, Embryonen oder anderes aus Keimbahnzellen hervorgegangenes Vermehrungsgut verstanden werden, wobei diese beispielhafte Aufzählung keine Limitierung des Erfindungsgegenstandes bedeutet.

Pflanzenteile, wie z.B. Blüten, Stengel, Früchte, Blätter, Wurzeln, die von transgenen Pflanzen oder deren Nachkommen stammen, die zuvor mit Hilfe des erfindungsgemässen Verfahrens transformiert worden sind und die somit wenigstens zum Teil aus transgenen Zellen aufgebaut sind, sind ebenfalls Gegenstand dieser Erfindung.

Das erfindungsgemässe Verfahren eignet sich für die Transformation aller Pflanzen, insbesondere solchen der systematischen Gruppen Angiospermae und Gymnospermae.

Unter den Gymnospermae sind von besonderem Interesse die Pflanzen der Klasse Coniferae.

Unter den Angiospermae sind neben den Laubbäumen und Sträuchern von besonderem Interesse Pflanzen der Familien Solanaceae, Cruciferae, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae, Malvaceae oder Gramineae und der Ordnung Leguminosae und hier vor allem der Familie Papilionaceae. Bevorzugt sind Vertreter der Solanaceae, Cruciferae, Leguminosae, Malvaceae und Gramineae.

Zu den Zielkulturen im Rahmen der vorliegenden Erfindung zählen beispielsweise auch jene, ausgewählt aus der Reihe: Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum und Sorghum, Gossypium, Ipomoea, Passiflora, Cyclamen, Malus, Prunus, Rosa, Rubus, Populus, Santalum, Allium, Lilium, Narcissus, Ananas, Arachis, Phaseolus und Pisum.

Besonders bevozugt sind dabei Vertreter aus der Familie der Gramineae, wie z.B. Pflanzen, die grossflächig angebaut werden und hohe Ernteerträge liefern. Als Beispiele seien genannt: Mais, Reis, Weizen, Gerste, Roggen, Hafer, Hirse oder Sorghum sowie Weidegräser.

Weitere Zielkulturen, die besonders bevorzugt sind für die Verwendung in dem erfindungsgemässen Verfahren, sind beispielsweise Pflanzen der Gattungen Allium, Avena, Hordeum, Oryza, Panicum, Saccharum, Secale, Setaria, Sorghum, Triticum, Zea, Musa, Cocos, Phoenix und Elaeis.

Der Nachweis einer erfolgreichen Transformation kann auf an sich bekannte Weise erfolgen, beispielsweise durch molekularbiologische Untersuchungen, zu denen besonders die "Southern blot"-Analyse gehört.

Die extrahierte DNA wird dabei zunächst mit Restriktionsenzymen behandelt, anschliessend einer Elektrophorese in einem 0,8%igen bis 1%igen Agarose-Gel unterworfen, auf eine Nitrozellulosemembran [Southern, E.M., J.Mol.Biol. 98, 503-517 (1975)] transferiert und mit der nachzuweisenden DNA, welche zuvor einer Nick Translation [Rigby, W.J., Dieckmann, M., Rhodes, C. und P. Berg, J.Mol.Biol. 113, 237-251)] unterworfen wurde (DNA-spezifische Aktivitäten von $5 \times 10^8$ bis $10 \times 10^8$ c.p.m./$\mu$g), hybridisiert. Die Filter werden dreimal je eine Stunde lang mit einer wässrigen Lösung von 0.03 M Natriumcitrat und 0.3 M Natriumchlorid bei 65° C gewaschen. Die hybridisierte DNA wird durch Schwärzung eines Röntgenfilms während 24 bis 48 Stunden sichtbar gemacht.

Zur Illustration der eher allgemeinen Beschreibung sowie zum besseren Verständnis der vorliegenden Erfindung soll nunmehr auf spezifische Ausführungsbeispiele Bezug genommen werden, die keinen limitierenden Charakter haben, es sei denn, es wird speziell darauf hingewiesen. Das gleiche gilt auch für alle beispielhaften Aufzählungen, die in der vorangegangenen Beschreibung enthalten sind.


Nichtlimitierende Ausführungsbeispiele:


Beispiel 1: Konstruktion des Plasmids pABDI

Man zerschneidet die frei zugänglichen Plasmide pkm 21 und pkm 244 [Beck, E. et al. Gene 19, 327-336 (1982)] mit der Restriktions-Endonuclease PstI. Die Fragmente der Plasmide, welche für die Rekombination verwendet werden, werden durch Elektrophorese in 0,8%igem Agarose-Gel gereinigt. Das aus der Verbindung der Bruchstücke erhaltene Plasmid pkm 21244 enthält eine Kombination der 5'- und 3'-Bal 31 - Deletionen des NPT-II-Gens, wie von Beck et al. in Gene 19, 327-336 (1982) beschrieben. Um das Promotorsignal des Cauliflower-Mosaik-Virus mit dem HindIII-Fragment des Plasmids pkm 21244 zu verbinden, wird das Kupplungsplasmid pJPAX konstruiert. Das Kupplungsplasmid pJPAX wird aus den Plasmiden pUC8 und pUC9 [Messing, J. and J. Vieira, Gene 19, 269-276 (1982)] erhalten. 10 Basenpaare von der Kupplungssequenz des Plasmids pUC9 werden durch Zerschneiden bei der HindIII- und der SalI-Position und nachfolgendem Auffüllen der haftfähigen Enden mit dem Polymerase-I-KlenowFragment [Jacobsen, H., et al., Eur. J. Biochem. 45, 623, (1974)] und Verbinden der Polynukleotidkette, wodurch die HindIII-Position wieder hergestellt wird, herausgetrennt. Ein synthetisches Kupplungsglied von 8 Basenpaaren (XhoI) wird an der SmaI-Position dieser abgetrennten Kupplungssequenz eingefügt. Die Rekombination der geeigneten XorI- und HindIII-Fragmente des Plasmids pUC8 und des modifizierten Plasmids pUC9 ergibt das Plasmid pJPAX mit einer teilweise asymmetrischen Kupplungssequenz mit den aufeinander folgenden Restriktionsstellen: EcoRI, SmaI, BamHI, SalI, PstI, HindIII, BamHI, XhoI und EcoRI. Die CaMV Gen VI Promotor-Region, die mit dem NPT II Strukturgen verknüpft wird, stammt aus dem Genom des CaMV-Stammes CM 4-184, einer Variante des CaMV-Stammes CM 1841, dessen komplette Nukleotidsequenz bei Gardner RC et al, 1981 beschrieben ist. Die CaMV Promotor-Region wird in dem 6 kBp (kBp: 1000 Basenpaare) umfassenden Cosmid pHC 79, einem Abkömmling des E. coli Plasmids pBR322 [Hohn B and Collins J, 1980], kloniert, wobei das CaMV Genom sowie das Cosmid pHC79 mit BstII geschnitten und die resultierenden Bruchstücke miteinander verknüpft werden. Die Verbindung des 5'-Expressionssignals des Cauliflower-Mosaik-Virus-Gens VI und des HindIII-Fragments des NPT-II-Gens wird beim Plasmid pJPAX durch Einfügung der Promotorregion des Cauliflower-Mosaik-Virus-Gens VI zwischen die PstI-und die Hind III-Position bewirkt. Das so erhaltene Plasmid wird an der einzigen HindIII-Position aufgeschnitten und das HindIII-Fragment des Plasmids pkm 21244 wird in diese Schnittstelle in beiden Orientierungsrichtungen eingebaut, wobei man die Plasmide pJPAX Cakm$^+$ und pJPAX Cakm$^-$ erhält. Um eine EcoRV-Sequenz in der Nähe des 3'Terminationssignals des NPT-II-Hybridgens zu schaffen, wird ein BamHI-Fragment des Plasmids pJPAX Cakm$^+$ in die BamHI-Position des Plasmids pBR327 [Soberon, X. et al., Gene 9, 287-305 (1980)] eingebaut. Man erhält das Plasmid pBR327 Cakm. Das EcoRV-Fragment des Plasmids pBR327 Cakm, das die neue DNA-Konstruktion enthält, wird verwendet, um die EcoRV-Region des Cauliflower-Mosaik-Virus-Gens VI zu ersetzen, welches an der SalI-Position im Plasmid pUC8 kloniert ist, wodurch man die Protein-kodierende DNA-Sequenz des NPT-II- Gens unter die Kontrolle der 5'- und 3'-Expressionssignale des Cauliflower-Mosaik-Virus-Gens VI stellt. Die so hergestellten Plasmide tragen die Bezeichnung pABDI und pABDII.


Beispiel 2: Konstruktion des Plasmids pDH51

Das Plasmid pDH 51 ist bei Pietrzak et al., 1986 beschrieben. Dieses Plasmid enthält die 35S Promotor-Region und Terminator-Region von CaMV, die getrennt sind durch eine eingeschobene Polylinker-Region, die zahlreiche Klonierungsstellen enthält.

Als Ausgangsmaterial für die 35S Promotor-/Terminator Region dient ein ScaI-Fragment von CaMV "S", das die Nukleotide 6808-7632 der Genkarte (Frank G et al., Cell, 21: 285-294, 1980) umfasst, und das in die SmaI-Stelle von pUC18 (Norrander J. et al., Gene, 26: 101-106, 1983) eingespleisst wird, unter Bildung von

15

Plasmid pDB21. Dies wird anschliessend mit dem Restriktionsenzym HphI verdaut. Die kohäsiven Enden werden durch Behandlung mit T4 DNA Polymerase entfernt.

Ein Fragment, welches die 35S Promotor-Region enthält [pUC18 pos. 21 (HphI)-412 (SmaI) plus CaMV pos. 6808-7437 (HphI)] wird mit KpnI Linkern verknüpft, mit NcoI (pos. 6909 CaMV) geschnitten, die kohäsiven Enden mit Hilfe des Klenow-Fragments der DNA-Polymerase aufgefüllt, mit EcoRI-Linkern verknüpft und schliesslich mit EcoRI und KpnI verdaut.

Das resultierende EcoRI/KpnI-Fragment, das die CaMV Promotor-Sequenz enthält, wird dann in das Plasmid pUC18 zwischen die KpnI und EcoRI Restriktionsschnittstellen eingespleisst. Man erhält auf diese Weise das Plasmid pDG2.

Ein zweites Fragment, das die Terminationssignale trägt [CaMV pos. 7439 (HphI)-7632 plus pUC18 pos. 413-1550 (HphI)], wird mit EcoRI geschnitten, die kohäsiven Enden werden mit Klenow aufgefüllt, mit HindIII Linkern versehen und mit HindIII und SphI verdaut.

Das resultierende SphI-HindIII Fragment, das die CaMV Terminator-Sequenz enthält, wird dann in das Plasmid pDG2 eingespleisst und zwar in die HindIII und SphI Restziktionsschnittstellen.

## Beispiel 3: Konstruktion des Plasmids pHP23

Die Konstruktion von Plasmid pHP23 erfolgt durch Insertion eines EcoRVFragments des Plasmids pABD1, welches das APH(3')II-Strukturgen sowie einen Teil der 19S RNA Promotor-Sequenz von CaMV enthält, in die SmaI-Schnittstelle des Plasmids pDH51.

## Beispiel 4: Isolierung und Injektion von zygotischen Mais-Embryonen

### a) Isolierung und Selektion der Embryonen von Mais

Junge zygotische Proembryonen und Embryonen werden aus Zea mays cv. Alpine, cv. Issar, G-4083 oder cv. Tokano, 3 bis 22 Tage nach Bestäubung isoliert (vgl. Abb. 1 Phase 17 bis 30).

Die Maiskolben werden durch eine 70%ige Ethanolbehandlung für 30 Sekunden Oberflächen-sterilisiert, und die vereinzelten jungen Maiskörner in -mit sterilem destillierten Wasser feuchtgehaltenen - Petrischalen bis zur Isolierung der zygotischen Embryonen aufbewahrt.

Die zygotischen Embryonen werden unter mikroskopischer Kontrolle (10-50x Vergrösserung) mit Hilfe feiner Skalpelle und feiner Pinzetten isoliert. Grösse und Entwicklungsstadium der isolierten Embryonen bewegt sich in einem Bereich zwischen Phase 17 und 30 der Abbildung 1.

Die Isolierung erfolgt zweckmässigerweise durch
    a) Herausschneiden eines medianen Längsschnittes im Bereich des Nucellus,
    b) anschliessende Präparation des Embryosackes und
    c) Herauspräparieren des Embryos aus dem isolierten Embryosack.

Für die Auswahl der Embryonen verwendet man in der Regel ein Stereomikroskop (50x - 200x) oder ein Inversionsmikroskop (60x-200x) sowie eine von Hand ausgezogene Mikrokapillare, die mit einem Silikonschlauch verbunden ist (für Embryonen von mehr als 20 Zellen) oder aber ein spezielles Mikroselektionssystem, das bei Koop und Schweiger, 1985; Spangenberg, 1986 und Schweiger et al., 1987 beschrieben ist.

Gleich nach der Isolierung werden die so ausgelesenen zygotischen Proembryonen und Embryonen für die nachfolgende Mikroinjektion in 50 μl bis 100 μl Tröpfchen von Kulturmedium (M1) ohne Ficoll transferiert, die zuvor auf Deckgläsern von 24 mm bis 40 mm aufgetragen wurden. Diese Mikroinjektionsplatten werden in Abschnitt [b); C)] beschrieben. Bis zur Injektion werden die Mikroinjektionsplatten in einem Zwei-Kompartimenten-System, wie in Abschnitt (c) beschrieben, eingebracht.

### b) Mikroinjektion von pHP23 Plasmid-DNA in Mais-Embryonen

### A) Apparatur

Die Mikroinjektion der Mais-Embryonen und -Proembryonen wird unter mikroskopischer Beobachtung an einem inversen Mikroskop oder einem Stereomikroskop mit einer Kombination aus Auf- und Durchlicht durchgeführt. Die Vergrösserung hängt von der Grösse der Embryonen ab und schwankt zwischen 10-800facher Vergrösserung.

Die Manipulation der zygotischen Proembryonen bzw. Embryonen wird mit Hilfe von zwei Mikromanipulatoren durchgeführt, die am Mikroskoptisch befestigt sein können (motorisch betriebene Manipulatoren) oder seitlich neben dem Mikroskop stehen (mechanische Manipulatoren). Beide Mikromanipulatoren sind mit Kapillarhalterungen ausgerüstet.

Als Mikroinjektionskapillare werden Mikroelektrodenkapillaren mit eingeschmolzenen Glasfilamenten aus Borosilikat verwendet, die einen Aussendurchmesser von 1,00 mm bis 1,50 mm aufweisen. Zur Fixierung der Embryonen beim Injektionsvorgang werden speziell für diesen Zweck hergestellte Haltekapillaren verwendet. Es handelt sich dabei um Glasrohrabschnitte aus Sodaglass mit einem Aussendurchmesser von 1,00 mm bis 1,50 mm. Beide Kapillarentypen werden auf einem handelsüblichen Kapillarenziehgerät ausgezogen, sodass geeignete Injektionskapillaren (Spitzdurchmesser ca. $\leq$ 1 $\mu$m) und Haltekapillaren entstehen.

Zur Fertigstellung der Haltekapillaren verwendet man eine Mikroschmiede, auf der die ausgezogene Haltekapillare vertikal am erforderlichen Oeffnungsdurchmesser von 0,02 mm bis 1,00 mm gebrochen und poliert wird (abgerundetes Ende mit kleiner Oeffnung).

Die Injektion der DNA-Lösung erfolgt über einen Mikroinjektor. Es handelt sich dabei um eine pneumatisch betriebene Druckvorrichtung, die eine sehr fein dosierte Injektion in die Zellen der Embryonen ermöglicht. Das Injektionsvolumen schwankt je nach Zellgrösse zwischen 1 pl und 100 pl. Geeignete Mikroinjektoren können beispielsweise bei der Firma Eppendorf, FRG oder Fa. Bachhofer, FRG bezogen werden.

## B) DNA-Lösung

Bei der mikroinjizierten DNA-Lösung (0,01 $\mu$g/$\mu$l bis 2,0 $\mu$g/$\mu$l) handelt es sich um überspiralisierte oder linearisierte pHP23 DNA oder aber um ein Gemisch aus überspiralisierter und linearisierter pHP23 DNA in einem Tris-HCl Puffer, bestehend aus 50 mM Tris-HCl, pH 7-7,8 und 50 mM NaCl.

## C) Mikroinjektion

Die Mikroinjektion der zuvor beschriebenen DNA-haltigen Lösung in die Zellen von Mais-Embryonen bzw. Proembryonen erfolgt analog dem bei Neuhaus et al., 1984; 1986 beschriebenen Verfahren. Die für die Mikroinjektion bevorzugten Bereiche sind in Abb. 1 durch Pfeile markiert.

Zunächst wird ein Deckglas (24x40 mm) halbseitig mit einem weiteren Deckglas (5 x 30 mm bis 35 mm) oder einem transparenten Tesafilmstreifen aus 1-4 übereinander liegenden Schichten der gleichen Grösse auf der Längsseite beklebt, sodass eine Kante entsteht. Auf das so präparierte Deckglas werden die Embryonen in 10 $\mu$l bis 200 $\mu$l Medientropfen Kulturmedium M1 ohne Ficoll transferiert. Mit Hilfe der Haltekapillare werden kleine Embryonen während der Injektion festgehalten, grössere Embryonen werden mit Hilfe der Haltekapillare und der Deckglaskante fixiert und anschliessend injiziert. Jeder Embryo wird je nach Zellzahl 4x bis 60x injiziert und zwischen den einzelnen Injektionsvorgängen gedreht, sodass möglichst alle Zellen getroffen werden. Anschliessend werden die injizierten zygotischen Embryonen in dem entsprechenden Medium weiterkultiviert.

## c) Mikrokultivierung von mikroinjizierten Proembryonen und Embryonen von Mais

Unmittelbar nach erfolgter Injektion werden die Embryonen mit einer von Hand ausgezogene Mikrokapillare, die mit einem Silikonschlauch verbunden ist, oder mit der gebogenen Spitze einer Nadel in die Vertiefungen einer Polycarbonat-Mikrokultivierungsplatte (Spangenberg, 1986; Spangenberg et al. 1986) transferiert, die jeweils 0,5 $\mu$l bis 2 $\mu$l Kulturmedium (M1) mit Ficoll enthalten. Alternativ hierzu können auch ca. 1 $\mu$l eines mit niedrig schmelzender Agarose (z.B. Sea Plaque®) verfestigten MI-Mediums in jede Vertiefung eingebracht und die Proembryonen bzw. Embryonen in ca. 1 $\mu$l eines flüssigen MI-Mediums, das über das verfestigte Medium geschichtet wird, kultiviert werden.

In der Regel werden die mikroinjizierten Embryonen einzeln mikrokultiviert. Es können aber auch bis zu 5 Embryonen zusammen in eine Vertiefung der Polycarbonat-Mikrokultivierungsplatte gegeben werden.

17

Die mit mikroinjizierten Embryonen beladenen Mikrokulturplatten werden in einem Zwei-Kompartimenten-System ("Falcon organ tissue culture dish"; Fa. Falcon, USA) eingebracht, das als feuchte Kammer fungiert und in der Regel im äusseren Kompartiment 2 ml bis 4 ml einer 0,2 M Mannit-Lösung (Koop und Schweiger, 1985; Spangenberg et al. 1986) Wasser oder MI-Kulturmedium enthält.

Selbstverständlich kann auch jedes andere, für die Kultivierung der Embryonen geeignete Zwei-Kompartimenten-System verwendet werden.

Alle 2 bis 3 Tage werden die injizierten Embryonen in frisches Kulturmedium (M1) mit Ficoll transferiert, wobei es von ihrem Entwicklungsstadium abhängt, ob sie in gleiches Volumen oder in grösseres Volumen von 4 $\mu$l bis 10 $\mu$l auf Terasakiplatten (Mikrowellplatte; 60 x 10 $\mu$l, Nunc, Roskilde, Dänemark) überführt werden.

Die Petrischalen (Zwei-Kompartimenten-System und Terasakiplatten) werden anschliessend mit Parafilm verschlossen und bei einer Temperatur von 25° bis 28°C in Dunkelheit oder einem Hell (Dämmerlicht, ca. 500 lux)/Dunkel-Rhythmus von 16/8 Stunden inkubiert.

d) Kultivierung der Embryonen und Regeneration ganzer Mais-Pflanzen

Nach dieser Zeit (10 bis 30 Tage nach Injektion) werden die gekeimten Pflänzchen (Länge 1 cm bis 3 cm), die aus 10 % bis 60 % der Proembryonen (vgl. Stadium 17 bis 24 der Abb. 1) und mehr als 90 % der injizierten Embryonen (vgl. Stadium 17 bis 24 der Abb. 1) erhalten werden, auf grössere Kulturgefässe (z.B. 330 ml Kulturdosen 68x110 mm, Fa. Greiner, FRG) übertragen, die 30 ml bis 50 ml festes Kulturmedium (M2) oder mit Gelrite verfestigtes MS-Medium ohne Hormonzusatz enthalten. Die Maispflanzen werden bei 25° bis 28°C und einem Hell/Dunkel Rhythmus von 16/8 Stunden (4000 Lux, kaltes Weisslicht) kultiviert, bis Wurzelbildung einsetzt und die Pflänzchen eine Grösse von 5 cm bis 15 cm erreicht haben. Dies geschieht in der Regel nach 15 bis 20 Tagen, danach werden die Pflanzen in das Gewächshaus gebracht, in Erde überführt und wie normale Maiskeimlinge bis zur Reife der Pflanzen weiterkultiviert.

Tabelle 1

| Kulturmedium (M1) für die kultivierung zygotischer Mais-Embryonen | |
|---|---|
| Kulturmedium M1 Bestandteil | Konzentration (mg/l) |
| $NH_4NO_3$ | 1650 |
| $KNO_3$ | 1900 |
| $CaCl_2 \cdot 2\,H_2O$ | 440 |
| $MgSO_4 \cdot 2\,H_2O$ | 370 |
| $NH_2PO_4$ | 170 |
| $FeNa_2$-EDTA | 40 |
| $H_3BO_3$ | 6,2 |
| $MnSO_4 \cdot 4\,H_2O$ | 22,3 |
| $ZnSO_4 \cdot 4\,H_2O$ | 8,6 |
| KI | 0,83 |
| $Na_2MoO_4 \cdot 2\,H_2O$ | 0,25 |
| $CuSO_4 \cdot 5\,H_2O$ | 0,025 |
| BAP[1] | 1 |
| Glutamin | 750 |
| Myo-inosit | 100 |
| Thiamin-HCl | 0,4 |
| Saccharose | 60000 |
| Ficoll 400®[2] | 200000 |
| Agarose | 6000 |
| pH 5,6 | |

[1] BAP Benzylaminopurin

[2] Ficoll 400® (Pharmacia:Synthetisches Polymer aus einer Co-Polymeristion von Saccharose und Epichlorhydrin mit einem Molekulargewicht von 400000.

Tabelle 2

| Kulturmedium (M2) für die kultivierung zygotischer Mais-Embryonen | |
| --- | --- |
| Kulturmedium M2 Bestandteil | Konzentration (mg/l) |
| $KH_2PO_4$ | 300 |
| $KNO_3$ | 1000 |
| $NH_4NO_3$ | 1000 |
| $Ca(NO_3)_4 \cdot 4\ H_2O$ | 500 |
| $MgSO_4 \cdot 7\ H_2O$ | 72 |
| KCl | 65 |
| $MnSO_4 \cdot H_2O$ | 5,0 |
| $Na_2MoO_4 \cdot 2\ H_2O$ | 0,25 |
| $ZnSO_4 \cdot H_2O$ | 0,17 |
| $H_3BO_3$ | 0,16 |
| KI | 0,08 |
| $Na_2$-EDTA | 37,25 |
| $FeSO_4 \cdot 7\ H_2O$ | 27,85 |
| Nikotinsäure | 5,0 |
| Pyridoxin-HCl | 5,0 |
| Thiamin-HCl | 1,0 |
| Inosit | 100 |
| Glycin | 2,0 |
| IAA[3] | 0,5 |
| Saccharose | 20000 |
| Agar | 8000 |
| Aktivkohle | 500 |
| pH 5,9 | |

[3] IAA Indolessigsäure

## Beispiel 5: Isolierung und Injektion von zygotischen Raps-Embryonen

### a) Isolierung und Selektion von Raps-Embryonen

Unreife zygotische Embryonen werden aus Brassica napus L. (Genotyp CrGC 5 "rapid cycling") und cv. Bronowski Ag903 isoliert, die im Gewächshaus bei einer Temperatur von 16° C und einem Hell/Dunkel-Rhythmus von 16/8 Stunden gezogen werden.

3 cm bis 6 cm lange Schoten werden 5 bis 20 Tage, vorzugsweise 8 bis 15 Tage nach der Bestäubung der Raps-Pflanzen gesammelt, 30 Minuten in 1%iger Calciumhypochloritlösung Oberflächen-sterilisiert und dreimal in sterilem destilliertem Wasser gewaschen. Anschliessend werden 5 bis 8 Schoten zusammen in eine Petrischale (10 cm Durchmesser) mit 4 ml destilliertem Wasser überführt. Die Petrischalen werden mit Parafilm verschlossen und bei 4° C oder bei Raumtemperatur (25° bis 28° C) bis zur Isolierung der unreifen zygotischen Embryonen aufbewahrt. Mit Hilfe feiner Pinzetten und Skalpelle werden die Schoten unter optischer Kontrolle bei einer 10- bis 20-fachen Vergrösserung unter einem Stereomikroskop geöffnet. 8 bis 15 Samenanlagen werden den Schoten entnommen, in 1 ml bis 3 ml hormonfreies Kulturmedium (R3) in Petrischalen von 6 cm Durchmesser überführt und bei 4° C oder 25° C bis zur Isolierung der zygotischen Embryonen aufbewahrt. Anschliessend werden mit Hilfe zweier Präpariernadeln die Samenanlagen geöffnet und die zygotischen Embryonen in Entwicklungsstadien und Grössen, die frühe globuläre Proembryonalstadien (vgl. Abb. 2, Phase O, P, Q) bis früh- oder spät-kotyledonäre ca. 2000-zellige Stadien (vgl. Abb. 2, Phase R, S, T) umfassen, unter mikroskopischer Kontrolle isoliert. Gleich nach der Isolierung werden die Embryonen analog zu dem unter Punkt 1a) beschriebenen Verfahren ausgelesen, wobei in diesem Falle hormonfreies Medium (R3) anstelle des unter Punkt 1a) genannten Mediums (M1) ohne Ficoll verwendet wird.

b) Mikroinjektion von pHP23 DNA in Raps-Embryonen

Die Einschleusung von pHP23 DNA in Raps-Embryonen via Mikroinjektion wird in genau analoger Weise zu dem unter Punkt 4b) beschriebenen Verfahren durchgeführt.
Die für die Mikroinjektion bevorzugten Bereiche sind in Abb. 2 mit Pfeilen markiert.

c) Mikrokultivierung mikroinjizierter Raps-Embryonen

Die Mikrokultivierung der mikroinjizierten Rapsembryonen wird analog zu dem unter Punkt 4c) für die Kultivierung der Maisembryonen bechriebenen Verfahren durchgeführt. In diesem Fall werden jedoch die Vertiefungen der Polycarbonat-Mikrokulturplatten in Abhängigkeit von Grösse und Entwicklungsstadium der mikroinjizierten Rapsembryonen mit verschiedenen Kulturmedien aufgefüllt. Junge globuläre Embryonen bis Embryonen im frühen herzförmigen Stadium werden in 0,5 $\mu$l bis 1,0 $\mu$l flüssiges Endosperm transferiert, das durch Absaugen des Inhaltes einer unreifen Raps-Samenanlage, die sich in einem ähnlichen Entwicklungsstadium befindet, isoliert wird.

Raps-Embryonen im späten herzförmigen bis frühen Torpedo-Stadium werden in 0,5 $\mu$l bis 1,0 $\mu$l Gemisch von flüssigem Rapsendosperm (wie oben gewonnen) und synthetischem Kulturmedium (R3) (normalerweise ein 1:1 Gemisch) mikrokultiviert.

Mikroinjizierte Raps-Embryonen, die sich im späten Torpedo-Stadium bis frühen Kotyledonar-Stadium befinden, werden direkt auf 0,5 $\mu$l bis 1,0 $\mu$l synthetischem Kulturmedium (R3) mikrokultiviert.

Die mikroinjizierten Embryonen werden in einer Dichte von 1 bis 5 Embryonen/Vertiefung auf den Mikrokulturplatten kultiviert, und in dem als Feuchtekammer dienenden Zwei-Kompartimenten-System (wie unter Punkt 4c) beschrieben) bei 25 °C im Dunkeln oder im Hell (Dämmerlicht, ca. 500 lux)/Dunkel Rhythmus von 16/8 Stunden inkubiert.

d) Kultivierung der mikroinjizierten Raps-Embryonen und Regeneration ganzer Pflanzen

Jeden 2. und 4. Tag, beginnend 1 bis 4 Tage nach der Mikroinjektion und in Abhängigkeit von der Grösse und dem Entwicklungsstadium der mikroinjizierten Raps-Embryonen, werden diese auf Vertiefungen von Poly carbonat-Mikrokulturplatten mit 0,5 $\mu$l bis 2,0 $\mu$l flüssigem Kulturmedium (R3) oder in 4 $\mu$l bis 10 $\mu$l des Kulturmediums (R4) in Vertiefungen von Terasaki-Platten überführt.

Der kleine Volumenbereich (0,5 $\mu$l bis 2,0 $\mu$l) wird für junge Raps-Embryonen, die Entwicklungsstadien zwischen dem frühen herzförmigen- und dem frühen Torpedo-Stadium erreicht haben, benutzt. Der grosse Volumenbereich wird (4 $\mu$l bis 10 $\mu$l) dagegen für mikroinjizierte Embryonen im Entwicklungsstadium zwischen dem späten Torpedo-Stadium und dem späten Kotyledonar-Stadium angewandt.

Diese Kulturphase erfolgt bei einer Temperatur von 25° bis 28° C und in einem Hell/Dunkel Rhythmus von 16/8 Stunden (4000 Lux, kaltes Weisslicht).

Die Regeneration ganzer Pflanzen aus den mikroinjizierten Raps-Embryonen, sowie die Induktion von sekundären Embryonen aus dem Stengel der Primärregenerate geschieht durch Transfer der mikroinjizierten Raps-Embryonen auf festes Kulturmedium (R5). Normalerweise werden immer 5 Embryonen zusammen auf 7 ml Kulturmedium (R5) in Petrischalen von 6 cm Durchmesser transferiert. Die Petrischalen werden anschliessend mit Parafilm versiegelt und bei 25° bis 28° C in einem Hell/Dunkel Rhythmus von 16/8 Stunden (4000 Lux, kaltes Weisslicht) für 1 bis 3 Wochen bis zur Pflanzenregeneration oder zur Induktion einer Sekundärembryogenese inkubiert.

Nach der Regeneration grüner Pflänzchen - durch direkte Keimung der mikroinjizierten Primär-Embryonen oder durch Differenzierung der induzierten Sekundär-Embryoide - werden diese für die weitere Entwicklung in 50 ml Plastik-Container mit 25 ml Kulturmedium (R6) überführt und bei einer Temperatur von 23° C bis 28° C und einer Beleuchtungsdauer von 10 bis 16 Stunden (4000 Lux, kaltes Weisslicht) kultiviert, bis sie eine Grösse von 1 cm bis 3 cm erreicht haben.

Nach der Ausbildung von Spross und Wurzeln werden die Raps-Pflänzchen in 330 ml Kulturdosen mit 30 ml bis 50 ml festes Kulturmedium (R6) überführt, bis sie eine Grösse von 5-10 cm erreicht haben. Dies geschieht in der Regel nach weiteren 1 bis 3 Wochen, danach werden die Pflanzen in das Gewächshaus gebracht und weiterhin wie normale Raps-Pflänzchen bis zur Reife der Pflanzen gezogen.

Tabelle 3

| Kulturmedien (R₃; R₄) für die kultivierung zygotischer Raps-Embryonen | | |
|---|---|---|
| Bestandteil | Konzentration (mg/l) | |
| | R₃ | R₄ |
| $Ca(NO_3)_2 \cdot 4 H_2O$ | 500 | 500 |
| $KNO_3$ | 125 | 125 |
| $MgSO_4 \cdot 7 H_2O$ | 125 | 125 |
| $KH_2PO_4$ | 125 | 125 |
| $MnSO_4 \cdot 4 H_2O$ | 25 | 25 |
| $H_3BO_3$ | 10 | 10 |
| $ZnSO_4 \cdot 4 H_2O$ | 10 | 10 |
| $Na_2MoO_4 \cdot 2 H_2O$ | 0,25 | 0,25 |
| $CuSO_4 \cdot 5 H_2O$ | 0,025 | 0,025 |
| $CoCl_2 \cdot 6 H_2O$ | 0,025 | 0,025 |
| $Na_2$-EDTA | 37,3 | 37,3 |
| $FeSO_4 \cdot 7 H_2O$ | 27,8 | 27,8 |
| Glycin | 2 | 2 |
| Nikotinsäure | 5 | 5 |
| Pyridoxin-HCl | 0,5 | 0,5 |
| Thiamin-HCl | 0,5 | 0,5 |
| Folsäure | 0,5 | 0,5 |
| Biotin | 0,05 | 0,05 |
| Inosit | 100 | 100 |
| Glutamin | 1000 | 1000 |
| Asparagin | 1000 | - |
| Serin | - | 100 |
| Saccharose | 20000 | 120000 |
| BAP[4] | 0,1 | 0,2 |
| IAA[5] | 0,5 | 0,3 |
| NAA[6] | - | 0,5 |
| | pH 5,8 | pH 6,0 |

[4] BAP: Benzylaminopurin
[5] IAA: Indolessigsäure
[6] NAA: Naphthyl-1-essigsäure

Tabelle 4

| Kulturmedien ($R_3$; $R_4$) für die kultivierung zygotischer Raps-Embryonen | | |
|---|---|---|
| Bestandteil | Konzentration (mg/l) | |
| | $R_5$ | $R_6$ |
| $KNO_3$ | 2500 | 2500 |
| $CaCl_2 \cdot 2 H_2O$ | 150 | 150 |
| $MgSO_4 \cdot 7 H_2O$ | 250 | 250 |
| $(NH_4)SO_4$ | 134 | 134 |
| $NaH_2PO_4 \cdot H_2O$ | 150 | 150 |
| KI | 0,75 | 0,75 |
| $H_3BO_3$ | 3,0 | 3,0 |
| $MnSO_4 \cdot H_2O$ | 10,0 | 25 |
| $ZnSO_4 \cdot 4 H_2O$ | 2,0 | 2,0 |
| $Na_2MoO_4 \cdot 2 H_2O$ | 0,25 | 0,25 |
| $CuSO_4 \cdot 5 H_2O$ | 0,025 | 0,025 |
| $CoCl_2 \cdot 6 H_2O$ | 0,025 | 0,025 |
| $Na_2$-EDTA | 37,3 | 37,3 |
| $FeSO_4 \cdot 7 H_2O$ | 27,8 | 27,8 |
| Inosit | 100 | 100 |
| Nikotinsäure | 1,0 | - |
| Pyridoxin-HCl | 1,0 | - |
| Thiamin-HCl | 10,0 | - |
| Saccharose | 30000 | 100 |
| BAP[7] | 0,05 | - |
| Difco-Bacto® Agar | 8000 | 7000 |
| Aktivkohle | - | 1000 |
| | pH 5,8 | pH 5,8 |

[7] BAP: Benzylaminopurin

**Beispiel 6a: Isolierung und Injektion von zygotischen Weizen-Proembryonen und -Embryonen mit anschliessender direkter Keimung**

a) Isolierung und Selektion zygotischer Weizen-Proembryonen und -Embryonen

Die Isolierung unreifer zygotischer Embryonen erfolgt 4 bis 12 Tage nach der Bestäubung aus den unreifen Samenanlagen von Weizen.

Die Präparation und Selektion der zygotischen Embryonen wird in genau analoger Weise zu dem unter Punkt 4a) für Mais bzw. dem unter Punkte 5a) für Raps beschriebenen Verfahren durchgeführt.

Nach der Oberflächen-Sterilisation der Samenanlagen und dem Einbringen in destilliertes Wasser werden die zygotischen Embryonen mit Hilfe eines Stereomikroskops, feiner Skalpelle sowie feiner Pinzetten isoliert und anschliessend für die nachfolgende Mikroinjektion selektiert. Am besten geeignet für die Mikroinjektion sind Embryonen, die sich zwischen dem 16 und 500 Zellenstadium befinden.

Die isolierten und selektionierten zygotischen Embryonen werden zunächst auf quadratische Plastikschälchen, die jeweils 4 Vertiefungen aufweisen (Multidish 4, Nuncton Delta SI, Fa. Nunc, Dänemark), in 0,5 ml Kulturmedium W1 (Tabelle 5) transferiert. Dort werden sie in der Regel für einen Tag bei einer Temperatur von 4°C im Dunkeln belassen. Die bevorzugte Kultivierungsdichte liegt bei 8 bis 10 Embryonen pro Vertiefung.

b) Mikroinjektion von pHP23 DNA in Weizen-Proembryonen und -Embryonen

Die Einschleusung von pHP23 DNA in Weizen Embryonen via Mikroinjektion wird in genau analoger Weise zu dem unter Punkt 4b) beschriebenen Verfahren durchgeführt. Die Injektion der DNA haltigen Lösung erfolgt bevorzugt im Bereich der embryonalen Achse, einer Region meristemoider Zellen, aus denen später die Plumula und die Radicula hervorgehen.

### c) Mikrokultivierung mikroinjizierter Weizen-Proembryonen und -Embryonen

Nach der Mikroinjektion werden die injizierten Embryonen für einen weiteren Tag unter den gleichen Bedingungen und auf den gleichen Kulturplatten weiterkultiviert.

1 Tag nach Injektion werden die Embryonen bei 25°C unter diffusem Licht für weitere 3 bis 5 Tage kultiviert. Nach einer Gesamtkultivierungsdauer von 5 bis 7 Tagen in flüssigem Kulturmedium werden die Embryonen auf Terasaki-Platten transferiert, die 2 Lagen unterschiedlicher Medien enthalten.

Die erste Lage besteht aus 10 μl Medium W2, das mit Sea Plaque® Agarose verfestigt wird, die zweite Lage bilden 10 μl flüssiges Medium W1, das dem festen Medium überschichtet wird. Selbstverständlich können für die Verfestigung von Medium W2 auch andere, für die Kultivierung pflanzlicher Embryonen geeignete, Verfestigungsmedien verwendet werden, vorzugsweise Agarose vom 'Low melting type' (LMP-Agarose). Die Kultivierung erfolgt auch in diesem Fall unter den gleichen Bedingungen, d.h. bei einer Temperatur von 25°C und unter diffusem Licht.

Nach einer Kultivierungsdauer von 7 bis 15 Tagen, abhängig von der Grösse der Embryonen, beginnen diese auszukeimen. Nach 10 bis 14 Tagen werden die mikroinjizierten Embryonen daher in Multiwell-Kulturschalen auf 1 ml bis 5 ml Kulturmedium W2 überführt, welches durch Sea Plaque® Agarose verfestigt wird, und bei einer Temperatur von 25°C in einem Hell/Dunkel Rhythmus von 16 h/8 h zur Differenzierung weiterkultiviert.

### d) Kultivierung der Embryonen und Regeneration ganzer Weizenpflanzen

Ca. 4 Wochen nach der erfolgten Mikroinjektion werden die gekeimten Pflänzchen in grössere Kulturgefässe überführt. Es handelt sich dabei um Kulturdosen mit einem Volumen von 60 ml, die 15 ml des Kulturmediums W3 enthalten, diese werden später gegen grössere Kulturdosen mit einem Volumen von 330 ml ausgetauscht, die 30 ml bis 50 ml W4 Medium enthalten.

Wenn die Pflänzchen eine bestimmte Grösse erreicht haben, werden sie in Erde überführt und ins Gewächshaus verbracht, wo sie wie normale Weizenkeimlinge weiterbehandelt werden.

24

Tabelle 5a

| Kulturmedien (W1; W2; W3; W4) für die Kultivierung zygotischer Weizen-Embryonen | | | | |
|---|---|---|---|---|
| | W1 [mg/l] | W2 [mg/l] | W3 [mg/l] | W4 [mg/l] |
| $KNO_3$ | 1900 | 1900 | 1900 | 1900 |
| $NH_4NO_3$ | 165 | 165 | 165 | 165 |
| $CaCl_2 \cdot 2\,H_2O$ | 440 | 440 | 440 | 440 |
| $MgSO_4 \cdot 7\,H_2O$ | 370 | 370 | 370 | 370 |
| $KH_2PO_4$ | 170 | 170 | 170 | 170 |
| $FeNa_2$-EDTA | 40 | 40 | 40 | 40 |
| $MnSO_4 \cdot 4\,H_2O$ | 22,3 | 22,3 | 22,3 | 22,3 |
| $H_3BO_3$ | 6,2 | 6,2 | 6,2 | 6,2 |
| $ZnSO_4 \cdot 4\,H_2O$ | 8,6 | 8,6 | 8,6 | 8,6 |
| KI | 0,83 | 0,83 | 0,83 | 0,83 |
| $Na_2MoO_4 \cdot 2\,H_2O$ | 0,25 | 0,25 | 0,25 | 0,25 |
| $CuSO_4 \cdot 5\,H_2O$ | 0,025 | 0,025 | 0,025 | 0,025 |
| Kinetin | - | - | 1,0 | 2,0 |
| BAP[8] | 0,5 | 0,5 | - | - |
| NAA[9] | - | - | 0,5 | 1,0 |
| IAA[10] | 0,1 | 0,2 | - | - |
| Thiamin, HCl | 1,0 | 1,0 | 0,4 | 0,4 |
| Saccharose | 20000 | 20000 | 30000 | 20000 |
| Glucose | 5000 | 5000 | - | - |
| Myo-inosit | 100 | 100 | 100 | 100 |
| Glutamin | 500 | 500 | 750 | 750 |
| Sea Plaque® Agarose[11] | - | 6000 | 7000 | 7000 |

[8] BAP: Benzylaminopurin

[9] NAA: Naphthyl-1-essigsäure

[10] IAA: Indolessigsäure

[11] Sea Plaque® Agarose: FMC Corporation, Marine Colloids Div. Rockland, USA

Beispiel 6b: Isolierung und Injektion von Weizen Pro-/Embryonen mit anschliessender Induktion adventiver Embryoide

a) Isolierung und Selektion zygotischer Pro-/Embryonen von Weizen

Die Isolierung unreifer zygotischer Embryonen erfolgt 4 bis 12 Tage nach der Bestäubung aus den unreifen Samenanlagen von Weizen.

Die Präparation und Selektion der zygotischen Embryonen wird in genau analoger Weise zu dem unter Punkt 4a) für Mais bzw. dem unter Punkt 5a) für Raps beschriebenen Verfahren durchgeführt.

Nach der Oberflächen-Sterilisation der Samenanlagen (15 Minuten lang in 1,8 %ige (V/V) Natriumhypochloritlösung mit anschliessender dreifacher Waschung mit sterilem destilliertem Wasser) und dem Einbringen in destilliertes Wasser, werden die zygotischen Pro-/Embryonen unter mikroskopischer Kontrolle (10 x - 50 x Vergrösserung) mit Hilfe eines Skalpells und Präpariernadeln isoliert. Grösse und Entwicklungsstadium der isolierten Pro-/Embryonen bewegt sich in einem Bereich zwischen ca. 20-zelligen globulären Proembryonen mit Suspensor und Embryonen mit Skutellum und Koleoptyl.

Gleich nach der Isolierung werden die ausgelesenen zygotischen Pro-/Embryonen anschliessend für die nachfolgende Mikroinjektion in 50 µl bis 100 µl Tröpfchen von Kulturmedium WZ 1 transferiert, die zuvor auf Deckgläsern von 24x40 mm aufgetragen wurden. Diese Mikroinjektionsplatten werden in Abschnitt [4b) C)] beschrieben. Bis zur Mikroinjektion werden die Mikroinjektionsplatten in einem Zwei-Kompartimenten-System, wie in Abschnitt 4c) beschrieben, eingebracht.

25

b) Mikroinjektion von pHP23 DNA in zygotischen Pro-/Embryonen von Weizen

Die Einschleusung von pHP23 DNA in Pro-/Embryonen von Weizen via Mikroinjektion wird in genau analoger Weise zu dem unter Punkt 4b) beschriebenen Verfahren durchgeführt.

c) Mikrokultivierung von mikroinjizierten Pro-/Embryonen von Weizen

Unmittelbar nach erfolgter Injektion werden die Embryonen mit einer von Hand ausgezogene Mikrokapillare, die mit einem Silikonschlauch verbunden ist, oder mit der gebogenen Spitze einer Nadel in die Vertiefungen einer Polycarbonat-Mikrokultivierungsplatte (Spangenberg, 1986, et al. 1986) transferiert, die jeweils 0,5 µl bis 1 µl Kulturmedium WZ 2 enthalten. Zusätzlich wird dieses feste Kulturmedium WZ 2 mit 0,5 µl bis 1 µl flüssigem Endosperm - durch Absaugen des Inhaltes einer unreifen Weizen-Samenanlage ähnliches Entwicklungsstadiums - überschichtet.

In der Regel werden die mikroinjizierten Pro-/Embryonen einzeln mikrokultiviert. Es können aber auch bis zu 10 Pro-/Embryonen zusammen in eine Vertiefung der Polycarbonat-Mikrokultivierungsplatte transferiert werden. Die mit mikroinjizierten Embryonen beladenen Mikrokulturplatten werden in einem Zwei-Kompartimenten-System ("Falcon organ tissue culture dish" No. 3037, Fa. Falcon, USA) eingebracht, das als Feuchtekammer fungiert und in der Regel im äusseren Kompartiment 2 ml bis 3 ml einer 0,2 M Mannit-Lösung enthält (Koop und Schweiger, 1985, Spangenberg, 1986, et al. 1986), dieses mit Parafilm verschlossen und bei einer Temperatur von 25-28° C im Dunkeln inkubiert.

d) Kultivierung der mikroinjizierten Weizen-Embryonen, Induktion sekundärer Embryoide und Regeneration ganzer Pflanzen

Alle 2 bis 3 Tage - beginnend 2 Tage nach der Injektion der Weizen Pro-/Embryonen - wird 0,5 µl bis 2 µl Kulturmedium WZ 3 zu den einzelnen - mit injizierten Pro-/Embryonen beladenen - Vertiefungen der Mikrokulturplatten gegeben bis die Pro-/Embryonen eine Grösse von ca. 2-5 mm erreicht haben.

Anschliessend und entsprechend ihrem Entwicklungsstadium werden die injizierten Embryonen einzeln oder bis zu 5 Embryonen zusammen auf 1 ml Kulturmedium WZ 4 in Vertiefungen quadratischer Plastikschälchen (4 well multidish, Fa. Nunc, Dänemark) transferiert.

Nach einer Kulturphase von 4 bis 6 Wochen auf Kulturmedium WZ 4 erfolgt die Induktion von Kallus mit Potential zu sekundärer Embryogenese. Diese embryogenen Kalli werden aufgeteilt und auf 10 ml Kulturmedium WZ 5 in Plastikdosen Sterilin 125 AP (Fa. Sterilin Ltd., Feltham, England) zur Differenzierung von Sprossen übertragen.

Die weitere Entwicklung dieser Sprosse sowie ihre Bewurzelung erfolgt nach wiederholtem Transfer auf 30 ml bis 80 ml Kulturmedium WZ 5 in grösseren Kulturgefässen (330 ml Kulturdosen, 68x110 mm, Fa. Greiner, FRG). Wenn die differenzierten Pflänzchen eine bestimmte Grösse (5 cm bis 10 cm) erreicht und ein ausreichendes Wurzelsystem entwickelt haben, werden sie in Erde überführt und ins Gewächshaus verbracht, wo sie wie normale Weizenkeimlinge weiterbehandelt werden.

Tabelle 5b

| Kulturmedien (W1; W2; W3; W4) für die Kultivierung zygotischer Weizen-Embryonen | | | |
|---|---|---|---|
| | W1 [mg/l] | W2 [mg/l] | W3 [mg/l] | W4 [mg/l] |
| $NH_4NO_3$ | 1650 | 1650 | 1650 | 3300 |
| $KNO_3$ | 1900 | 1900 | 1900 | 3800 |
| $CaCl_2 \cdot 2\,H_2O$ | 440 | 440 | 440 | 880 |
| $MgSO_4 \cdot 7\,H_2O$ | 370 | 370 | 370 | 740 |
| $KH_2PO_4$ | 170 | 170 | 170 | 340 |
| KI | 0,83 | 0,83 | 0,83 | 1,66 |
| $H_3BO_3$ | 6,2 | 6,2 | 6,2 | 12,40 |
| $MnSO_4 \cdot 4\,H_2O$ | 22,3 | 22,3 | 22,3 | 44,60 |
| $ZnSO_4 \cdot 4\,H_2O$ | 8,6 | 8,6 | 8,6 | 17,20 |
| $Na_2MoO_4 \cdot 2\,H_2O$ | 0,25 | 0,25 | 0,25 | 0,5 |
| $CuSO_4 \cdot 5\,H_2O$ | 0,025 | 0,025 | 0,025 | 0,5 |
| $CoCl_2 \cdot 6\,H_2O$ | 0,025 | 0,025 | 0,025 | 0,5 |
| $Na_2$-EDTA | 37,3 | 37,3 | 37,3 | 74,6 |
| $FeSO_4 \cdot 7\,H_2O$ | 27,8 | 27,8 | 27,8 | 55,6 |
| Nicotinsäure | 0,5 | 0,5 | 0,5 | 0,5 |
| Pyridoxin-HCl | 0,5 | 0,5 | 0,5 | 0,5 |
| Thiamin-HCl | 0,1 | 0,1 | 0,1 | 0,1 |
| Glycin | 2,0 | 2,0 | 2,0 | 2,0 |
| Cefotaxime | - | - | - | 0,1 |
| LMP-Agarose | - | 6000 | - | 6000 |
| Saccharose | 30000 | 30000 | 30000 | 20000 |
| Inositol | 200 | 200 | 200 | 200 |
| Caseinhydrolysat | 200 | 200 | 200 | 200 |
| Glutamin | - | - | - | 500 |
| 2,4-D[12] | 1,0 | 1,0 | - | 2,0 |
| Kinetin | 1,0 | 1,0 | - | - |
| ABA[13] | 0,5 | 0,5 | - | - |
| $GA_3$[14] | 0,1 | 0,1 | - | - |

[12] 2,4-D: 2,4-Dichlorphenoxyessigsäure
[13] ABA Abszisinsäure
[14] $GA_3$ Giberellinsäure

Tabelle 5c

| Kulturmedium WZ 5 | |
|---|---|
| | WZ 5 [mg/l] |
| $KH_2PO_4$ | 300 |
| $KNO_3$ | 1000 |
| $NH_4NO_3$ | 1000 |
| $Ca(NO_3)_4 \cdot 4\ H_2O$ | 500 |
| $MgSO_4 \cdot 7\ H_2O$ | 72 |
| KCl | 65 |
| $MnSO_4 \cdot H_2O$ | 5,0 |
| $ZnSO_4 \cdot H_2O$ | 0,17 |
| $H_3BO_3$ | 0,16 |
| KI | 0,08 |
| $Na_2$-EDTA | 37,25 |
| $FeSO_4 \cdot 7\ H_2O$ | 27,85 |
| Nicotinsäure | 5,0 |
| Pyridoxin-HCl | 5,0 |
| Thiamin-HCl | 1,0 |
| Inosit | 100 |
| Glycin | 2,0 |
| IAA[15] | 0,5 |
| Saccharose | 20000 |
| Agar | 8000 |
| Aktivkohle | 500 |
| pH 5,9 | - |

[15] IAA Indolessigsäure

Beispiel 7: Isolierung und Injektion von zygotischen Reis-Embryonen

a) Isolierung und Selektion zygotischer Reis-Proembryonen und -Embryonen

Die Isolierung unreifer zygotischer Proembryonen bzw. Embryonen erfolgt 6 bis 12 Tage nach der Bestäubung aus den unreifen Samenanlagen von Reis.

Die Präparation und Selektion der zygotischen Proembryonen bzw. Embryonen wird in genau analoger Weise zu dem unter Beispiel 4a) für Mais bzw. dem unter Beispiel 5a) für Raps beschriebenen Verfahren durchgeführt.

Nach der Oberflächensterilisation der Samenanlagen und dem Einbringen in destilliertes Wasser werden die zygotischen Proembryonen bzw. Embryonen mit Hilfe eines Stereomikroskopes (Zeiss), feiner Skalpelle sowie feiner Pinzetten isoliert und anschliessend für die nachfolgende Mikroinjektion selektioniert. Am besten geeignet für die Mikroinjektion sind Proembryonen bzw. Embryonen, die sich zwischen Phase 20 und Phase 25 (siehe Abb. 1) befinden.

Die isolierten und selektionierten zygotischen Proembryonen bzw. Embryonen werden zunächst in quadratische Plastikschälchen, die jeweils 4 Vertiefungen aufweisen (Multidish 4, Nuncton Delta SI, Fa. Nunc, Dänemark), in 0,5 ml Kulturmedium RS1 (Tabelle 6) transferiert. Die bevorzugte Kultivierungsdichte liegt bei 4 bis 6 Proembryonen bzw. Embryonen pro Vertiefung.

b) Mikroinjektion von pHP 23 DNA in Reis-Proembryonen bzw. -Embryonen

Die Einschleusung von pHP 23 DNA in Reis-Proembryonen bzw. -Embryonen via Mikroinjektion wird in genau analoger Weise zu dem unter Beispiel 4b) beschriebenen Verfahren durchgeführt. Die Injektion der DNA haltigen Lösung erfolgt bevorzugt in die mit einem Pfeil bezeichneten embryonalen Zellen (vgl. Abb

28

1).

## c) Mikrokultivierung mikroinjizierter Reis-Embryonen

Nach der Mikroinjektion werden die injizierten Proembryonen bzw. Embryonen für einen weiteren Tag unter den gleichen Bedingungen und auf den gleichen Kulturplatten weiterkultiviert.

2 Tage nach der Injektion werden die Pro-/Embryonen bei 25°C und unter diffusem Licht für weitere 5 bis 7 Tage kultiviert. Nach einer Gesamtkultivierungsdauer von insgesamt 7 bis 10 Tagen in flüssigem Kulturmedium werden die Embryonen auf Terasaki-Platten transferiert, die 2 Lagen unterschiedlicher Medien enthalten. Die erste Lage besteht aus 5 µl Medium RS2 das mit einer niedrig schmelzenden Agarose, wie z.B. mit Sea Plaqueo® Agarose (0,6 % v/w) verfestigt wird, die zweite Lage bilden 5 µl flüssiges Medium RS1, das dem festen Medium überschichtet wird. Selbstverständlich können für die Verfestigung von Medium RS2 auch andere, für die Kultivierung pflanzlicher Embryonen geeignete, Verfestigungsmedien verwendet werden, vorzugsweise Agarose vom 'Low Melting Type' (LMP-Agarose). Die Kultivierung erfolgt auch in diesem Fall unter den gleichen Bedingungen, d.h. bei einer Temperatur von 25°C und unter diffusem Licht.

Nach einer Kultivierungsdauer von 7 bis 15 Tagen, abhängig von der Grösse der verwendeten Pro-/Embryonen, beginnen diese auszukeimen. Nach 15 Tagen werden die mikroinjizierten Pro-/Embryonen daher in Multiwell-Kulturschalen auf 1 bis 2 ml Kulturmedium RS2 überführt, welches z.B. durch Sea Plaque® Agarose verfestigt wird, und bei einer Temperatur von 25°C in einem Hell/Dunkel Rhythmus von 16/8 Stunden zur Differenzierung weiterkultiviert.

## d) Kultivierung der Embryonen und Regeneration ganzer Reispflanzen

Ca. 4-5 Wochen nach der erfolgten Mikroinjektion werden die gekeimten Pflänzchen in grössere Kulturgefässe überführt. Es handelt sich dabei um handelsübliche Kulturdosen mit einem Volumen von 60 ml, die 15 ml des Kulturmediums RS3 enthalten. Diese werden später gegen grössere Kulturdosen mit einem Volumen von 330 ml ausgetauscht, die 30 bis 40 ml RS4 Medium enthalten.

Wenn die Pflänzchen eine bestimmte Grösse erreicht haben, werden sie in Erde überführt und ins Gewächshaus verbracht, wo sie wie normale Reis-Keimlinge weiterbehandelt werden.

Tabelle 6

| Medien für die Kultivierung zygotischer Reis-Pro-Embryonen | | | |
|---|---|---|---|
| | RS1 [mg/l] | RS2 [mg/l] | RS3 [mg/l] | RS4 [mg/l] |
| $KNO_3$ | 1900 | 1900 | 1900 | 1900 |
| $NH_4NO_3$ | 165 | 1650 | 1650 | 1650 |
| $CaCl_2 \cdot 2\,H_2O$ | 440 | 440 | 440 | 440 |
| $MgSO_4 \cdot 7\,H_2O$ | 370 | 370 | 370 | 370 |
| $KH_2PO_4$ | 170 | 170 | 170 | 170 |
| $FeNa_2$-EDTA | 40 | 40 | 40 | 40 |
| $MnSO_4 \cdot 4\,H_2O$ | 22,3 | 22,3 | 22,3 | 22,3 |
| $H_3BO_3$ | 6,2 | 6,2 | 6,2 | 6,2 |
| $ZnSO_4 \cdot 4\,H_2O$ | 8,6 | 8,6 | 8,6 | 8,6 |
| KI | 0,83 | 0,83 | 0,83 | 0,83 |
| $Na_2MoO_4 \cdot 2\,H_2O$ | 0,25 | 0,25 | 0,25 | 0,25 |
| $CuSO_4 \cdot 5\,H_2O$ | 0,025 | 0,025 | 0,025 | 0,025 |
| Kinetin | - | - | 1,0 | 1,0 |
| BAP[16] | 0,5 | 0,5 | - | - |
| IAA[17] | 0,2 | 0,2 | 0,5 | 0,5 |
| NAA[18] | - | - | 0,5 | 0,5 |
| Thiamin, HCl | 1,0 | 0,4 | 0,4 | 0,4 |
| Saccharose | 20000 | 20000 | 25000 | 20000 |
| Glucose | 5000 | 5000 | - | - |
| Myo-inosit | 100 | 100 | 100 | 100 |
| Casein Hydrolysat | 300 | - | - | - |
| Glutamin | 500 | 160 | 160 | 160 |
| Agarose/Agar | - | Sea plaque® 0,6 % | Agar 0,75 % | Agar 0,75 % |

[16] BAP: Benzylaminopurin

[17] IAA: Indolessigsäure

[18] NAA: Naphthyl-1-essigsäure

## Beispiel 8: Isolierung und Injektion von zygotischen Gerste-Proembryonen und Embryonen

### a) Isolierung und Selektion zygotischer Gerste-Proembryonen und -Embryonen

Unreife zygotische Proembryonen und Embryonen werden aus Hordeum vulgare cv. Igri, 6 bis 14 Tage nach der Bestäubung isoliert.

Die aus den Gerste-Aehren gewonnenen Samenanlagen werden 15 Minuten lang in einer 1,8 %igen (v/v) Natriumhypochlorit-Lösung Oberflächensterilisiert und anschliessend dreimal in sterilem destilliertem Wasser gewaschen.

Die Präparation und Selektion der zygotischen Pro-/Embryonen wird in genau analoger Weise zu dem unter Punkt 4a) für Mais bzw. unter Punkt 5a) für Raps beschriebenen Verfahren durchgeführt.

Nach der Oberflächensterilisation der Samenanlagen und dem Einbringen in destilliertes Wasser werden die zygotischen Pro-/Embryonen unter mikroskopischer Kontrolle (10 x - 50 x Vergrösserung) mit Hilfe eines Skalpells und feiner Pinzetten isoliert.

Grösse und Entwicklungsstadium der isolierten Pro-/Embryonen bewegt sich in einem Bereich, der frühe globuläre Proembryonen mit Suspensor und unreife Embryonen mit Skutellum und Koleoptyl umfasst.

Die isolierten und selektionierten zygotischen Embryonen werden zunächst auf quadratische Plastikschälchen, die jeweils 4 Vertiefungen aufweisen (Multidish 4, Nunclon Delta SI, Fa. Nunc, Dänemark) in 0,5 ml Kulturmedium G1 transferiert, bei einer bevorzugten Dichte von 8 bis 10 Pro-/Embryonen pro Vertiefung und dort bis zur Mikroinjektion bei 25° C aufbewahrt.

b) Mikroinjektion von pHP23 DNA in zygotischen Pro-/Embryonen von Gerste

Die Einschleusung von pHP23 DNA in Gerste-Pro-/Embryonen via Mikroinjektion wird in genau analoger Weise zu dem unter Punkt 4b) beschriebenen Verfahren durchgeführt. Die Injektion der DNA haltigen Lösung erfolgt bevorzugt im Bereich der embryonalen Achse, einer Region meristemoider Zellen, aus denen später die Plumula und die Radicula hervorgehen. Der bevorzugte Injektionsort ist in Abb. 1 durch einen Pfeil näher bezeichnet.

c) Mikrokultivierung mikroinjizierter Gerste-Pro-/Embryonen

Die Mikrokultivierung der mikroinjizierten Pro-/Embryonen von Gerste wird analog zu dem unter Punkt 4c) für die Kultivierung von Mais-Embryonen beschriebenen Verfahren durchgeführt.
In diesem Fall werden jedoch die Vertiefungen der Polycarbonat-Mikrokulturplatten mit 0,5 μl bis 1,0 μl flüssigem Kulturmedium G1 angefüllt. Anschliessend werden die mikroinjizierten Pro-/Embryonen in einer Dichte von 1 bis 20 Pro-/Embryonen pro Vertiefung auf die Mikrokulturplatten transferiert und die Vertiefung mit 0,5 μl bis 1 μl flüssigem Kulturmedium G1 aufgefüllt.

d) Kultivierung der Gerste-Pro-/Embryonen und Regenerierung ganzer Pflanzen

Die mikroinjizierten Pro-/Embryonen werden regelmässig alle 3 bis 12 Tage auf frisches Kulturmedium G1 übertragen und in neue Mikrokulturkammern überführt, wobei die Vertiefung 1 μl bis 10 μl Kulturmedium G1 enthalten, abhängig von der Grösse und Zahl der mikrokultivierten Pro-/Embryonen. Wenn die Pro-/Embryonen eine Grösse von 2 mm bis 5 mm erreicht haben, werden sie in Medium G2 überführt und zwar in einer Dichte von 16 Embryonen pro 10 ml Kulturmedium und Petrischale. Diese werden mit Parafilm versiegelt und anschliessend im Dunkeln für einen Zeitraum von 5 Tagen bei einer Temperatur von 25° C inkubiert. Anschliessend werden die Kulturen bei einer Temperatur von 23° C und einer Beleuchtungsdauer von 10 Stunden/Tag (4000 Lux, kaltes Weisslicht) gehalten, bis zum Ergrünen.
Nach der Regeneration grüner Pflänzchen werden diese für die weitere Entwicklung in handelsübliche 60 ml Plastik Container mit 25 ml Medium G3 überführt und bei einer Temperatur von 23° C bis 26° C und einer Beleuchtungsdauer von 10 bis 16 Stunden (4000 Lux, kaltes Weisslicht) kultiviert, bis sie eine Grösse von 1 cm bis 3 cm erreicht haben. Die Wurzelbildung wird durch erneutes Ueberführen der regenerierten Pflänzchen in Kulturmedium G3 erreicht. Nach der Ausbildung mehrerer Sprosse und Wurzeln kann die Wintergerste vernalisiert werden, indem man die Pflanzentöpfe in 240 ml Container überführt und bei 4° C und einer Beleuchtungsdauer von 10 bis 16 Stunden bei 2000 Lux (Phillips, warmes Weisslicht) für einen Zeitraum von 6 bis 8 Wochen kultiviert.

Tabelle 7

| Medien für die Kultivierung zygotischer Gerste-Pro-/Embryonen | | | |
|---|---|---|---|
| Bestandteil | G1 [mg/l] | G2 [mg/l] | G3 [mg/l] |
| $NH_4NO_3$ | 165 | 165 | 165 |
| $KNO_3$ | 1900 | 1900 | 1900 |
| $CaCl_2 \cdot 2\,H_2O$ | 440 | 440 | 440 |
| $MgSO_4 \cdot 7\,H_2O$ | 370 | 370 | 370 |
| $NH_2PO_4$ | 170 | 170 | 170 |
| $FeNa_2$-EDTA | 40 | 40 | 40 |
| $H_3BO_3$ | 6,2 | 6,2 | 6,2 |
| $MnSO_4 \cdot 4\,H_2O$ | 22,3 | 22,3 | 22,3 |
| $ZnSO_4 \cdot 4\,H_2O$ | 8,6 | 8,6 | 8,6 |
| KI | 0,83 | 0,83 | 0,83 |
| $Na_2MoO_4 \cdot 2\,H_2O$ | 0,25 | 0,25 | 0,25 |
| $CuSO_4 \cdot 5\,H_2O$ | 0,025 | 0,025 | 0,025 |
| BAP[19] | - | 0,4 | - |
| Glutamin | 750 | 750 | 750 |
| Myo-inosit | 100 | 100 | 100 |
| Thiamin, HCl | 0,4 | 0,4 | 0,4 |
| Saccharose | 20000 | 20000 | 20000 |
| "Sea Plaque"® Agarose | - | 6000 | 6000 |
| pH | 5,6 | 5,6 | 5,6 |

[19] BAP Benzylaminopurin

## Beispiel 9: Isolierung und Injektion von zygotischen Baumwoll Proembryonen und Embryonen

### a) Isolierung und Selektion der Pro-/Embryonen aus Baumwolle

Junge zygotische Proembryonen und Embryonen werden aus unreifen 1 cm bis 2 cm grossen Früchten von Gossypium hirsutum ca. 4 bis 20 Tage nach der Bestäubung isoliert.

Die Fruchtstände werden zuerst unter fliessendem Leitungswasser vorgereinigt, eventuell unter Zusatz eines kommerziell erhältlichen Küchenreinigers (Lux, Pril, usw.). Nach gründlicher Reinigung wird der Fruchtstand mit 70 % Ethanol abgespritzt und anschliessend mit destilliertem Wasser gewaschen. Die unreifen Früchte werden nun mit einer 3,6 % Natriumhypochloridlösung, die als Zusatz 0,1 % Tween 20 enthält, für 45 min sterilisiert. Anschliessend werden die Früchte mit sterilem Wasser ausreichend gewaschen und in einer feucht gehaltenen Petrischale aufbewahrt (Raumtemperatur) Mit Hilfe feiner Pinzetten und Skalpelle werden die Früchte unter dem Stereomikroskop mit einer 10- bis 20-fachen Vergrösserung geöffnet. Aus den Früchten werden die Embryosäcke entnommen und auf quadratische Plastikschälchen, die jeweils 4 Vertiefungen aufweisen (Multi dish 4, Nunclon Delta SI, Fa. Nunc, Dänemark) in ca. 0,5 ml hormonfreies Medium (C1) überführt. Anschliessend werden mit Hilfe zweier Präpariernadeln aus den Embryosäcken die Proembryonen und Embryonen in verschiedenen Grössen, die von globulären 20-zelligen Stadien (vgl. Abb. 2, Phase O, P, Q) bis früh- bis spätkotyledonären ca. 2000-zelligen Stadien (vgl. Abb. 2, Phase R, S, T) reichen, unter mikroskopischer Beobachtung isoliert. Nach der Isolation werden die Embryonen analog zu dem unter Beispiel 4a) beschriebenen Verfahren ausgelesen, wobei in diesem Fall hormonfreies Medium (C1) verwendet wird.

### b) Mikroinjektion von pHP23 DNA in Baumwoll-Pro-/Embryonen

Die Einschleusung von pHP23 in diese Pro-/Embryonen via Mikroinjektion wird in genau analoger Weise zu dem unter Beispiel 4b) beschriebenen Verfahren durchgeführt. Der bevorzugte Injektionsort ist in Abb. 2 durch einen Pfeil markiert.

c) Mikrokultivierung mikroinjizierter Baumwoll-Pro-/Embryonen

Die Kultivierung der mikroinjizierten Baumwoll-Pro-/Embryonen wird analog zu dem unter Beispiel 4c) für die Kultivierung der Maisembryonen beschriebenen Verfahren durchgeführt. Die erste Mikrokulturphase in C1 Medium dauert etwa 7 Tage. Anschliessend werden die Pro-/Embryonen für 4 bis 6 Wochen in C2 Medium in geeigneten Kulturgefässen (siehe Beispiel 4c und 4d) kultiviert. Die anschliessende Regeneration zu kleinen Pflänzchen erfolgt in C3 Medium. Wenn diese Pflänzchen eine Grösse von ca. 6 bis 10 cm erreicht haben, können sie ins Gewächshaus überführt werden, wo sie wie normale Baumwollpflanzen bis zur Reife weiterkultiviert werden können.

Tabelle 7

| Medien für die Kultivierung zygotischer Baumwoll-Embryonen | | | |
|---|---|---|---|
| Bestandteil | C1 [mg/l] | C2 [mg/l] | C3 [mg/l] |
| $NH_4NO_3$ | 240.12 | 1650 | 1650 |
| $KNO_3$ | 505.56 | 1900 | 1900 |
| $CaCl_2 \cdot 2\,H_2O$ | 176.42 | 440 | 440 |
| $MgSO_4 \cdot 7\,H_2O$ | 492.96 | 370 | 370 |
| $KH_2PO_4$ | 27.20 | 170 | 170 |
| $H_3BO_3$ | 1,85 | 6,3 | 6,3 |
| $MnSO_4 \cdot 4\,H_2O$ | 5,07 | 22,3 | 22,3 |
| $ZnSO_4 \cdot 7\,H_2O$ | 2,59 | 8,6 | 8,6 |
| KI | 0,25 | 0,83 | 0,83 |
| $Na_2MoO_4 \cdot 2\,H_2O$ | 0,22 | 0,25 | 0,25 |
| $CuSO_4 \cdot 5\,H_2O$ | 0,0075 | 0,025 | 0,025 |
| $CoCl_2 \cdot 6\,H_2O$ | 0,0071 | 0,025 | 0,025 |
| $Na_2FeEDTA$ | 20 | 40 | 40 |
| Inosit | 100 | 100 | 100 |
| Nicotinsäure | 0,5 | 1,0 | 1,0 |
| Pyridoxin-HCl | 0,5 | 1,0 | 1,0 |
| Thiamin-HCl | 10 | 10 | 10 |
| Glycin | 2,0 | - | - |
| $MgCl_2$ | 750 | - | - |
| ABA[20] | - | 0,1 | - |
| GA[21] | - | 0,1 | 0,1 |
| Glucose | 30000 | 30000 | - |
| Saccharose | - | - | 20000 |
| Gelrite® | - | - | 1600 |

[20] ABA Abszisinsäure
[21] GA Gibberellinsäure

Beispiel 10: Isolierung und Injektion von zygotischen Soyabohnen-Proembryonen und Embryonen

a) Isolierung und Selektion der Pro-/Embryonen aus Soyabohnen

Junge zygotische Proembryonen und Embryonen werden aus 10 mm bis 50 mm langen Schoten (ca. 3 bis 20 Tage nach der Bestäubung) von Glycine max cv Marple Arrow isoliert. Die Pflanzen werden im Gewächshaus bei einer Temperatur von 19°C bis 21°C und einem Hell/Dunkelrhythmus von 16/8 Stunden gezogen.

Vor der Isolierung werden die Schoten durch eine 10 minütige Behandlung mit einer 1,5 % in Calziumhypochloridlösung, die als Zusatz 0,1 % Tween 20 enthält, sterilisiert und anschliessend ausreichend mit sterilem destilliertem Wasser gespült. Die so behandelten Schoten werden in befeuchteten Petrischalen bei

33

ca. 4° C im Dunkel bis zur weiteren Behandlung aufbewahrt. Mit Hilfe feiner Pinzetten und Skalpelle werden die Schoten unter dem Stereomikrosop bei einer 10- bis 20-fachen Vergrösserung geöffnet. Die Samenanlagen werden den Schoten entnommen und in ca. 1 ml hormonfreies Medium (S3) in Petrischalen von 3 cm Durchmesser überführt. Aus den Samenanlagen werden die Embryosäcke entnommen und in ca. 1 ml hormonfreies Medium (S3) in Petrischalen von 3 cm Durchmesser überführt. Anschliessend werden mit Hilfe zweier Präpariernadeln aus den Embryosäcken die Proembryonen und Embryonen in verschiedenen Grössen, die von frühen globulären Proembryonalstadien (vgl. Abb. 2, Phasen O, P, Q) bis früh- bis spätkotyledonären ca. 2000-zelligen Stadien (vgl. Abb. 2, Phase R, S, T) reichen, unter mikroskopischer Beobachtung isoliert. Nach der Isolation werden die Embryonen analog zu dem unter Beispiel 4a) beschriebenen Verfahren ausgelesen, wobei in diesem Fall hormonfreies Medium (S3) ohne Ficoll verwendet wird.

b) Mikroinjektion von pHP23 DNA in Soyabohnen-Pro-/Embryonen

Die Einschleusung von pHP23 in diese Pro-/Embryonen via Mikroinjektion wird in genau analoger Weise zu dem unter Beispiel 4b) beschriebenen Verfahren durchgeführt.

c) Mikrokultivierung mikroinjizierter Sojabohnen-Pro-/Embryonen

Die Kultivierung der mikroinjizierten Soyabohnen-Pro-/Embryonen wird analog zu dem unter Beispiel 4c) für die Kultivierung von Maisembryonen beschriebenen Verfahren durchgeführt. In diesem Fall werden jedoch die Vertiefungen der Polycarbonat-Mikrokulturplatten in Abhängigkeit der Grösse und der Entwicklungsstadien der mikroinjizierten Pro-/Embryonen mit unterschiedlichen Mengen an Kulturmedium gefüllt. Junge globuläre Proembryonen werden auf eine 0,1 μl bis 0,5 μl Schicht Medium S1 gelegt und mit 0,1 μl bis 0,5 μl S3 Medium überschichtet. Auf diese Weise werden 1-10 Proembryonen in einer Vertiefung der Mikrokulturplatte kultiviert. Herzförmige und spätere Kotyledonarstadien werden auf einer 0,2 μl bis 0,5 μl Schicht von Medium S1 gelegt und mit 0,2 μl bis 0,5 μl S3 Medium überschichtet [vgl. Beispiel 7c)]. Diese grösseren Entwicklungsstadien werden immer vorzugsweise einzeln kultiviert. Die Mikrokulturplatten werden in dem als Feuchtkammer dienenden Zwei-Kompartimenten-System [wie unter Beispiel 4c) beschrieben] bei 21° C im Dunkeln oder im Hell (Dämmerlicht ca. 500 Lux)/Dunkel Rhythmus von 16/8 Stunden inkubiert.

d) Kultivierung der mikroinjizierten Pro-/Embryonen und Regeneration ganzer Pflanzen

Jeden 2. und 4. Tag beginnend 1 bis 4 Tage nach Mikroinjektion und in Abhängigkeit von der Grösse und dem Entwicklungsstadium der mikroinjizierten Embryonen werden diese durch Zugabe von 0,2 μl bis 0,5 μl S3 Medium mit neuem Kulturmedium versehen. Nach ca. 6 bis 10 Tagen, jeweils abhängig von der Grösse der kultivierten Embryonen, werden diese auf 4 μl bis 10 μl des Kulturmediums S2 in Vertiefungen von Terrasaki Platten überführt. Es werden auch hier die kultivierten Pro-/Embryonen nach dem Transfer in die Vertiefungen mit 0,2 μl bis 1 μl S3 Kulturmedium überschichtet [vgl. Beispiel 7c)]. Um eine ausreichende Feuchtigkeit zu gewährleisten werden ca. 1 ml bis 2 ml steriles Wasser am Rand der Vertiefungen der Terrasaki Platten abgesetzt. Die Embryonen werden wiederum bei 25° C und einem Hell/Dukelrhythmus von 16/8 Stunden (4000 Lux, kaltes Weisslicht) inkubiert.

Alle 3 bis 4 Tage werden die sich entwickelnden Embryonen durch Zugabe von 0,2 μl bis 1,0 μl S3 Medium befeuchtet. Nach der Regeneration von kleinen Keimlingen werden diese in Costar-Platten ("Costar wells"; 24 wells; 16 mm Durchmesser; # 3524; Fa. Tissue Culture Cluster, Cambridge, Mass.) auf S3 Medium (mit 0,8 % Agarose) überführt und unter den oben beschriebenen Kulturbedingungen weiter kultiviert. Eventuell kann es erforderlich sein erneut eine Wurzelinduktion durch Zugabe von Hormonen in das Medium auszulösen. Dies kann beispielsweise dadurch erreicht werden, dass die Pflänzchen für 3 bis 6 Tage auf wurzelinduzierendem Medium (S4) gehalten werden. Nach Ausbildung mehrerer Blättchen und Wurzeln werden diese Pflänzchen in 300 ml Kulturdosen mit 20 ml bis 50 ml S3 Medium, das 0,8 % Agarose enthält, überführt. Wenn diese Pflänzchen eine Grösse von ca. 6 cm bis 10 cm erreicht haben, können sie ins Gewächshaus gebracht werden, wo sie wie normale Soyabohnenpflanzen bis zur Reife weiterkultiviert werden können.

## Tabelle 9

| Medien für die Kultivierung zygotischer Soyabohnen-Embryonen | | | | |
|---|---|---|---|---|
| Bestandteil | S1 [mg/l] | S2 [mg/l] | S3 [mg/l] | S4 [mg/l] |
| $NH_4NO_3$ | 1650 | 1650 | 1650 | - |
| $KNO_3$ | 1900 | 1900 | 1900 | 1900 |
| $CaCl_2 \cdot 2\,H_2O$ | 440 | 440 | 440 | 440 |
| $MgSO_4 \cdot 2\,H_2O$ | 370 | 370 | 370 | 370 |
| $NH_2PO_4$ | 170 | 170 | 170 | 170 |
| $FeNa_2$-EDTA | 40 | 40 | 40 | 40 |
| $H_3BO_3$ | 6,2 | 6,2 | 6,2 | 6,2 |
| $MnSO_4 \cdot 4\,H_2O$ | 22,3 | 22,3 | 22,3 | 22,3 |
| $ZnSO_4 \cdot 4\,H_2O$ | 8,6 | 8,6 | 8,6 | 8,6 |
| KI | 0,83 | 0,83 | 0,83 | 0,83 |
| $Na_2MoO_4 \cdot 2\,H_2O$ | 0,25 | 0,25 | 0,25 | 0,25 |
| $CuSO_4 \cdot 5\,H_2O$ | 0,025 | 0,025 | 0,025 | 0,025 |
| Inosit | 10000 | 10000 | 10000 | 10000 |
| Thiamin-HCl | 40 | 40 | 40 | 40 |
| Saccharose | 30000 | 20000 | 10000 | 10000 |
| BAP[22] | 0,6 | 0,4 | - | - |
| Zeatin | 0,1 | 0,05 | - | - |
| IBA[23] | - | - | - | 1 |
| Sea Plaque® Agarose | 6000 | 6000 | - | - |
| Difco-Bacto®-Agar | - | - | - | 7000 |
| pH | 5,8 | 5,8 | 5,8 | 5,8 |

[22] BAP: Benzylaminopurin
[23] IBA: Indolbuttersäure ("Indolebutyric acid")

Beispiel 11: Isolierung und Injektion von zygotischen Sonnenblumen Proembryonen und Embryonen

a) Isolierung und Selektion der Pro-/Embryonen aus Sonnenblumen

Junge zygotische Proembryonen und Embryonen werden aus unreifen 6 mm bis 8 mm langen Früchten (hellgrau bis weisse Fruchtwand) von Helianthus annum ca. 4-20 Tage nach Bestäubung isoliert. Die Fruchtstände werden zuerst unter fliessendem Leitungswasser vorgereinigt, eventuell unter Zusatz eines kommerziell erhältlichen Küchenreinigers (Lux, Pril, usw.). Nach gründlicher Reinigung wird der Fruchtstand mit 70 % Ethanol abgespritzt und anschliessend mit destilliertem Wasser gewaschen. Die unreifen Früchte mit grauer bis weisser Fruchtwand werden anschliessend von dem Fruchtstand mit Hilfe von Pinzetten entfernt und in 330 ml Plastikdosen gesammelt. Jede dieser Dosen sollte etwa 1/4 gefüllt sein. Die unreifen Früchte werden nun mit einer 1,5 %igen Calziumhypochloridlösung, die als Zusatz 0,1 % Tween 20 enthält, für 15 min sterilisiert. Anschliessend werden die Früchte mit sterilem Wasser ausreichend gewaschen und in einer feucht gehaltenen Petrischale aufbewahrt (4˚C). Mit Hilfe feiner Pinzetten und Skalpelle werden die Früchte unter dem Stereomikrosop bei einer 10-20 fachen Vergrösserung geöffnet. Aus den Früchten werden die Embryosäcke entnommen und in ca. 1 ml hormonfreies Medium (H3) in Petrischalen von 3 cm Durchmesser überführt. Anschliessend werden mit Hilfe zweier Präpariernadeln aus den Embryosäcken die Proembryonen und Embryonen in verschiedenen Grössen, die von frühen globulären Proembryonalstadien (vgl. Abb. 2, Phasen O, P, Q) bis frühbis spätkotyledonären ca. 2000-zelligen Stadien (vgl. Abb. 2, Phase R, S, T) reichen, unter mikroskopischer Beobachtung isoliert. Nach der Isolation werden die Embryonen analog zu dem unter Beispiel 4a) beschriebenen Verfahren ausgelesen, wobei in diesem Fall hormonfreies Medium (H3) ohne Ficoll verwendet wird.

b) Mikroinjektion von pHP23 DNA in Sonnenblumen Pro-/Embryonen

Die Einschleusung von pHP23 in diese Pro-/Embryonen via Mikroinjektion wird in genau analoger Weise zu dem unter Beispiel 4b) beschriebenen Verfahren durchgeführt.

#### c) Mikrokultivierung mikroinjizierter Sonnenblumen Embryonen

Die Kultivierung der mikroinjizierten Sonnenblumen Embryonen wird analog zu dem unter Beispiel 4c) für die Kultivierung von Maisembryonen beschriebenen Verfahren durchgeführt. In diesem Fall werden jedoch die Vertiefungen der Polycarbonat-Mikrokulturplatten in Abhängigkeit der Grösse und der Entwicklungsstadien der mikroinjizierten Embryonen mit unterschiedlichen Mengen an Kulturmedium gefüllt. Junge globuläre Proembryonen werden auf eine 0,1 $\mu$l bis 0,5 $\mu$l Schicht Medium H1 gelegt und mit 0,1 $\mu$l bis 0,5 $\mu$l H3 überschichtet [vgl. Beispiel 7c)]. Auf diese Weise können 1-10 Proembryonen in einer Vertiefung der Mikrokulturplatte kultiviert werden. Herzförmige und spätere Kotyledonarstadien werden auf eine 0,2 $\mu$l bis 0,5 $\mu$l Schicht von H1 gelegt und mit 0,2 $\mu$l bis 0,5 $\mu$l H3 Medium überschichtet. Diese grösseren Entwicklungsstadien werden vorzugsweise einzeln kultiviert. Die Mikrokulturplatten werden in dem als Feuchtekammer dienenden Zwei-Kompartimenten-System [wie unter Beispiel 4c) beschrieben] bei 25° C im Dunkeln oder im Hellen (Dämmerlicht; ca. 500 lux)/Dunkel Rhythmus von 16/8 Stunden inkubiert.

#### d) Kultivierung der mikroinjizierten Pro-/Embryonen und Regeneration ganzer Pflanzen

Jeden 2. und 4. Tag beginnend 1 bis 4 Tage nach der Mikroinjektion und in Abhängigkeit von der Grösse und dem Entwicklungsstadium der mikroinjizierten Embryonen, werden diese durch Zugabe von 0,2 $\mu$l bis 0,5 $\mu$l H3 Medium mit neuem Kulturmedium versehen. Nach ca 6-10 Tagen, jeweils abhängig von der Grösse der kultivierten Embryonen, werden diese auf 4 $\mu$l bis 10 $\mu$l des Kulturmediums H2 in Vertiefungen von Terrasaki Platten überführt. Es werden auch hier die kultivierten Embryonen nach dem Transfer in die Vertiefungen mit 0,2 $\mu$l bis 1 $\mu$l H3 Kulturmedium überschichtet. Um eine ausreichende Feuchtigkeit zu gewährleisten, werden ca. 1 ml bis 2 ml steriles Wasser am Rand der Vertiefungen der Terrasaki Platten abgesetzt. Die Embryonen werden wiederum bei 25° C und einem Hell/Dukelrhythmus von 16/8 Stunden (4000 lux, kaltes Weisslicht) inkubiert.
Alle 3-4 Tage werden die sich entwickelnden Embryonen durch Zugabe von 0,2 $\mu$l bis 1,0 $\mu$l H3 Medium befeuchtet. Nach der Regeneration von kleinen Keimlingen werden diese in Costar-Platten auf H3 Medium (mit 0,8 % Agarose) überführt und unter den oben beschriebenen Kulturbedingungen weiter kultiviert. Eventuell kann es erforderlich sein erneut eine Wurzelinduktion durch Zugabe von Hormonen in das Medium auszulösen. Dies kann beispielsweise dadurch erreicht werden, dass die Pflänzchen für 3-6 Tage auf einem wurzelinduzierendem Medium (H4) gehalten werden. Nach Ausbildung mehrerer Blättchen und Wurzeln werden diese Pflänzchen in 330 ml Kulturdosen mit 30 ml bis 50 ml H3 Medium, das 0,8 % Agarose enthält, überführt. Wenn diese Pflänzchen eine Grösse von ca. 6 bis 10 cm erreicht haben, können sie ins Gewächshaus gebracht werden, wo sie wie normale Sonnenblumenpflanzen bis zur Reife weiterkultiviert werden können.

Tabelle 10

| Medien für die Kultivierung zygotischer Sonnenblumen-Embryonen | | | | |
|---|---|---|---|---|
| Bestandteil | H1 [mg/l] | H2 [mg/l] | H3 [mg/l] | H4 [mg/l] |
| $NH_4NO_3$ | 1650 | 1650 | 1650 | - |
| $KNO_3$ | 1900 | 1900 | 1900 | 1900 |
| $CaCl_2 \cdot 2\,H_2O$ | 440 | 440 | 440 | 440 |
| $MgSO_4 \cdot 2\,H_2O$ | 370 | 370 | 370 | 370 |
| $NH_2PO_4$ | 170 | 170 | 170 | 170 |
| $FeNa_2$-EDTA | 40 | 40 | 40 | 40 |
| $H_3BO_3$ | 6,2 | 6,2 | 6,2 | 6,2 |
| $MnSO_4 \cdot 4\,H_2O$ | 22,3 | 22,3 | 22,3 | 22,3 |
| $ZnSO_4 \cdot 4\,H_2O$ | 8,6 | 8,6 | 8,6 | 8,6 |
| KI | 0,83 | 0,83 | 0,83 | 0,83 |
| $Na_2MoO_4 \cdot 2\,H_2O$ | 0,25 | 0,25 | 0,25 | 0,25 |
| $CuSO_4 \cdot 5\,H_2O$ | 0,025 | 0,025 | 0,025 | 0,025 |
| Inosit | 100 | 100 | 100 | 100 |
| Nikotinsäure | 0,5 | 0,5 | 0,5 | 0,5 |
| Pyridoxin-HCl | 0,5 | 0,5 | 0,5 | 0,5 |
| Thiamin-HCl | 0,1 | 0,1 | 0,1 | 0,1 |
| Caseinhydrolysat | 500 | 500 | 500 | 500 |
| Saccharose | 30000 | 20000 | 10000 | 10000 |
| BAP[24] | 0,6 | 0,4 | - | - |
| NAA[25] | - | - | - | 0,1 |
| Sea Plaque® Agarose | 6000 | 6000 | - | - |
| Difco-Bacto®-Agar | - | - | - | 7000 |

[24] BAP: Benzylaminopurin
[25] NAA: Naphthyl-1-essigsäure

**Beispiel 12: Isolierung und Injektion von zygotischen Arabidopsis Proembryonen und Embryonen**

**a) Isolierung und Selektion der Pro-/Embryonen aus Arabidopsis**

Junge zygotische Proembryonen und Embryonen werden aus 5-15 mm langen Schoten (ca. 3-20 Tage nach der Bestäubung) von Arabidopsis thaliana cv. Columbia isoliert. Die Pflanzen werden im Gewächshaus bei einer Temperatur von 16 °C bis 18 °C und einem Hell/Dunkelrhythmus von 16/8 Stunden gezogen. Vor der Isolierung werden die Schoten durch eine 10 minütige Behandlung mit 1,5 % Calciumhypochlorid sterilisiert und anschliessend ausreichend mit sterilem destilliertem Wasser gespült. Die so behandelten Schoten werden in befeuchteten Petrischalen bei ca. 4 °C im Dunkel bis zur weiteren Behandlung aufbewahrt. Mit Hilfe feiner Pinzetten und Skalpelle werden die Schoten unter dem Stereomikrosop bei einer 10-20 fachen Vergrösserung geöffnet. Die Samenanlagen werden den Schoten entnommen und in ca. 1 ml hormonfreies Medium (A3) in Petrischalen von 3 cm Durchmesser überführt. Anschliessend werden mit Hilfe zweier Präpariernadeln aus den Samenanlagen die Proembryonen und Embryonen in verschiedenen Grössen, die von frühen globulären Proembryonalstadien (vgl. Abb. 2, Phasen O, P, Q) bis früh- bis spätkotyledonären ca. 2000-zelligen Stadien (vgl. Abb. 2, Phase R, S, T) reichen, unter mikroskopischer Beobachtung isoliert. Nach der Isolation werden die Embryonen analog zu dem unter Beispiel 4a) beschriebenen Verfahren ausgelesen, wobei in diesem Fall hormonfreies Medium (A3) ohne Ficoll verwendet wird.

**b) Mikroinjektion von pHP23 DNA in Arabidopsis-Pro-/Embryonen**

Die Einschleusung von pHP23 in diese Pro-/Embryonen via Mikroinjektion wird in genau analoger Weise zu dem unter Beispiel 4b) beschriebenen Verfahren durchgeführt.

c) Mikrokultivierung mikroinjizierter Arabidopsis-Pro-/Embryonen

Die Kultivierung der mikroinjizierten Arabidopsis-Pro-/Embryonen wird analog zu dem unter Beispiel 4c) für die Kultivierung von Maisembryonen beschriebenen Verfahren durchgeführt. In diesem Fall werden jedoch die Vertiefungen der Polycarbonat-Mikrokulturplatten in Abhängigkeit von der Grösse und den Entwicklungsstadien der mikroinjizierten Embryonen mit unterschiedlichen Mengen an Kulturmedium gefüllt. Junge globuläre Proembryonen werden auf eine 0,1 μl bis 0,5 μl Schicht von A1 gelegt und mit 0,1 μl bis 0,5 μl A3 Medium überschichtet [vgl. Beispiel 7c)]. Auf diese Weise können 1-10 Proembryonen in einer Vertiefung der Mikrokulturplatte kultiviert werden. Herzförmige und spätere Kotyledonarstadien werden auf eine 0,2 μl bis 0,5 μl Schicht von Medium A1 gelegt und mit 0,2 μl bis 0,5 μl A3 Medium überschichtet. Diese grösseren Entwicklungsstadien werden vorzugsweise einzeln kultiviert. Die Mikrokulturplatten werden in dem als Feuchtekammer dienenden Zwei-Kompartimenten-System [wie unter Beispiel 4c) beschrieben] bei 21°C im Dunkeln oder im Dauerlicht (ca. 500 lux) inkubiert.

d) Kultivierung der mikroinjizierten Pro-/Embryonen und Regeneration ganzer Pflanzen

Jeden 2. und 4. Tag beginnend 1 bis 4 Tage nach der Mikroinjektion und in Abhängigkeit von der Grösse und dem Entwicklungsstadium der mikroinjizierten Embryonen werden diese durch Zugabe von 0,2 μl bis 0,5 μl A3 Medium mit neuem Kulturmedium versehen. Nach ca. 6-10 Tagen, jeweils abhängig von der Grösse der kultivierten Embryonen, werden diese auf 4 μl bis 10 μl des Kulturmediums A2 in Vertiefungen von Terrasaki Platten überführt. Es werden auch in diesem Fall die kultivierten Embryonen nach dem Transfer in die Vertiefungen mit 0,2 μl bis 1 μl A3 Kulturmedium überschichtet. Um eine ausreichende Feuchtigkeit zu gewährleisten werden ca. 1 ml bis 2 ml steriles Wasser am Rand der Vertiefungen der Terrasaki Platten abgesetzt. Die Embryonen werden wiederum bei 21°C und Dauerlicht (ca 500 lux) inkubiert.

Alle 3-4 Tage werden die sich entwickelnden Embryonen durch Zugabe von 0,2 μl bis 1,0 μl A3 Medium befeuchtet. In dieser Kulturphase entwickeln sich bereits kleine Pflänzchen (10-15 Tage), die anschliessend auf Costar-Kulturplatten mit 1 ml bis 1,3 ml A3 Medium, das 0,8 % Agarose enthält, überführt werden. Nach Ausbildung mehrerer Blättchen und Wurzeln werden diese Pflänzchen in handelsübliche 330 ml Kulturdosen mit 30 ml bis 50 ml A3 Medium mit 0,8 % Agarose überführt. Wenn diese Pflänzchen eine Grösse von ca. 5 cm bis 6 cm erreicht haben, können sie ins Gewächshaus gebracht werden, wo sie wie normale Arabidopsispflanzen bis zur Reife weiterkultiviert werden können.

Tabelle 11

| Medien für die Kultivierung von Arabidopis-Embryonen | | | |
|---|---|---|---|
| Bestandteil | A1 [mg/l] | A2 [mg/l] | A3 [mg/l] |
| $KNO_3$ | 2500 | 2500 | 2500 |
| $CaCl_2 \cdot 2\ H_2O$ | 150 | 150 | 150 |
| $MgSO_4 \cdot 7\ H_2O$ | 250 | 250 | 250 |
| $(NH_4)SO_4$ | 134 | 134 | 134 |
| $NaH_2PO_4 \cdot H_2O$ | 150 | 150 | 150 |
| KI | 0,75 | 0,75 | 0,75 |
| $H_3BO_3$ | 3,0 | 3,0 | 3,0 |
| $MnSO_4 \cdot 4\ H_2O$ | 10,0 | 10,0 | 10,0 |
| $ZnSO_4 \cdot 4\ H_2O$ | 2,0 | 2,0 | 2,0 |
| $Na_2MoO_4 \cdot 2\ H_2O$ | 0,25 | 0,25 | 0,25 |
| $CuSO_4 \cdot 5\ H_2O$ | 0,025 | 0,025 | 0,025 |
| $CoCl_2 \cdot 6\ H_2O$ | 0,025 | 0,025 | 0,025 |
| $Na_2$-EDTA | 37,3 | 37,3 | 37,3 |
| $FeSO_4 \cdot 7\ H_2O$ | 27,8 | 27,8 | 27,8 |
| Inosit | 100 | 100 | 100 |
| Nikotinsäure | 1,0 | 1,0 | 1,0 |
| Pyridoxin-HCl | 1,0 | 1,0 | 1,0 |
| Thiamin-HCl | 10,0 | 10,0 | 10,0 |
| Saccharose | 30000 | 20000 | 4000 |
| BAP[26] | 0,6 | 0,1 | - |
| Sea Plaque® Agarose | 6000 | 6000 | - |
| Kinetin | 0,05 | 0,05 | - |
| | ph 5,8 | ph 5,8 | ph 5,8 |

[26] BAP: Benzylaminopurin

## Beispiel 13: Nachweis des NPT-II-Gens im Pflanzenerbgut

Blattgewebe der regenerierten und transformierten Pflanzen werden bei 0°C in 15 %-iger Saccharoselösung, enthaltend 50 mMol/l Ethylendiamin-N,N,N',N'-tetraessigsäure, EDTA), 0,25 Mol/l Natriumchlorid und 50 mMol/l $\alpha,\alpha,\alpha$-Tris-(hydroxymethyl)-methylamin-Hydrochlorid (TRIS-HCl) bei pH 8,0 homogenisiert. Durch Zentrifugieren des Homogenisats für 5 Minuten bei 1000 g trennt man grob die Zellkerne ab. Die Zellkerne werden in 15 %-iger Saccharoselösung, enthaltend 50 mMol/l EDTA und 50 mMol/l TRIS-HCl, bei pH 8,0 resuspendiert, mit Natrium-dodecylsulfat (SDS) bis zu einer Endkonzentration von 0,2 % versetzt und 10 Minuten auf 70°C erhitzt. Nach Abkühlung auf 20-25°C wird der Mischung Kaliumacetat bis zu einer Konzentration von 0,5 Mol/l zugesetzt. Dieses Gemisch wird 1 Stunde bei 00C inkubiert. Der ausgefallene Niederschlag wird durch Zentrifugieren (15 Minuten bei 4°C in einer Mikrozentrifuge) zentrifugiert. Aus der überstehenden Flüssigkeit wird durch Versetzen mit dem 2,5-fachen Volumen Ethanol bei 20-25°C die DNA ausgefällt. Die abgetrennte DNA wird in einer Lösung von 10 mM TRIS-HCl, enthaltend 10 μg/ml Ribonuclease A, gelöst, 10 Minuten bei 37°C inkubiert, mit Proteinase K bis zu einer Konzentration von 250 μg/ml versetzt und eine weitere Stunde bei 37°C inkubiert. Die Proteinase K wird durch Phenol-und Chloroform/ Isoamylalkohol-Extraktionen entfernt. Aus der wässrigen Phase wird die DNA durch Zusatz von 0,6 Volumenteilen 0,6 M isopropanolischer Natriumacetat-Lösung ausgefällt und in 50 μl einer Lösung, enthaltend 10 mMol/l TRIS-HCl und 5 mMol/l EDTA, bei pH 7,5 gelöst. Durch diese Präparation erhält man DNA-Sequenzen, die vorwiegend mehr als 50'000 Basenpaare enthalten. Restriktion dieser DNA mit EcoRV-Endonuclease, Hybridisierung der Fragmente mit radioaktiv markierten HindIII-Bruchstücken des NPT-II-Gens und Vergleich mit dem Plasmid pABDI zeigt in einer "Southern Blot"-Analyse die Anwesenheit des NPT-II-Gens in der Zellkern-DNA der transformierten Pflanzen-Zellen.

## Beispiel 14: "Dot-blot"-Analyse

10 µg genomischer DNA werden über Nacht mit EcoRI verdaut und anschliessend ohne vorherige elektrophoretische Auftrennung direkt punktförmig auf eine Nitrozellulosemembran aufgetragen (vgl. Verfahrensvorschrift von Schleicher und Schüll GmbH, Dassel, FRG).

Die Hybridisierungsreaktion und die anschliessende Autoradiographie werden in analoger Weise zu dem Southern blot-Verfahren durchgeführt.

Beispiel 15: Southern blot-Analyse:

Die Southern blot-Analyse der Pflanzen DNA wird entsprechend einer bei Paszkowski und Saul, 1983, beschriebenen Methode durchgeführt.

Ungefähr 5 µg genomischer DNA werden wahlweise mit EcoRV oder HindIII-Restriktionsenzymen geschnitten und die erhaltenen Bruchstücke durch Elektrophorese in einem 0,8 % bis 1 % Standard-Agarose-Gel aufgetrennt. (Maniatis T et al., 1982).

Die Nick-Translation wird dabei ersetzt durch die "random primer" Methode, die bei Feinberg und Vogelstein, 1983, beschrieben ist.

Beispiel 16: Untersuchung der Aminoglykosidphosphotransferase-Enzymaktivität transformierter Pflanzen

Blattstücke (100 bis 200 mg) werden in 20 µl Extraktionspuffer in einem Eppendorf-Zentrifugenröhrchen homogenisiert. Der Puffer ist eine Modifikation des von Herrera-Estrella, L., DeBlock, M., Messens, E., Hernalsteens, J.-P., Van Montagu, M. und J. Schell, EMBO J. 2, 987-995 (1983) verwendeten Puffers, in welchem Albumin aus Rinderblut durch 0,1 M Saccharose ersetzt wird. Die Extrakte werden 5 Minuten lang bei 12'000 g zentrifugiert und die überstehende Phase mit Bromphenolblau bis zu einer Endkonzentration von 0,004 % versetzt. Durch Elektrophorese in einem 10%igen, nicht denaturierenden Polyacrylamid-Gel werden die Proteine aus 35 µl der überstehenden Phase separiert. Das Gel wird mit einem Kanamycin und $\gamma^{32}$P-markierte ATP enthaltenden Agarose-Gel überschichtet, inkubiert, und die phosphorylierten Reaktionsprodukte werden auf Whatman-p81-Phosphozellulose-Papier übertragen. Das Papier wird sechsmal mit deionisiertem Wasser von 90°C gewaschen und dann autoradiografiert.

Ergebnisse:

Die Regeneration ganzer, transgener Pflanzen aus mikroinjizierten zygotischen Embryonen via direkter Embroygenese konnte sowohl beim Raps als auch bei Mais, Gerste und Reis innerhalb von 8 Wochen durchgeführt werden, wobei die erzielten Regenerationsraten sehr hoch liegen. Bei Pflanzen mit Sekundärembryogenese wurden die sekundären Regeneranten zur Bestimmung der Transformationsfrequenz verwendet. Für alle übrigen Pflanzen wird die folgende Methode angewendet: nach der Befruchtung werden die Samen dieser Pflanzen zum Keimen gebracht und diese Pflanzengeneration dazu verwendet, um den Nachweis für die Transformation und Expression des Gens zu erbringen. Die Transformationsfrequenzen liegen in einem Bereich von 5 % - 35 %.

Die stabile Integration der vollständigen mikroinjizierten Gene in die hochmolekulare DNA der F1-Generation, hervorgegangen aus dem primären Regeneranten, konnte an Hand der Southern blot-Analyse der genomischen DNA besagter F1-Generationspflanzen gezeigt werden. Die Expression der integrierten Gene wurde mit Hilfe des unter Beispiel 11 (Untersuchung der Aminoglykosidphosphotransferas-Enzymaktivität) beschriebenen Enzym-Assays nachgewiesen.

Hinterlegung:

Das im Rahmen der vorliegenden Erfindung verwendete Plasmid pHP23 wurde bei der als Internationale Hinterlegungsstelle anerkannten 'Deutschen Sammlung von Mikroorganismen' (DSM), in Braunschweig, Bundesrepublik Deutschland, entsprechend den Anforderungen des Budapester Vertrages für die Internationale Anerkennung der Hinterlegung von Mikroorganismen zum Zwecke der Patentierung, hinterlegt. Eine Erklärung zur Lebensfähigkeit der hinterlegten Proben wurde durch die besagte Internationale Hinterlegungsstelle ausgefertigt.

| Mikroorganismus | Hinterlegungsdatum | Hinterlegungs-Nummer* | Datum der Lebensfähigkeits-Bescheinigung |
|---|---|---|---|
| pHP23 (Escherichia coli DH1 transformiert mit pHP23 Plasmid DNA | 21.12.1987 | DSM 4323 | 21.12.1987 |

* Hinterlegungs-Nummer, ausgegeben durch die oben bezeichnete Internationale Hinterlegungsstelle.

Literaturverzeichnis

Barton, K.A., et al., Plant Physiol., 85: 1103-1109, 1987
Beck et al., Gene, 19: 327-336, 1982
Berry-Lowe et al., J. Mol., Appl. Genet., 1: 483-498, 1982
Birk, Y., et al., Biochim. Biophys. Acta, 67: 326-328, 1963
Cashmore A, "Genetic Engineering of Plants, an Agricultural Perspective", Plenum, New York, 1983, Seite 29-38
Chilton M-D, Scientific American, 248: 50-59, 1983
Coruzzi G et al, The Journal of Biological Chemistry, 258: 1399, 1983
De La Pena et al, Nature, 325: 274-276, 1987
Dunsmuir Pet al, J. Mol. Appl. Genet., 2: 285, 1983
Feinberg und Vogelstein, Analytical Biochemistry, 132: 6-13, 1983
Frank G et al, Cell, 21: 285-294, 1980
Gardner RC et al, Nucl. Acids Res., 9: 2871-2888, 1981
Grimsley NH et al, Nature, 325: 177-179, 1987
Hammond et al., J. Biol. Chem., 259: 9883-9890, 1984
Harris R et al, Plant Cell Reports, 7: 337-340, 1988
Hernalsteens JP et al, EMBO J., 3: 3039-3041, 1984
Herrera-Estrella L et al, EMBO J., 2: 987-995, 1983
Hilder, V.A., et al., Nature, 330: 160-163, 1987
Hohn B und Collins J, Gene, 11: 291-298, 1980
Hohn T et al, "Molecular Biology of Plant Tumors", Academic Press, Seite 549-560, 1982
Hooykaas-Van-Slogteren et al, Nature, 311: 763-764, 1984
Howard et al., Planta, 170: 535, 1987
Itakura, K., et al., J. Am. Chem. Soc., 97: 7327, 1975
Jacobson H et al, Eur. J. Biochem., 45: 623, 1974
Koop H-V und Schweiger HG, J. Plant Physiol. 121: 245-257, 1985
Maniatis et al, Molecular Cloning, Cold Spring Harbor Laboratories, 1982
Melnikow P.V. et al., GB 2,200,367
Messing und Vieira, Gene 19: 269-276, 1982
Morelli et al., Nature, 315: 200, 1985
Morikawa H und Yamada Y, Plant Cell Physiol., 26: 229-236, 1985
Neuhaus G et al, EMBO J, 3: 2169-2172, 1984
Neuhaus G et al, EMBO J, 5: 1437-1444, 1986
Nomura K und Komamine A, Plant Sci., 44: 53-58, 1986
Norrander J et al, Gene, 26: 101-106, 1983
Odell, J.T., et al., Nature, 313, 810, 1985
Paszkowski J et al, EMBO J, 3: 2717-2722, 1984
Paszkowski J und Saul M, Methods in Enzymology, 118: 627-646, 1986
Pennica, P., et al., Nature, 301: 214, 1983
Pietrzak M et al, Nucl. Acids Res., 14: 5868, 1986
Potrykus I et al, "Direct Gene Transfer to Protoplasts: An Efficient and Generally Applicable Method for Stable Alterations of Plant Genoms" in: Freeling M (ed.), PlantGenetics, A.R. Liss Inc., New York, pp. 181-199, 1986
Randolph LF, J. Agricultural Res., 53: 881-916, 1936
Rhodes CA, Biotechnology, 6: 56-60, 1988
Rigby WJ et al, J. Mol. Biol., 113: 237-251, 1977
Ryan, C., et al., Ann. Rev. Plant Physiol., 24: 173-196, 1973
Schweiger HG et al, Theor. Appl. Genet., 73: 769-783, 1987
Soberon X et al, Gene, 9: 287-305, 1980
Southern EM, J. Mol. Biol. 98: 503-517, 1975
Spangenberg G, "Manipulation individueller Zellen der Nutzpflanze Brassica napus L. mit Hilfe von Elektrofusion, Zellrekonstruktion und Mikroinjektion", PhD thesis, Universität Heidelberg, 1986
Spangenberg G et al, Europ. J. Cell Biol., 42: 236-238, 1986
Spena et al., EMBO J., 4: 2736, 1985

Steinbiss HH und Stable P, Protoplasma, 116: 223-227, 1983
Toriyama K et al, Theor. Appl. Genet., 73: 16-19, 1986
Vaeck, M., et al., Nature, 328: 33-37, 1987
Yamada A et al, Plant Cell Rep., 5: 85-88, 1986

## Ansprüche

1. Verfahren zur Einschleusung genetischen Materials in Pflanzen, dadurch gekennzeichnet, dass man eine DNA-haltige Lösung in junge zygotische Proembryonen bzw. Embryonen von Pflanzen, die ein hohes Regenerierungspotential in Form einer natürlichen Embryogenese besitzen, mikroinjiziert, die mikroinjizierten Pro-/Embryonen in geeigneten, die Embryogenese fördernden Kulturmedien mikrokultiviert und aus den so erhaltenen, in der Regel chimären Regeneranten gegebenenfalls nichtchimäre, transgene Pflanzen herstellt.

2. Verfahren gemäss Anspruch 1, das durch die folgenden spezifischen Verfahrensschritte gekennzeichnet ist:

a) Isolierung einzelner, für die Mikroinjektion geeigneter mehr- bis vielzelliger zygotischer Pro-/Embryonen aus den Samenanlagen von DonorPflanzen;

b) Selektion der isolierten zygotischen Pro-/Embryonen für die sich anschliessende Mikroinjektion und Einbringen der selektionierten Pro-/Embryonen in ein geeignetes Kulturmedium;

c) Mikroinjektion einer DNA-haltigen Lösung, enthaltend eine oder mehrere DNA-Fragmente in die unter a) isolierten zygotischen Pro-/Embryonen;

d) Mikrokultivierung der mikroinjizierten zygotischen Pro-/Embryonen in geeigneten, eine direkte Embryogenese fördernden Kulturmedien;

f) Regeneration besagter transformierter zygotischer Pro-/Embryonen zu vollständigen, in der Regel chimären Pflanzen; und

g) Herstellung nicht-chimärer, transgener Pflanzen mit neuen und wünschenswerten Eigenschaften.

3. Verfahren gemäss Anspruch 2, dadurch gekennzeichnet, dass die Kultivierung der mikroinjizierten Pro-/Embryonen unter Bedingungen erfolgt, die die Entwicklung einer morphogenen Zellkultur oder die Bildung sekundärer Embryonen ermöglicht.

4. Verfahren gemäss Anspruch 1, dadurch gekennzeichnet, dass man die Mikroinjektion mit Hilfe einer Mikroinjektionsapparatur durchführt, die sich im wesentlichen aus folgenden Teilen zusammensetzt:

a) einem Mikroskop zur optischen Kontrolle des Injektionsvorganges

b) motorisch oder mechanisch betriebenen Mikromanipulatoren mit Kapillarhalterungen

c) Mikroinjektionskapillaren mit einem Aussendurchmesser von 1,0 mm bis 2,0 mm und einem Spitzendurchmesser von $\leq$ 1 $\mu$m bis 10 $\mu$m

d) Haltekapillaren mit einem Spitzendurchmesser von 0,02 mm bis 1,0 mm

e) einer pneumatisch gesteuerten Druckvorrichtung zur Injektion der DNA-haltigen Lösung.

5. Verfahren gemäss Anspruch 4, dadurch gekennzeichnet, dass es sich bei den Mikroinjektionskapillaren um Mikroelektroden mit eingeschmolzenen Glasfilamenten aus Borosilikat handelt.

6. Verfahren gemäss Anspruch 1, dadurch gekennzeichnet, dass der Mikroinjektionsvorgang in folgenden Teilschritten abläuft:

a) Einbringen der Proembryonen bzw. Embryonen in 10 $\mu$l bis 200 $\mu$l Medientropfen auf Deckgläser

b) Fixieren der Embryonen mit Hilfe der Haltekapillare

c) Injektion der DNA haltigen lösung in die Einzelzellen des Pro-/Embryos mit Hilfe der Mikroinjektionskapillare

d) Drehen der Pro-/Embryonen nach jedem Injektionsvorgang und erneute Injektion in Einzelzellen

e) Kultivierung der injizierten Pro-/Embryonen in geeigneten Kulturmedien.

7. Verfahren gemäss Anspruch 6, dadurch gekennzeichnet, dass man die DNA-haltige Lösung in pflanzliche Pro-/Embryonen injiziert, die sich in einem optimalen Entwicklungsstadium befinden, d.h. die sich gute Mikroinjizierbarkeit, gute Isolierbarkeit und gute Regenerationsfähigkeit auszeichnen.

8. Verfahren gemäss Anspruch 6, dadurch gekennzeichnet, dass man Embryonen verwendet, die sich in einem früheren globulären Proembryonalstadium bis frühen Embryonalstadium befinden.

9. Verfahren gemäss Anspruch 6, dadurch gekennzeichnet, dass man frühe Embryonalstadien verwendet, die ausser dem Scutellum bereits eine gut erkennbare Spross- und Wurzelmeristemanlage aufweisen.

10. Verfahren gemäss Anspruch 6, dadurch gekennzeichnet, dass man die DNA-haltige Lösung in pflanzliche Proembryonen bzw. Embryonen mikroinjiziert, die sich im 2 bis ca. 5000 Zellstadium befinden.

11. Verfahren gemäss Anspruch 10, dadurch gekennzeichnet, dass sich die pflanzlichen Pro-/Embryonen im 4 bis 100 Zellstadium befinden.

12. Verfahren gemäss Anspruch 6, dadurch gekennzeichnet, dass man den Mikroinjektionsvorgang 1 bis 1000 mal pro Embryo bzw. Proembryo durchführt.

13. Verfahren gemäss Anspruch 12, dadurch gekennzeichnet, dass man 4 bis 60 mal pro Embryo bzw. Proembryo mikroinjiziert.

14. Verfahren gemäss Anspruch 12, dadurch gekennzeichnet, dass man die DNA-haltige Lösung in das Gewebe der Sprossmeristemanlage injiziert.

15. Verfahren gemäss Anspruch 6, dadurch gekennzeichnet, dass das Injektionsvolumen 1 pl bis 100 pl pro injizierter Zelle beträgt.

16. Verfahren gemäss Anspruch 6 dadurch gekennzeichnet, dass die DNA-Konzentration der injizierten DNA-haltigen Lösung zwischen 0,1 $\mu g/\mu l$ und 10 $\mu g/\mu l$ beträgt.

17. Verfahren gemäss Anspruch 6, dadurch gekennzeichnet, dass es sich bei besagter DNA-haltiger Lösung um eine Pufferlösung handelt.

18. Verfahren gemäss Anspruch 17, dadurch gekennzeichnet, dass der pH-Wert der Pufferlösung in einem Bereich zwischen pH 7,0 und pH 8,0 liegt.

19. Verfahren gemäss Anspruch 6, dadurch gekennzeichnet, dass besagte DNA-haltige Injektionslösung die transformierende DNA

a) in linearisierter Form enthält;

b) in überspiralisierter Form enthält; oder

c) in Form eines Gemisches aus linearisierter und überspiralisierter DNA enthält.

20. Verfahren gemäss Anspruch 19, dadurch gekennzeichnet, dass besagte transformierende DNA von neutralen DNA-Sequenzen flankiert wird.

21. Verfahren gemäss Anspruch 6, dadurch gekennzeichnet, dass die zur Mikroinjektion bestimmte DNA-haltige Lösung ein rekobminantes DNA-Molekül enthält, das in der transformierten Pflanze zu nützlichen und wünschenswerten Eigenschaften führt.

22. Verfahren gemäss Anspruch 21, dadurch gekennzeichnet, dass besagtes rekombinantes DNA-Molekül im wesentlichen aus einem (oder mehreren) Strukturgen(en) besteht, das (die) für eine nützliche und wünschenswerte Eigenschaft kodiert(en) und das (die) mit in der pflanzlichen Zelle aktiven Expressionssignalen in operabler Weise verknüpft ist (sind), so dass eine Expression in der pflanzlichen Zelle gewährleistet ist.

23. Verfahren gemäss Anspruch 21, dadurch gekennzeichnet, dass besagtes rekombinantes DNA-Molekül entweder aus genomischer DNA, aus einer cDNA oder aus synthetischer DNA oder aus einer Kombination besagter DNA besteht.

24. Verfahren gemäss Anspruch 22, dadurch gekennzeichnet, dass besagtes Strukturgen

a) der transformierten Pflanze eine erhöhte Resistenz oder Toleranz gegenüber Pathogenen, Herbiziden, Insektiziden oder anderen Bioziden verleiht;

b) der transformierten Pflanze eine erhöhte Resistenz oder Toleranz gegenüber nachteiligen Umwelteinflüssen verleiht, ausgewählt aus der Gruppe: Hitze, Wind, Kälte, Trockenheit, Feuchtigkeit, besondere extreme Bodenverhältnisse, osmotischer Stress;

c) die Bildung und Qualität von Reserve- und Speicherstoffen in Blättern, Samen, Knollen, Wurzeln und Stengeln verbessert; oder

d) für pharmazeutisch akzeptable Wirksubstanzen oder andere physiologisch akitive Substanzen kodiert.

25. Verfahren gemäss Anspruch 2, dadurch gekennzeichnet, dass die DNA-haltige Lösung eine nicht-kodierende DNA mit regulatorischer Funktion enthält.

26. Verfahren gemäss Anspruch 22, dadurch gekennzeichnet, dass besagte Expressionssignale aus Genen von Pflanzen oder Pflanzenviren stammen.

27. Verfahren gemäss Anspruch 26, dadurch gekennzeichnet, dass besagte Expressionssignale aus Genen des Cauliflower Mosaik Virus (CaMV) stammen oder aber diesen homolog sind.

28. Verfahren gemäss Anspruch 27, dadurch gekennzeichnet, dass es sich um

a) die 35 S-Expressionssignale des CaMV Genoms oder um deren Homologe; oder

b) die 19 S Expressionssignale des CaMV-Genomes oder um deren Homologe handelt.

29. Verfahren gemäss Anspruch 3, dadurch gekennzeichnet, dass die Mikrokultivierung der mikroinjizierten Proeembryonen und Embryonen in einem flüssigen Kulturmedium durchgeführt wird, das die direkte Embryogenese anregt.

30. Verfahren gemäss Anspruch 29, dadurch gekennzeichnet, dass besagtes Kulturmedium mit einem osmotisch neutralen Verdickungsmittel versetzt ist.

31. Verfahren gemäss Anspruch 29, dadurch gekennzeichnet, dass besagtes flüssiges Kulturmedium einem Festmedium überschichtet ist.

32. Verfahren gemäss Anspruch 30, dadurch gekennzeichnet, dass es sich bei besagtem Verdickungsmittel um Co-Polymere aus Saccharose und Epichlorhydrin mit einem Molekulargewicht zwischen 70 000 und 400 000 handelt.

33. Verfahren gemäss Anspruch 31, dadurch gekennzeichnet, dass besagtes Festmedium ein Verfestigungsmittel ausgewählt aus der Gruppe bestehend aus Agar, Agarose, Alginat, Pektinat, Gelatine oder Gelrite® enthält.

34. Verfahren gemäss Anspruch 29, dadurch gekennzeichnt, dass besagtes Kulturmedium die folgende Zusammensetzung aufweist:

a) für Wachstum und Entwicklung der pflanzlichen Zelle essentielle mineralische Stoffe bzw. Elemente in ihren Verbindungen, ausgewählt aus der Gruppe bestehend aus Stickstoff, Phosphor, Schwefel, Kalium, Calcium, Magnesium und Eisen u.a. (Makroelemente) sowie Natrium, Zink, Kupfer, Mangan, Bor, Vanadium, Selen und Molybdän u.a. (Mikroelemente)

b) essentielle Vitamine, ausgewählt aus der Gruppe bestehend aus Nikotinsäure, Pyridoxin, Thiamin, Inosit, Biotin, Liponsäure, Riboflavin, Ca-Pantothenat u.a.

c) eine leicht assimilierbare Kohlenstoffquelle, ausgewählt aus der Gruppe Saccharose, Glucose u.a.

d) verschiedene Wachstumsregulatoren in Form natürlicher oder synthetischer Phytohormone, ausgewählt aus der Klasse der Auxine und Cytokinine.

e) osmotische Stabilisatoren, ausgewählt aus der Gruppe bestehend aus Zuckeralkoholen, Zucker- oder Salzionen u.a.

35. Verfahren gemäss Anspruch 34, dadurch gekennzeichnet, dass besagte Kulturmedien die verschiedenen Wachstumsregulatoren aus der Gruppe der Auxine und Cytokinine in einem ausgewogenen Verhältnis zueinander enthalten, sodass ein kontrollierter Ablauf der direkten Embryogenese gewährleistet ist.

36. Verfahren gemäss Anspruch 35, dadurch gekennzeichnet, dass die Phytohormone aus der Gruppe der Auxine und der Cytokinine in einem Konzentrationsverhältnis zwischen 0,01 mg/l und 20,0 mg/l vorliegen, abhängig vom jeweiligen Entwicklungsstand der Embryonen.

37. Verfahren gemäss Anspruch 34, dadurch gekennzeichnet, dass der pH-Wert des Kulturmediums zwischen pH 4.5 und pH,7.5 beträgt.

38. Verfahren gemäss Anspruch 34, dadurch gekennzeichnet, dass man für die Kultivierung der Embryonen komplexe Nährmedium verwendet, die ganz oder zumindest teilweise aus natürlichen Komponenten zusammengesetzt sind.

39. Verfahren gemäss Anspruch 38, dadurch gekennzeichnet, dass es sich bei besagten natürlichen Komponenten um solche, ausgewählt aus der Gruppe bestehend aus Kartoffelextrakt, Kokosnussmilch oder flüssiges Endosperm u.a., handelt.

40. Verfahren gemäss Anspruch 29, dadurch gekennzeichnet, dass die Kultivierungsdichte per Pro-/Embryonen zwischen 1 Pro-/Embryo pro µl Kulturmedium und 50 Pro-/Embryonen pro µl Kulturmedium beträgt, abhängig von der Grösse und dem Entwicklungsstand der Pro-/Embryonen.

41. Verfahren gemäss Anspruch 29, dadurch gekennzeichnet, dass die Mikrokultivierung der Pro-/Embryonen zumindest zeitweise in einem Zweikompartimenten-System durchgeführt wird.

42. Verfahren gemäss Anspruch 29, dadurch gekennzeichnet, dass man die mikroinjizierten Pro-/Embryonen alle 1 bis 12 Tage in frisches Kulturmedium transferiert bzw. mit frischem Kulturmedium überschichtet.

43. Verfahren gemäss Anspruch 2, dadurch gekennzeichnet, dass man die Pro-/Embryonen, wenn sie ein bestimmtes Entwicklungsstadium erreicht haben, in der Regel nach 10 bis 30 Tagen, zur Differenzierung der Spross- und Wurzelscheitel auf feste Kulturmedien, die üblicherweise für die Regeneration von Pflanzen verwendet werden und die gegebenenfalls von einem Flüssigmedium überschichtet sein können, überführt.

44. Verfahren gemäss Anspruch 2, dadurch gekennzeichnet, dass man die Pro-/Embryonen nach einer Kultivierungsdauer von 4 bis 8 Wochen, spätestens aber nach dem Auskeimen der Embryonen auf Festmedien überträgt, die keine oder aber nur geringe Hormonkonzentration aufweisen.

45. Verfahren gemäss Anspruch 44, dadurch gekennzeichnet, dass man die regenerierten Pflänzchen in Erde pflanzt und in gleicher Weise wie normale Sämlinge weiterbehandelt.

46. Verfahren zur Herstellung reinerbiger homozygoter Transformanten gemäss Anspruch 1, dadurch gekennzeichnet, dass man

a) bei Pflanzen die einer sekundären Embryogenese oder einer sekundären Adventivspross-Bildung zugänglich sind, in vitro die sekundären Strukturen, die sich aus Einzelzellen ableiten, von den primär entstandenen, meist chimären Regeneranten trennt, vereinzelt und zu ganzen Pflanzen regeneriert oder

b) transgene chimäre Pflanzen gegebenenfalls wiederholt mit sich selbst kreuzt.

47. Verfahren gemäss Anspruch 1, dadurch gekennzeichnet, dass man Proembryonen bzw. Embryonen von Pflanzen, ausgewählt aus der Gruppe bestehend aus den Familien Solanaceae, Cruciferae, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae, Gramineae oder der Ordnung Leguminoseae verwendet.

48. Verfahren gemäss Anspruch 1, dadurch gekennzeichnet, dass man Proembryonen bzw. Embryonen von Pflanzen der Familie Malvaceae verwendet.

49. Verfahren nach Anspruch 48, dadurch gekennzeichnet, dass man Proembryonen bzw. Embryonen von Pflanzen, ausgewählt aus der Gruppe bestehend aus den Gattungen Allium, Avena, Hordeum, Oryzae, Panicum, Saccharum, Secale, Setaria, Sorghum, Triticum, Zea, Musa, Cocos, Phoenix, Elaeis verwendet.

50. Verfahren zur Herstellung transgener Pflanzen gemäss Anspruch 1.

51. Transgene Pflanzen sowie deren sexuelle und asexuelle Nachkommenschaft, hergestellt nach einem Verfahren gemäss Anspruch 1.

52. Transgenes Saatgut von Pflanzen und deren Nachkommen gemäss Anspruch 51.

53. Vermehrungsgut transgener Pflanzen gemäss Anspruch 51 ausgewählt aus der Gruppe bestehend aus Protoplasten, Zellen, Zellklonen, Zellagglomeraten, Kallus- und/oder Gewebekulturen, Samen, Pollen, Eizellen, Zygoten, Embryonen oder anderem aus Keimbahn-Zellen hervorgegangenem Vermehrungsgut sowie die daraus abgeleiteten Nachkommen.

54. Alle Kreuzungs- und Fusionsprodukte transgener Pflanzen gemäss Anspruch 51, die noch die durch die Transformation bedingten neuen Eigenschaften aufweisen.

55. Varianten und Mutanten von transgenen Pflanzen gemäss Anspruch 51 sowie von deren sexueller und asexueller Nachkommenschaft, die noch die charakteristischen, durch die Transformation bedingten Eigenschaften des pflanzlichen Ausgangsmaterials, das für die Herstellung besagter Mutanten und Varianten verwendet wurde, aufweisen.

56. Pflanzenteile, wie z.B. Blüten, Stengel, Früchte, Blätter, Wurzeln, die von transgenen Pflanzen gemäss Anspruch 51 oder deren Nachkommen stammen und die wenigstens zum Teil aus transgenen Zellen aufgebaut sind.

57. Rekombinantes DNA-Molekül gemäs Anspruch 21, zur Verwendung in einem Verfahren gemäss Anspruch 1.

58. Transgene Pflanzen, die aus transformierten zygotischen Proembryonen oder aus transformierten zygotischen Embryonen regeneriert werden.

**Abbildung 1:** Teil I

PROEMBRYO
Sprossmeristemanlage
Wurzelmeristemanlage
EMBRYO

PROEMBRYO / EMBRYO

Keimblattanlage

Meristemanalgen

UNREIFER
EMBRYO

REIFER
EMBRYO

**Abbildung 1:** Teil II

**Abbildung 2**